(19) Europäisches Patentamt
European Patent Office
Office européen des brevets

(11) EP 4 785 961 A1

(12) EUROPEAN PATENT APPLICATION
published in accordance with Art. 153(4) EPC

(43) Date of publication:
**05.08.2026 Bulletin 2026/32**

(21) Application number: **24870849.7**

(22) Date of filing: **26.09.2024**

(51) International Patent Classification (IPC):
***A61K 47/68*** *(2017.01)* ***A61K 31/4745*** *(2006.01)*
***A61P 35/00*** *(2006.01)*

(52) Cooperative Patent Classification (CPC):
**A61K 31/4745; A61K 47/68; A61P 35/00**

(86) International application number:
**PCT/CN2024/121324**

(87) International publication number:
**WO 2025/067306 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.09.2023 CN 202311248255**
**24.09.2024 CN 202411331453**

(71) Applicant: **Fortvita Biologics Inc.**
**1205 Grand Cayman (KY)**

(72) Inventors:
- **HE, Kaijie**
**Suzhou, Jiangsu 215123 (CN)**
- **GUAN, Jian**
**Suzhou, Jiangsu 215123 (CN)**

(74) Representative: **Sagittarius IP**
**Marlow International**
**Parkway**
**Marlow SL7 1YL (GB)**

Remarks:
The complete document including Reference Table(s) and the Sequence Listing(s) can be downloaded from the EPO website

# (54) ANTIBODY-DRUG CONJUGATE TARGETING HER3

(57) The present disclosure relates to an antibody-drug conjugate targeting HER3, a pharmaceutical composition comprising the antibody-drug conjugate, use of the antibody-drug conjugate in the treatment or prevention of a disease or disorder associated with HER3 activity, particularly a HER3-positive tumor, and use of the antibody-drug conjugate in the preparation of a medicament for the treatment or prevention of a disease or disorder associated with HER3 activity, particularly a HER3-positive tumor.

EP 4 785 961 A1

## Description

### FIELD OF THE INVENTION

The present disclosure relates to an HER3-targeted antibody-drug conjugate (ADC), a pharmaceutical composition comprising the same, use of the ADC in treating or preventing a disease or disorder associated with HER3 activity, particularly an HER3-positive tumor, and use of the ADC in preparing a medicament for treating or preventing a disease or disorder associated with HER3 activity, particularly an HER3-positive tumor.

### BACKGROUND OF THE INVENTION

HER3 (encoded by ErbB3) is a member of the HER protein receptor family. HER3 was originally considered to have no kinase activity, but recent research results show that HER3 is capable of binding to ATP and promoting intracellular domain phosphorylation, but the kinase activity is relatively weak. Studies have found that HER3 plays an important role in HER2/HER3 heterodimerization and subsequent activation of PI3K/AKT cascade signals. Therefore, HER3 has received extensive attention and research in recent years.

HER3 is overexpressed in a variety of cancers, such as gastric cancer, breast cancer, colorectal cancer and lung cancer, and the expression of HER3 is correlated with the poor postoperative survival rate of patients. Studies have shown that MET amplification may be activated to induce resistance to EGFR-targeted therapies by maintaining HER3-mediated PI3K/AKT signaling; in addition, HER3 not only has enhanced expression in a variety of cancers, but also has potential functions in mediating resistance to targeted therapies. Therefore, HER3 is a potential and attractive therapeutic target.

Patritumab is an IgG1 HER3 monoclonal antibody with extremely strong cellular internalization. Daiichi Sankyo used the strong endocytosis of HER3 to combine patritumab with a cleavable tetrapeptide linker and a topoisomerase I inhibitor (DXd) to form a novel HER3-DXd (U3-1402) ADC molecule with a DAR of 8. The data from the U3-1402 clinical study showed that HER3-DXd not only had anti-tumor activity in breast and lung cancers, but also had anti-tumor activity in tumor patients with multiple EGFR resistance mechanisms (including EGFR C797S mutation, MET amplification, HER2 mutation, BRAF fusion, and PIK3CA mutation), and sustained efficacy was clinically observed.

Although the above drugs have appeared in the prior art, there is still a wide demand for therapeutic drugs that can target HER3 and be used for treating HER3-positive tumors, particularly antibody-drug conjugates with high affinity, good specificity, low risk of immunogenicity, strong anti-tumor activity and/or better bystander effect.

### SUMMARY OF THE INVENTION

The present disclosure is based on the HER3-targeted antibody-drug conjugate developed by the inventors, which has stronger anti-tumor activity, better bystander effect, and better efficacy and PK effect compared to the prior art. Therefore, the HER3-targeted antibody-drug conjugate of the present disclosure is particularly suitable for treating or preventing a disease or disorder associated with HER3 activity, particularly a HER3-positive tumor.

In one aspect, the present disclosure provides an antibody-drug conjugate having formula (I), a stereoisomer or pharmaceutically acceptable salt or solvate thereof:

$$Ab\text{-}(L\text{-}D)_n \qquad (I)$$

wherein,

Ab is an antibody or an antigen-binding fragment thereof that binds to HER3, comprising a heavy chain variable region and a light chain variable region, wherein the heavy chain variable region comprises a HCDR1, a HCDR2, and a HCDR3, comprising the amino acid sequences set forth in SEQ ID NO: 1, SEQ ID NO: 2, and SEQ ID NO: 3, respectively; and the light chain variable region comprises a LCDR1, a LCDR2, and a LCDR3, comprising the amino acid sequences set forth in SEQ ID NO: 4, SEQ ID NO: 5, and SEQ ID NO: 6, respectively

L is a linker having the following structure

: $-Z-E-NH-CH_2-$,

wherein Z is linked to Ab, and $-CH_2-$ is linked to D;

Z is selected from

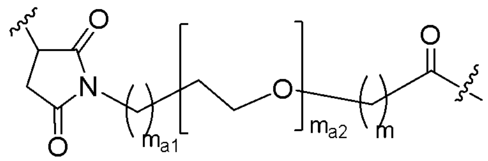

,

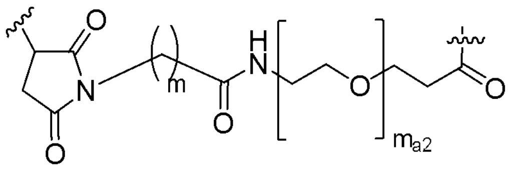

, and

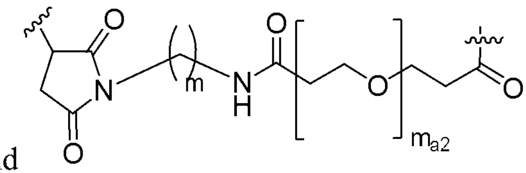

;

wherein $m_{a1}$ and $m_{a2}$ are independently selected from an integer of 0-20;
m is selected from an integer of 1-10,
the carbonyl group at the right end of Z is covalently linked to E;
E is a peptide residue comprising 2-10 amino acids, wherein the peptide residue is optionally substituted with one or more groups selected from $C_{1-6}$ alkyl and a polyol group, wherein the N-terminus of the peptide residue is covalently linked to Z,
D is a cytotoxic compound having a structure of formula $-Q-L^2-L^1-D^1$,
wherein Q is-O- or -S-;
$L^1$ is absent or -($C_1$-$C_{10}$ alkylene)-;
$L^2$ is absent, *-($C_1$-$C_{10}$ alkylene)-C(O)N($R^5$)- or *-($C_1$-$C_{10}$ alkylene)-N($R^5$)C(O)-; wherein * indicates that the end is covalently linked to Q; and $R^5$ is H or $C_1$-$C_6$alkyl,
wherein $D^1$ has a structure of formula (D-1):

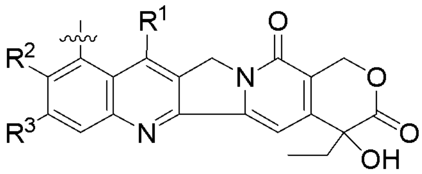

(D-1)

wherein $R^1$ is selected from H, $C_1$-$C_6$ alkyl, $C_2$-$C_6$alkenyl, $C_2$-$C_6$alkynyl, $C_1$-$C_6$haloalkyl, $C_2$-$C_6$ haloalkenyl and $C_2$-$C_6$ haloalkynyl;
$R^2$ is selected from H, halogen, $C_1$-$C_6$alkyl, $C_1$-$C_6$haloalkyl, -$OR^4$ and -$SR^4$; $R^3$ is selected from H, halogen, CN, $C_1$-$C_6$alkyl, $C_1$-$C_6$haloalkyl and -$OR^4$; or $R^2$ and $R^3$ together form - $O(CH_2)_{n1}O$- or -$O(CF_2)_{n1}O$-, wherein n1 is 1 or 2;
$R^4$ is selected from H and $C_1$-$C_4$ alkyl; and
n represents DAR, and n is a natural number selected from 1-15.

In some embodiments of the present disclosure, the Ab comprises a heavy chain variable region, wherein the heavy chain variable region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 7; or
(ii) comprises or consists of the amino acid sequence set forth in SEQ ID NO: 7; or
(iii) comprises or consists of an amino acid sequence having one or more amino acid substitutions, insertions or deletions compared with the amino acid sequence set forth in SEQ ID NO: 7, wherein optionally, the amino acid substitutions, insertions or deletions do not occur in the CDRs.

In some embodiments of the present disclosure, the Ab comprises a light chain variable region, wherein the light chain variable region

(i) comprises or consists of an amino acid sequence having at least 90%, 91%, 92%, 93%, 94%, 95%, 96%, 97%, 98%, or 99% identity to the amino acid sequence set forth in SEQ ID NO: 9; or
(ii) comprises or consists of the amino acid sequence set forth in SEQ ID NO: 9; or
(iii) comprises or consists of an amino acid sequence having one or more amino acid substitutions, insertions or

deletions compared with the amino acid sequence set forth in SEQ ID NO: 9, wherein optionally, the amino acid substitutions, insertions or deletions do not occur in the CDRs.

In some embodiments of the present disclosure, the Ab further comprises a heavy chain constant region, wherein the heavy chain constant region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 11; or
(ii) comprises or consists of the amino acid sequence set forth in SEQ ID NO: 11; or
(iii) comprises or consists of an amino acid sequence having one or more amino acid substitutions, insertions, or deletions compared with the amino acid sequence set forth in SEQ ID NO: 11.

In some embodiments of the present disclosure, the Ab further comprises a light chain constant region, wherein the light chain constant region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 12; or
(ii) comprises or consists of the amino acid sequence set forth in SEQ ID NO: 12; or
(iii) comprises or consists of an amino acid sequence having one or more amino acid substitutions, insertions, or deletions compared with the amino acid sequence set forth in SEQ ID NO: 12.

In some embodiments of the present disclosure, the Ab comprises a heavy chain and/or a light chain, wherein The heavy chain

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 13; or
(ii) comprises or consists of the amino acid sequence set forth in SEQ ID NO: 13; or
(iii) comprises or consists of an amino acid sequence having one or more amino acid substitutions, insertions, or deletions compared with the amino acid sequence set forth in SEQ ID NO: 13; and/or

The light chain

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 14; or
(ii) comprises or consists of the amino acid sequence set forth in SEQ ID NO: 14; or
(iii) comprises or consists of an amino acid sequence having one or more amino acid substitutions, insertions, or deletions compared with the amino acid sequence set forth in SEQ ID NO: 14.

In some embodiments of the present disclosure, the Ab is an intact antibody. In some embodiments of the present disclosure, the Ab is a multispecific antibody, e.g., a bispecific antibody.
In some embodiments of the present disclosure, the Ab is selected from an antigen-binding fragment Fv, Fab, Fab', or F(ab') $_2$; a single-chain antibody scFv; or a single-domain antibody VHH.
In some embodiments of the present disclosure, the linker is linked to Ab via a side chain of a cysteine residue.
In some embodiments of the present disclosure, the antibody-drug conjugate has the following structure:

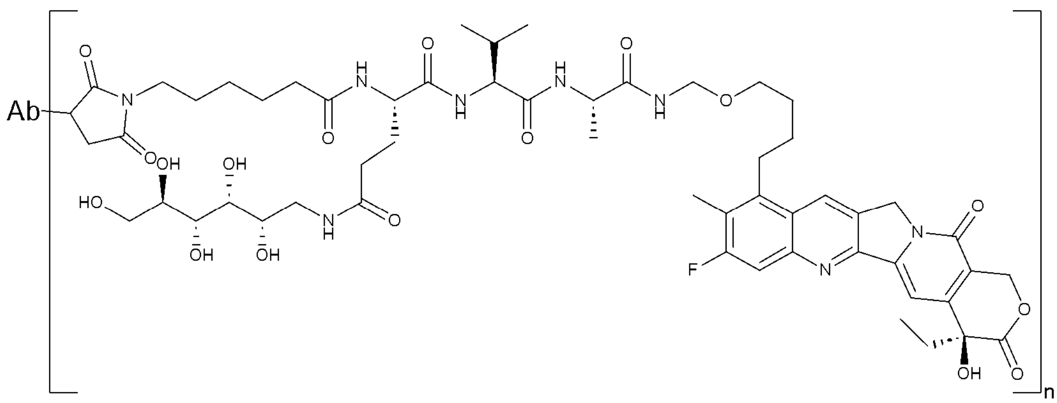

wherein Ab is as defined herein, and n is 1, 2, 3, 4, 5, 6, 7, 8, 9 or 10.

In some embodiments of the present disclosure, the antibody-drug conjugate has an average DAR of 2-10, 4-9, or 7.5-8.5.

In one aspect, the present disclosure provides a pharmaceutical composition comprising the antibody-drug conjugate or the stereoisomer thereof, or the pharmaceutically acceptable salt or solvate thereof described in the present disclosure, and a pharmaceutically acceptable carrier or excipient.

In one aspect, the present disclosure provides use of the antibody-drug conjugate or the stereoisomer thereof, or the pharmaceutically acceptable salt or solvate thereof described herein in preparing a medicament for treating or preventing a disease or disorder associated with HER3 activity.

In some embodiments of the present disclosure, the disease or disorder associated with HER3 activity is a HER3-positive tumor.

In some embodiments of the present disclosure, the HER3-positive tumor is selected from the group consisting of breast cancer, lung cancer, pancreatic cancer, colorectal cancer, gastric cancer, head and neck squamous cell carcinoma, ovarian cancer, melanoma, prostate cancer, and bladder cancer.

In one aspect, the present disclosure provides the antibody-drug conjugate or the stereoisomer thereof, or the pharmaceutically acceptable salt or solvate thereof described herein for use in treating or preventing a disease or disorder associated with HER3 activity.

In some embodiments of the present disclosure, the disease or disorder associated with HER3 activity is a HER3-positive tumor.

In some embodiments of the present disclosure, the HER3-positive tumor is selected from the group consisting of breast cancer, lung cancer, pancreatic cancer, colorectal cancer, gastric cancer, head and neck squamous cell carcinoma, ovarian cancer, melanoma, prostate cancer, and bladder cancer.

In one aspect, the present disclosure provides a method for treating or preventing a disease or disorder associated with HER3 activity, which comprises administering to a subject an effective amount of the antibody-drug conjugate or the stereoisomer thereof, or the pharmaceutically acceptable salt or solvate thereof described herein.

In some embodiments of the present disclosure, the disease or disorder associated with HER3 activity is a HER3-positive tumor.

In some embodiments of the present disclosure, the HER3-positive tumor is selected from the group consisting of breast cancer, lung cancer, pancreatic cancer, colorectal cancer, gastric cancer, head and neck squamous cell carcinoma, ovarian cancer, melanoma, prostate cancer, and bladder cancer.

In some embodiments of the present disclosure, the method further comprises conjointly administering one or more therapeutic agents selected from the group consisting of chemotherapeutic agents, angiogenesis inhibitors, cytokines, cytotoxic agents, other antibodies, small molecule drugs, and immunomodulatory agents.

In some embodiments of the present disclosure, the antibody-drug conjugate or the stereoisomer thereof, or the pharmaceutically acceptable salt or solvate thereof and the one or more therapeutic agents are administered simultaneously or sequentially in any order.

In some embodiments of the present disclosure, the method further comprises administering to the patient one or more treatment modalities selected from radiation therapy or surgery.

Compared with the prior art, the antibody-drug conjugate targeting HER3 provided by the present disclosure has the following advantages:

1) the small molecule drug moiety MB1 released in vivo by the antibody-drug conjugate targeting HER3 of the present invention has stronger activity than DXd in the prior art, and in vitro and in vivo efficacy data show better anti-tumor activity;
2) the small molecule drug moiety MB3 in the HER3-targeting antibody-drug conjugate of the present invention itself has good hydrophilicity, but after intracellular cleavage, it is easier to release the lipophilic free small molecule drug MB1, and MB3 comprehensively shows a stronger bystander effect;
3) the HER3-targeting antibody-drug conjugate of the present invention shows excellent effects of high in vivo drug exposure, good PK and good efficacy in pathological experiments;
4) The HER3-targeting antibody-drug conjugate of the present invention shows high drug safety and clinical treatment safety in toxicology experiments, and can be administered at higher drug doses.

## BRIEF DESCRIPTION OF THE DRAWINGS

FIG. 1 shows the results of the in vitro killing of SW620 by different HER3-ADCs.
FIG. 2 shows the results of the in vitro killing of MCF-7 cells by different HER3-ADCs.

FIG. 3 shows the results of the evaluation of the in vitro efficacy of potential drug resistance of different Payloads in the Elacridar-resistant HCT15 cell line.
FIG. 4 shows the bystander effect results of HER3-ADCs conjugated with different linkers and payloads.
FIG. 5 shows a schematic structural diagram of the HER3-MB3 molecule in Example 1.
FIG. 6 shows the relative expression level of HER3 in tumor cell lines.
FIG. 7 shows the results of in vitro killing of HER3-MB3 by NUGC4.
FIG. 8 shows the results of in vitro killing of HCC1569 by HER3-MB3.
FIG. 9 shows the results of in vitro killing of HER3-MB3 by NCI-H508.
FIG. 10 shows the results of in vitro killing of SW620 by HER3-MB3.
FIG. 11 shows the results of in vitro killing by HER3-MB3 in HCC827WT and HCC827+HER3OX.
FIG. 12 shows the bystander effect of HER3-MB3.
FIG. 13 shows the efficacy results of HER3-MB3 in an SW620 mouse tumor model.
FIG. 14 shows the body weight change results of HER3-MB3 in the SW620 mouse tumor model.
FIG. 15 shows the efficacy results of HER3-MB3 in a NUGC4 mouse tumor model.
FIG. 16 shows the body weight change results of HER3-MB3 in the NUGC4 mouse tumor model.
FIG. 17 shows a graph showing the relationship between the blood concentration of HER3-ADC in mice and time.
FIG. 18 shows a toxicokinetic study of HER3-MB3 in cynomolgus monkeys.

## DETAILED DESCRIPTION OF THE INVENTION

Reference will now be made in detail to certain embodiments, examples of which are illustrated in the accompanying detailed description. While the enumerated embodiments will be described, it will be understood that they are not intended to limit the invention to those embodiments. On the contrary, the invention is intended to cover all alternatives, modifications, and equivalents, which may be included within the scope of the invention as defined by the claims. Those skilled in the art will recognize many methods and materials similar or equivalent to those described herein, which can be used in the practice of the present invention. The invention is in no way limited to the methods and materials described. In the event that one or more of the incorporated literature and similar materials differs from or contradicts this disclosure, including but not limited to defined terms, term usage, described techniques, or the like, this disclosure controls.

It will be appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## I. DEFINITIONS

Unless defined otherwise, technical and scientific terms used herein have the same meaning as commonly understood by one of ordinary skill in the art to which this invention belongs.

The term "or" refers to a single element of a list of selectable elements unless the context clearly dictates otherwise. The term "and/or" refers to any one, any two, any three, any more, or all of the listed optional elements.

As used herein, the term "about" denotes a value within a range of $\pm$10% of a given numerical value.

As used herein, the terms "comprises," "comprising," "includes," "including," "has," "having" and similar referents do not exclude unrecited elements. These terms also include cases consisting only of the recited elements.

As used herein, the term "HER3" (human epidermal growth factor receptor 3, also known as ErbB3) is a receptor protein tyrosine kinase and belongs to the epidermal growth factor receptor (EGFR) subfamily of receptor protein tyrosine kinases, which also includes HER1 (also known as EGFR), HER2, and HER4. HER3 is a transmembrane receptor and consists of an extracellular ligand-binding domain (ECD), a dimerization domain within the ECD, a transmembrane domain, an intracellular protein tyrosine kinase domain (TKD), and a C-terminal phosphorylation domain. HER3 has been found to be overexpressed in several types of cancers (e.g., breast cancer, lung cancer, pancreatic cancer, colorectal cancer, gastric cancer, head and neck squamous cell carcinoma, ovarian cancer, melanoma, prostate cancer, and bladder cancer). A correlation between the expression of HER2/HER3 and the progression from non-invasive to invasive stage has been shown.

As used herein, the term " antibody " is used in its broadest sense and includes immunoglobulins or other types of molecules that contain one or more antigen binding domains that specifically bind an antigen, be it a protein or polypeptide that exhibits binding specificity for a particular antigen. Specific examples of the antibody may include intact antibodies (e.g., classical four-chain antibody molecules), single-chain antibodies, single-domain antibodies, multispecific antibodies (e.g., bispecific antibodies), and the like. A classical antibody molecule is generally a tetramer consisting of two identical heavy chains and two identical light chains linked to each other by a disulfide bond. Heavy and light chains are

divided into variable regions (V) at the amino terminus and constant regions (C) at the carboxy terminus based on conservative differences in amino acid sequences. The variable regions are used to recognize and bind to antigens, and the constant regions (e.g., Fc fragments) are used to initiate downstream effects, such as antibody-dependent cell-mediated cytotoxicity (ADCC). In the variable regions of the heavy and light chains, the amino acid composition and arrangement order of three local regions have a higher degree of variation, which are key positions for the binding of the antibody to the antigen, and thus are also known as complementarity determining regions (CDRs). The amino acid sequences of the CDRs can be readily determined using art-recognized numbering schemes, such as Kabat, Chothia, IMGT, AbM, or Contact. In a specific embodiment, the CDRs of the antibodies described herein have been determined according to the Kabat numbering scheme. Herein, the three heavy chain complementarity determining regions are referred to as HCDR1, HCDR2 and HCDR3, respectively, and the three light chain complementarity determining regions are referred to as LCDR1, LCDR2 and LCDR3, respectively.

Antibodies can be divided into five major distinct classes, IgA, IgD, IgE, IgG, and IgM, based on the amino acid sequences of their heavy chain constant regions. These antibody types can be further divided into subclasses, e.g., IgGl, IgG2a, IgG2b, IgG3, etc., according to the size of the hinge region, the position of the interchain disulfide bond, and the difference in molecular weight. Light chains can be divided into κ and λ types according to the amino acid composition and arrangement of the light chain constant region of the antibody. The subunit structures and three-dimensional conformations of different classes of immunoglobulins are known in the art.

As used herein, the "antigen-binding fragment" of an antibody refers to an amino acid fragment in an antibody molecule that participates in antigen-specific binding, e.g., Fab, Fab', $F(ab')_2$, and the like. Those skilled in the art know how to obtain these antigen-binding fragments. For example, a classical antibody molecule can be digested with papain to give an Fab fragment, and be digested with pepsin to give an $F(ab')_2$, and an Fab' fragment is formed by cleaving the disulfide bond between the hinge regions of the F(ab') 2 by treatment with a reducing agent.

As used herein, the term "single chain fragment variable (scFv)" is composed of a heavy chain variable region and a light chain variable region of an antibody linked by a short peptide to form a peptide chain. By proper folding, the variable regions from the heavy and light chains interact through non-covalent bonds to form Fv segments, so that scFv can retain its affinity activity for antigens relatively well.

As used herein, the term " single domain antibody (sdAb) ", or also referred to as "VHH antibody", refers to an antibody molecule having antigen-binding ability and comprising a heavy chain variable region and no light chain. From a structural perspective, single-domain antibodies can also be considered to be fragments of classical four-chain antibody molecules. Single-domain antibodies were first discovered in animals of the family Camelidae. Subsequently, researchers discovered more single-domain antibodies with antigen-binding ability by screening antibody libraries (e.g., phage display libraries). Single-domain antibodies have some advantages over common antibody molecules (e.g., classical antibody molecules), for example, including but not limited to: being smaller in molecular weight, easily reaching tissues or sites that are difficult for common antibody molecules to reach when used in humans, or being able to come into contact with epitopes that are difficult for common antibody molecules to reach in proteins or polypeptides; being more stable, and being better able to tolerate, for example, changes in temperature and pH, and the effects of denaturants and proteases.

As used herein, the term "Fc fragment" refers to the handle region of the Y-shaped classical antibody molecule, i.e., the fragment crystallizable (Fc), comprising the second and third constant domains (CH2 and CH3 domains) of the heavy chain. An antibody Fc region can be obtained by hydrolyzing an antibody molecule with a proteolytic enzyme (e.g., papain). In some examples, the Fc region may comprise a hinge, a CH2 and a CH3. Dimerization between two Fc-containing polypeptides can be mediated when the Fc region comprises a hinge. The Fc fragment may be from IgG, IgM, IgD, IgE or IgA. In some examples, the Fc region is from IgG1, IgG2, IgG3, or IgG4. An "Fc fragment" also includes a variant Fc fragment from a native Fc fragment that has been altered but still retains its effector function. A "variant Fc fragment" comprises an amino acid sequence having at least one amino acid alteration from the amino acid sequence of a native Fc fragment. In some examples, the variant Fc fragment has at least one amino acid substitution, e.g., about 1 to about 10 amino acids, and preferably about 1 to about 5 amino acid substitutions, compared to the parent Fc fragment (native Fc fragment). In some examples, the variant Fc fragment Fc region has at least about 80% sequence identity, at least about 90% sequence identity, at least about 95%, at least about 96%, at least about 97%, at least about 98%, or at least about 99% sequence identity to the parent Fc fragment. Effector functions of the "Fc fragment" may include binding to Fc receptors, Clq binding and complement-dependent cytotoxicity (CDC), antibody-dependent cell-mediated cytotoxicity (ADCC), mediating phagocytosis, and the like.

As used herein, the term "murine antibody" refers to an antibody in which the variable and constant regions, if any, are derived from mouse or rat immunoglobulin sequences. Murine antibodies may conveniently be obtained by immunizing a mouse or rat with the corresponding antigen and isolating the antibody of interest therefrom. Alternatively, it can be obtained by isolating and culturing cells expressing the antibody of interest (e.g., B cells) after immunizing mice or rats with the corresponding antigen. Alternatively, after immunizing a mouse or rat with the corresponding antigen, cells expressing the antibody of interest are isolated and cultured, and fused with immortalized cells such as myeloma cells to obtain hybridoma cells, which can be cultured to obtain the antibody of interest (e.g., monoclonal antibody) in large

quantities over a long period of time. In some embodiments, the "murine antibody" is a mouse antibody. "Humanized antibody" refers to a chimeric antibody obtained by artificially engineering a non-human antibody, i.e., an antibody whose variable regions and constant regions (if any) are not derived from a human immunoglobulin, to incorporate the amino acid sequence of a human antibody. The humanized antibody may comprise a constant region and/or a framework region of a human antibody. Humanized antibodies can be obtained by genetic engineering means, for example, by replacing the constant region of a murine antibody with the constant region of a human antibody and/or replacing the framework region of a murine antibody with the framework region of a human antibody. Such humanization generally does not affect the binding specificity of the original antibody to the corresponding antigen, and thus such antigens are also included within the scope of the present invention.

As used herein, the term "monoclonal antibody" refers to a homogeneous antibody directed against a particular antigenic epitope. In contrast to polyclonal antibodies, which typically include different antibodies directed against different antigenic determinants (epitopes), each monoclonal antibody is directed against a single antigenic determinant on the antigen. The modifier "monoclonal" indicates the uniform character of the antibody and is not to be construed as requiring production of the antibody by any particular method. The monoclonal antibodies of the present invention may be produced by hybridoma methods or recombinant DNA methods well known in the art, or obtained by screening methods described elsewhere herein.

As used herein, the term "immunoconjugate" refers to an antibody or antigen-binding fragment thereof to which an additional chemical group or peptide fragment is attached. For the purpose of the present invention, the chemical group or peptide fragment may facilitate the detection of the antibody or the antigen-binding fragment thereof, or facilitate the detection of an immune complex (i.e., antigen-antibody complex) formed by the antibody or the antigen-binding fragment thereof and a corresponding antigen (e.g., collagen type IV $\alpha$1 chain), and thus may comprise a "detectable label". A "detectable label" may be used to indicate the presence or amount of the corresponding antigen in a sample, or to track the location of the corresponding antigen within a cell or body. Examples of detectable labels include various enzymes that can be used in immunoassays, such as horseradish peroxidase (HRP) and alkaline phosphatase (ALP); fluorophores (such as FAM and FITC) or fluorescent proteins (such as GFP); and radioisotopes (such as 3H, 14C, and 35S). When the detectable label is an enzyme, the presence or amount of the antibody or the antigen-binding fragment thereof linked to the enzyme can be determined by the enzymatic activity of the enzyme.

As used herein, the term "purification tag" refers to an amino acid sequence that facilitates the isolation of a polypeptide or protein of interest from a cell culture or supernatant in which it is expressed. Examples thereof include, but are not limited to, a His6 tag, a Flag tag, an MBP tag, a GST tag, a SUMO tag, and the like.

For an antibody or an antigen-binding fragment thereof, "binding," "directed against," or "specifically binding" means that one molecule (e.g., an antibody or an antigen-binding fragment thereof) has a higher binding affinity for another molecule (e.g., an antigen) relative to other molecules present in the environment at the same time. One molecule may bind to, be directed against, or specifically bind to more than one molecule. For example, a bispecific antibody may have higher binding affinity for two different antigens relative to other molecules. The binding affinity of an antibody for an antigen can be measured by a number of parameters, such as the EC50 value or KD value for the binding of the antibody to the antigen.

$EC_{50}$ (concentration for 50% of maximal effect) refers to the concentration that causes 50% of the maximal effect. When used in an enzyme-linked immunosorbent assay (ELISA) to indicate the binding ability of an antibody molecule to a corresponding antigen, it may refer to the concentration of the antibody molecule at which half of the maximum detection signal (e.g., colorimetric or fluorescence intensity) is generated. The lower the EC50 value, the greater the binding affinity to the antigen.

The KD value can also be used to measure the binding affinity between an antibody and its antigen. The KD value is the equilibrium dissociation constant between the antibody and its antigen, i.e., the ratio of koff/kon. Therefore, the lower the KD value (the lower the concentration), the higher the affinity of the antibody.

As used herein, the terms "polypeptide" and "protein" are used interchangeably and refer to a polymer of amino acid residues. Such polymers of amino acid residues may contain natural or unnatural amino acid residues and include, but are not limited to, peptides, oligopeptides, dimers, trimers, and multimers composed of amino acid residues. Both full-length proteins and fragments thereof are encompassed by this definition. The term also includes post-expression modifications of the polypeptide, such as glycosylation, sialylation, acetylation, phosphorylation, and the like. Furthermore, for purposes of the present invention, a "polypeptide" refers to a protein that includes modifications, such as deletions, additions, and substitutions (generally conservative in nature) to the native sequence, so long as the protein retains the desired activity. These modifications may be deliberate, as induced via site-directed mutagenesis, or may be accidental, such as via mutation of the host producing the protein or error due to PCR amplification.

As used herein, the term "functional variant" refers to a variant molecule obtained by introducing one or more amino acid insertions, deletions or substitutions on the basis of a parent protein molecule (e.g., a native protein molecule), which still retains at least part of the function (especially the function of interest, such as the ability to bind to a corresponding antigen) of the parent protein molecule. For example, a functional variant of an antibody molecule may

retain at least 20%, 30%, 40%, 50%, 60%, 70%, 80%, or 90% of the antigen-binding ability of its parent molecule, or even have higher binding ability than the parent molecule. In some embodiments, the functional variant of the antibody molecule may retain at least 80%, 85%, 90%, 95%, or even 100% or more of the antigen-binding affinity of its parent molecule. For the antibody molecule or the antigen-binding fragment thereof, the functional variant generally comprises amino acid changes in the variable region framework sequence and/or the constant region, but it is not excluded that one or a few amino acid changes can be made to the CDR region sequence.

As used herein, the terms "nucleic acid molecule," "nucleic acid," and "polynucleotide" are used interchangeably to refer to a polymer of nucleotides. Such nucleotide polymers may contain natural and/or non-natural nucleotides and include, but are not limited to, DNA, RNA, and PNA. A "nucleic acid sequence" refers to a linear sequence of nucleotides contained in a nucleic acid molecule or polynucleotide.

As used herein, the term "vector" refers to a nucleic acid molecule that can be engineered to contain a polynucleotide of interest (e.g., a coding sequence of a polypeptide of interest) or a nucleic acid molecule (e.g., a nucleic acid, a plasmid, or a virus) that can replicate in a host cell. The vector may comprise one or more of the following components: an origin of replication, one or more regulatory sequences (such as a promoter and/or an enhancer) that regulate the expression of the polynucleotide of interest, and/or one or more selectable marker genes (such as an antibiotic resistance gene and a gene useful in colorimetric analysis, e.g., β-galactose). The term "expression vector" refers to a vector for expressing a polypeptide of interest in a host cell.

As used herein, the term "host cell" refers to a cell which may be or has been the recipient of a vector or isolated polynucleotide. The host cell may be prokaryotic or eukaryotic. Exemplary eukaryotic cells include mammalian cells, such as primate or non-primate animal cells; fungal cells, such as yeast; plant cells; and insect cells. Non-limiting exemplary mammalian cells include, but are not limited to, CHO cells, HEK-293 cells, BHK cells, or PER-C6 cells, and derivative cells thereof, such as 293-6E, CHO-DG44, CHO-K1, CHO-S, and CHO-DS cells. Host cells include progeny of a single host cell, and the progeny may not necessarily be completely identical (in morphology or in genomic DNA complement) to the original parent cell due to natural, accidental, or deliberate mutation. Host cells may be isolated cells or cell lines, also including cells transfected in vivo with a nucleic acid molecule or expression vector provided herein.

When referring to amino acid or nucleotide sequences, the term "sequence identity" (also referred to as "sequence identity") refers to the amount of identity between two amino acid or nucleotide sequences (e.g., a query sequence and a reference sequence), generally expressed as a percentage. Generally, sequence alignment and introduction of gaps (if any) are performed prior to calculating the percent identity between two amino acid or nucleotide sequences. If the amino acid residues or bases in the two sequences are identical at a certain alignment position, the two sequences are considered to be identical or matched at that position; if the amino acid residues or bases in the two sequences are different, the two sequences are considered to be inconsistent or mismatched at that position. In some algorithms, the number of matched positions is divided by the total number of positions in the alignment window to obtain sequence identity. In other algorithms, the number of gaps and/or the length of the gaps are also taken into account. Commonly used alignment algorithms or software include DANMAN, CLUSTALW, MAFFT, BLAST, MUSCLE, and the like. For the purpose of the present invention, the published alignment software BLAST (available from https://www.ncbi.nlm.nih.gov/) can be employed to obtain an optimal sequence alignment and calculate the sequence identity between two amino acid or nucleotide sequences by using default settings.

As used herein, the terms "cancer" and "cancerous" refer to or describe the physiological condition in mammals in which a population of cells is characterized by unregulated cell growth. As used herein, the terms "cancer cells" and "tumor cells" refer to the total number of cells from a tumor, including tumorigenic stem cells (cancer stem cells) and non-tumorigenic cells that make up the majority of the tumor cell population. Examples of cancer include, but are not limited to, carcinoma, lymphoma, blastoma, sarcoma, and leukemia. More specific examples of such cancers include squamous cell carcinoma, lung cancer such as small-cell lung cancer, non-small cell lung cancer, lung adenocarcinoma, squamous cell lung cancer, peritoneal cancer, hepatocellular carcinoma, gastric cancer, gastrointestinal cancer, pancreatic cancer, glioblastoma, cervical cancer, ovarian cancer, liver cancer, bladder cancer, breast cancer, colon cancer, colorectal cancer, endometrial or uterine carcinoma, salivary gland carcinoma, kidney cancer, prostate cancer, vulval cancer, thyroid cancer, melanoma, head and neck squamous cell carcinoma, and various types of head and neck cancer.

As used herein, the term "tumor" refers to any mass of tissue resulting from cellular overgrowth or proliferation, whether benign (noncancerous) or malignant (cancerous), including precancerous lesions.

The terms "cancer" and "tumor" are used interchangeably herein and include solid tumors and hematological tumors.

As used herein, the term "metastasis" refers to the process by which a cancer spreads or metastasizes from the site of origin to other areas of the body as a similar cancerous lesion occurs in a new location. A "metastatic" cell is a cell that has lost adhering contact with neighboring cells and has migrated through the blood or lymph from the primary site of disease to invade neighboring body structures.

As used herein, the term "subject" refers to any animal (e.g., a mammal), including, but not limited to, humans, non-human primates, rodents, and the like, who is to be the recipient of a particular treatment. Generally, the terms

"subject" and "patient" are used interchangeably herein to refer to a human subject.

As used herein, the terms "treat," "treating," and "prevention" refer to: 1) curing, slowing, alleviating symptoms, and/or stopping the progression of a diagnosed pathological condition or disease; 2) a pre-implemented method of stopping, delaying, or slowing the progression of a target pathological condition or disease. Thus, persons in need of treatment include those already with the disease; those prone to the disease; and those in need of prevention. A subject is successfully "treated" according to the methods of the present disclosure if the patient exhibits one or more of the following: reduction or complete absence of cancer cells; reduction in tumor size; inhibition or absence of cancer cell infiltration into peripheral organs (these include spread of cancer cells to soft tissue and bone); inhibition or absence of tumor metastasis; inhibition or absence of tumor growth; alleviation of one or more symptoms associated with the particular cancer; reduction in morbidity and mortality; and improvement in quality of life. In the context applicable to cancer, the term "prevention" refers to the administration of a medical means, such as a drug, to a subject, particularly a subject at risk of cancer, prior to the appearance of a symptom or pathological condition associated with the cancer.

As used herein, the term "antibody-drug conjugate" refers to a targeted biological drug obtained by linking a bioactive compound fragment (drug molecule) to an antibody or an antigen-binding fragment thereof portion via a linking unit. The linking unit, also known as a linker, can be cleaved in a specific environment (e.g., an intracellular low pH environment) or under a specific action (e.g., the action of a lysosomal protease), thereby separating the biologically active compound fragment from the antibody or the antigen-binding fragment thereof. ADCs can utilize the high affinity and specificity of target antibodies to recognize tumor cell surface antigens, and deliver bioactive compounds, such as small molecule cytotoxic drugs, to tumor cells through strong intracellular internalization, thereby achieving accurate and efficient killing of tumor cells.

As used herein, the term "linker" refers to a fragment that links a biologically active compound fragment (drug molecule) to a portion of an antibody or an antigen-binding fragment thereof. It should be understood that the linker has a functional group that can form a bond with a functional group of the antibody or the antigen-binding fragment thereof before being linked to the antibody or the antigen-binding fragment thereof.

The term "linker-payload" refers to a compound formed by linking a payload, e.g., a drug (e.g., a small molecule drug), to a linker.

As used herein, the term "biologically active compound fragment" refers to a biologically active drug, such as a small molecule cytotoxic drug, that can be formed from a biologically active compound fragment in an antibody-drug conjugate (or antibody-drug conjugate, ADC) after cleavage/degradation/enzyme digestion of a linker between tumor tissues or within tumor cells, which is well known in the art.

As used herein, the term " DAR (drug to antibody ratio) ", also known as "drug-to-antibody ratio", is also referred to herein as "connection number", and refers to the number of drug moieties conjugated to an antibody in an antibody-drug conjugate molecule. The DAR can vary and will be limited by the number of available sites on the antibody. It should be understood that the DAR or "connection number" should be a natural number greater than 0, i.e., a positive integer. In some embodiments, the antibody-drug conjugate of the present disclosure has a DAR of 1-15, e.g., 1-14, 2-13, 3-12, 4-11, 5-10, 6-9, or 7-8, e.g., 1, 2, 3, 4, 5, 6, 7, 8, 9, 10, 11, 12, 13, 14, or 15, and e.g., a range with any two values of 1, 2, 3, 4, 5, 6, 7, 8, 9, 10, 11, 12, 13, 14, and 15 as endpoints. During the conjugation process, a heterogeneous mixture of different antibody-drug conjugate (ADC) molecules (i.e., ADC molecules with different DARs) is generally produced. Therefore, the term " antibody-drug conjugate (ADC) " also refers to such a mixture of ADC molecules with different DARs. The term " average DAR " refers to the average DAR of a population of ADC molecules in such a mixture, which is also referred to as the "average connection number" in the present disclosure. As is well known in the art, DAR and drug loading distribution can be determined, for example, by using hydrophobic interaction chromatography (HIC) or reversed phase high-performance liquid chromatography (RP-HPLC), wherein HIC is particularly suitable for determining average DAR. In some embodiments, the average DAR (average connection number) of the antibody-drug conjugate described herein is a value between 1-15, e.g., a value in the range of 1-14, 2-13, 3-12, 4-11, 5-10, 6-9, or 7-8, e.g., a value in the range of values with any two values of 1, 2, 3, 4, 5, 6, 7, 8, 9, 10, 11, 12, 13, 14, and 15 as endpoints; it should be understood that the "value" includes integers and decimals. A numerical value of 1-15 refers to any numerical value between 1 and 15, including integers and decimals, and including end values. In some embodiments, the average DAR (average connection number) is, for example, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8.0, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0, or is selected from two values in a range having these values as endpoints.

The term "pharmaceutical combination" refers to a non-fixed combination product or a fixed combination product. The term "non-fixed combination" means that the active ingredients (e.g., (i) the antibody-drug conjugate, the stereoisomer thereof, or the pharmaceutically acceptable salt or solvate thereof of the present invention and (ii) an additional therapeutic agent) are administered to a patient as separate entities simultaneously, without specific time limitation, or sequentially at identical or different time intervals, wherein such administration provides two or more prophylactically or

therapeutically effective active agents in the patient. In some embodiments, the antibody-drug conjugate, the stereoisomer thereof, or the pharmaceutically acceptable salt or solvate thereof of the present invention and the additional therapeutic agent used in the pharmaceutical combination are administered at levels that do not exceed their levels when used alone. The term "fixed combination" means that two or more active agents are administered to a patient simultaneously in the form of a single entity. The dosages and/or time intervals for the two or more active agents are preferably selected so that the combined use of the parts will produce a greater effect in the treatment of the disease or condition than can be achieved by the use of either component alone. The ingredients may each be in a separate formulation form, which may or may not be the same.

The term "immunomodulatory agent," as used herein, refers to a natural or synthetic active agent or drug that inhibits or modulates (e.g., activates) an immune response. The immune response may be a humoral response or a cellular response. Immunomodulatory agents include immunosuppressants. In some embodiments, the immunomodulatory agent of the present invention includes an immune checkpoint inhibitor or an immune checkpoint agonist.

"Chemotherapeutic agents" include chemical compounds useful in the treatment of cancer or immune system diseases.

The term "small molecule drug" refers to a low molecular weight organic compound capable of modulating a biological process, in particular altering or preventing a pathological process. "Small molecule" is defined as a molecule having a molecular weight of less than 10 kD, usually less than 2 kD and preferably less than 1 kD, more preferably less than 500 D. Small molecule drugs include, but are not limited to, organic molecules, organic molecules containing inorganic components, molecules containing radioactive atoms, synthetic molecules, peptidomimetics, and antibody mimetics. As therapeutic agents, small molecules may be more permeable to cells, less susceptible to degradation, and less likely to elicit an immune response than large molecules.

The term "alkyl" as used herein refers to a fully saturated branched or unbranched hydrocarbon group. The alkyl group preferably comprises 1-16 carbon atoms, e.g., 1-12 carbon atoms, 1-10 carbon atoms, 1-6 carbon atoms, or 1-4 carbon atoms. Representative examples of alkyl include, but are not limited to, methyl, ethyl, n-propyl, iso-propyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl and the like.

The term "alkylene" refers to an alkyl group as defined above, but which is divalent, i.e., having two single bonds to the other two groups. Non-limiting examples of alkylene include $-CH_2-$, $-CH_2CH_2-$, $-CH_2CH_2CH_2-$, $-CH_2CH_2CH_2CH_2-$, $-CH(-CH_2CH_3)-$, or $-CH_2CH(-CH_3)-$.

The term "alkenyl" refers to a straight or branched chain hydrocarbon group containing from 2 to 16 carbon atoms and containing at least one double bond and no triple bonds. Alkenyl groups preferably contain 2-12 carbon atoms, 2-10 carbon atoms, 2-8 carbon atoms, 2-6 carbon atoms, or 2-4 carbon atoms. Representative examples of alkenyl include, but are not limited to, ethenyl, propenyl, butenyl, pentenyl, hexenyl, and the like.

The term "alkynyl" refers to a straight or branched chain hydrocarbon group containing from 2 to 16 carbon atoms and containing at least one triple bond. Alkynyl groups preferably contain 2-12 carbon atoms, 2-10 carbon atoms, 2-8 carbon atoms, 2-6 carbon atoms or 2-4 carbon atoms. Representative examples of alkynyl include, but are not limited to, ethynyl, propynyl, butynyl, pentynyl, hexynyl, and the like.

The term "halogen" or "halo" refers to fluorine (-F), chlorine (-Cl), bromine (-Br), and iodine (-I).

The term "haloalkyl" refers to an alkyl group, as defined herein, substituted with one or more halo groups, as defined herein. Haloalkyl may preferably be monohaloalkyl, dihaloalkyl or polyhaloalkyl (including perhaloalkyl). Monohaloalkyl groups may contain one iodo, bromo, chloro or fluoro in the alkyl. Dihaloalkyl and polyhaloalkyl groups may contain two or more of the same halo atoms or a combination of different halo groups in the alkyl group. Preferably, the polyhaloalkyl groups contain up to 12, 10 or 8 or 6 or 4 or 3 or 2 halogen groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, and dichloropropyl. A perhaloalkyl group refers to an alkyl group in which all of the hydrogen atoms are replaced with halogen atoms.

The term "haloalkenyl" refers to an alkenyl group, as defined herein, substituted with one or more halo groups, as defined herein. The term "haloalkynyl" refers to an alkynyl group, as defined herein, substituted with one or more halo groups, as defined herein. The meaning of "halo" defined with respect to "haloalkyl" is applicable to both "haloalkenyl" and "haloalkynyl".

The term "polyol group" refers to an alkyl group as defined above containing multiple (e.g., 2-10, e.g., 3, 4, 5, 6, 7, or 8) hydroxyl groups, optionally containing one or more (e.g., 2, 3, or 4) other groups (e.g., amino, carbonyl). Non-limiting examples of "polyol group" include, for example,

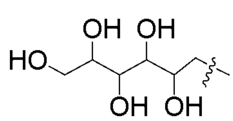 , 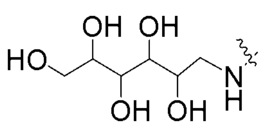 , 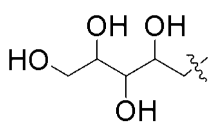 , or 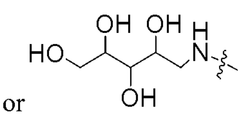 ,

wherein, a chiral center without a stereo configuration indicated may be in R or S configuration, preferably

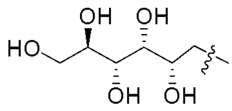 or 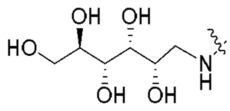 .

The term "amino acid" refers to naturally occurring and synthetic amino acids. The amino acids may be L or D isomers. The writing of conventional amino acids referred to herein follows conventional usage. See, e.g., Immunology-A Synthesis (2nd Edition, E. S. Golub and D. R. Gren, Eds., Sinauer Associates, Sunderland, Mass. (1991)), herein incorporated by reference. In addition, in the present disclosure, amino acids are generally represented by single-letter and three-letter abbreviations well known in the art. For example, the amino acid may be selected from the group consisting of phenylalanine (Phe; F), tyrosine (Tyr; Y), leucine (Leu; L), glycine (Gly; G), alanine (Ala; A), valine (Val; V), lysine (Lys; K), citrulline (Cit), serine (Ser; S), glutamic acid (Glu; E), aspartic acid (Asp; D), asparagine (Asn), isoleucine (Ile), arginine (Arg), proline (Pro), and glutamine (Gln).

The "anti-tumor compound" is a pharmaceutically active compound having an effect on a tumor, including, but not limited to, cytotoxic or chemotherapeutic agents, especially small molecule cytotoxic or chemotherapeutic agents, such as camptothecin compounds, e.g., those disclosed in WO2021/173773, WO2022180581, CN 102574866, etc., exatecan (topoisomerase I inhibitor Exatecan), Dxd (a novel topoisomerase I inhibitor Exatecan derivative); auristatin compounds, e.g., monomethyl auristatin E (MMAE); or maytansinoid compounds, e.g., small molecule microtubule inhibitor DM1. It should be understood that the anti-tumor compound may be substituted with isotopes including, but not limited to, e.g., deuterium, tritium, etc. For example, after a carbon-deuterium bond is substituted for a carbon-hydrogen bond, since the former is more stable than the latter, the substitution can directly affect properties such as absorption, distribution, metabolism, and excretion of certain drugs, thereby improving the efficacy, safety, and tolerability of the drugs. Therefore, the "anti-tumor compound" of the present application may encompass compounds substituted with deuterium.

"Substituted with deuterium" means that hydrogens in the molecule are replaced with deuterium, for example, one or more hydrogens, e.g., 1-10 (e.g., 1, 2, 3, 4, 5, 6, 7, 8, 9, or 10) hydrogens, are replaced with deuterium.

## II. Antibody-drug conjugate

The present disclosure provides an antibody-drug conjugate having formula (I), or a stereoisomer or pharmaceutically acceptable salt or solvate thereof:

$$Ab\text{-}(L\text{-}D)_n \qquad (I)$$

wherein,

Ab is an antibody or an antigen-binding fragment thereof that binds to HER3, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein the HCDR1, the HCDR2, the HCDR3, the LCDR1, the LCDR2, and the LCDR3 are determined by Kabat numbering scheme, respectively, wherein
HCDR1 comprises or consists of an amino acid sequence of GYYWS (SEQ ID NO: 1);
HCDR2 comprises or consists of an amino acid sequence of EINHSGSTNYNPSLKS (SEQ ID NO: 2);
HCDR3 comprises or consists of an amino acid sequence of DKWTWYFDL (SEQ ID NO: 3);
LCDR1 comprises or consists of an amino acid sequence of RSSQSVLYSSSNRNYLA (SEQ ID NO: 4);
LCDR2 comprises or consists of an amino acid sequence of WASTRES (SEQ ID NO: 5);
LCDR3 comprises or consists of an amino acid sequence of QQYYSTPRT (SEQ ID NO: 6);
L is a linker having the following structure: $-Z-E-NH-CH_2-$,
wherein Z is linked to Ab, and $-CH_2-$ is linked to D;
Z is selected from

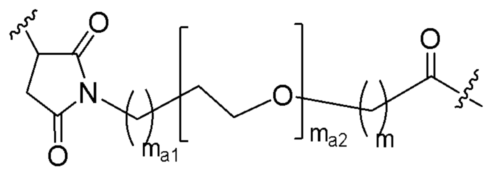

,

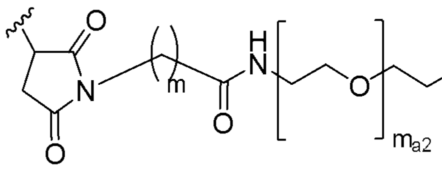, and 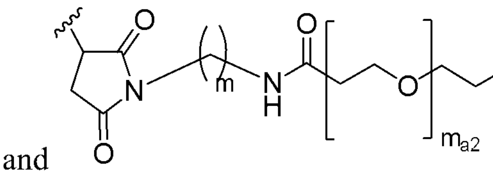 ;

wherein $m_{a1}$ and $m_{a2}$ are independently selected from an integer of 0-20, for example, 0, 1, 2, 3, 4, 5, 6, 7, 8, 9, 10, 11, 12, 13, 14, 15, or 16;

m is selected from an integer of 1-10, e.g., 1, 2, 3, 4, 5, 6, 7, or 8;

preferably

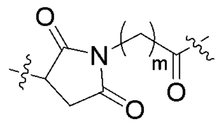,

wherein m is an integer of 1-10, e.g., 1, 2, 3, 4, 5, 6, 7, or 8, preferably 5; a carbonyl group at the right end thereof is covalently linked to E;

E is a peptide residue having 2-10 amino acids, wherein the peptide residue is optionally substituted with one or more (e.g., 2, 3, or 4) groups selected from $C_{1-6}$ alkyl and a polyol group, wherein the N-terminus of the peptide residue is covalently linked to Z;

D is a cytotoxic compound having a structure of formula $-Q-L^2-L^1-D^1$,

wherein Q is-O- or -S-;

$L^1$ is absent or -($C_1$ -$C_{10}$ alkylene)-;

$L^2$ is absent, *-($C_1$ -$C_{10}$ alkylene)-C(O)N($R^5$)- or *-($C_1$ -$C_{10}$ alkylene)-N($R^5$)C(O)-; wherein * indicates that the end is covalently linked to Q; and $R^5$ is H or $C_1$ -$C_6$ alkyl,

wherein $D^1$ has a structure of formula (D-1):

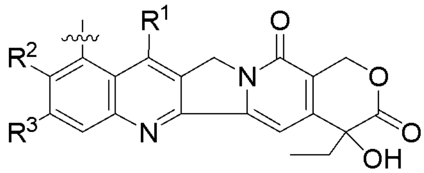 (D-1)

wherein $R^1$ is selected from H, $C_1$-$C_6$ alkyl, $C_2$-$C_6$ alkenyl, $C_2$-$C_6$ alkynyl, $C_1$-$C_6$ haloalkyl, $C_2$-$C_6$ haloalkenyl and $C_2$-$C_6$ haloalkynyl;

$R^2$ is selected from H, halogen, $C_1$-$C_6$ alkyl, $C_1$-$C_6$ haloalkyl, $-OR^4$ and $-SR^4$; $R^3$ is selected from H, halogen, CN, $C_1$-$C_6$ alkyl, $C_1$-$C_6$ haloalkyl and $-OR^4$; or $R^2$ and $R^3$ together form - $O(CH_2)_{n1}$ O- or $-O(CF_2)_{n1}O-$, wherein n1 is 1 or 2;

$R^4$ is selected from H and $C_1$-$C_4$ alkyl; and

n represents DAR, and n is a natural number selected from 1-15, e.g., a natural number in the range of 1-14, 2-13, 3-12, 4-11, 5-10, 6-9, or 7-8, e.g., a natural number in the range of a numerical value with any two of the numerical values of 1, 2, 3, 4, 5, 6, 7, 8, 9, 10, 11, 12, 13, 14, and 15 as endpoints; for example, n is 1, 2, 3, 4, 5, 6, 7, 8, 9, or 10.

It is understood that a peptide residue of 2-10 amino acids refers to a peptide residue consisting of 2-10 amino acid residues.

In some embodiments, in the antibody-drug conjugate of formula (I) of the present disclosure, the Ab comprises a heavy chain variable region ($V_H$), wherein the heavy chain variable region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 7; or

(ii) comprises or consists of the amino acid sequence set forth in SEQ ID NO: 7; or

(iii) comprises or consists of an amino acid sequence having one or more amino acid substitutions, insertions or deletions compared with the amino acid sequence set forth in SEQ ID NO: 7, wherein optionally the amino acid substitutions, insertions or deletions do not occur in the CDRs, wherein the number of amino acid changes (i.e., substitutions, insertions or deletions ) is not more than 10, e.g., not more than 9, 8, 7, 6, 5, 4, 3, 2 or 1, and the amino acid substitutions are, for example, conservative amino acid substitutions;

wherein the amino acid sequence set forth in SEQ ID NO: 7 is as follows:

QVQLQQWGAGLLKPSETLSLTCAVYGGSFSGYYWSWIRQPPGKGLEWIGEINHS GSTNYNPSLKSRVTISVETSKNQFSLKLSSVTAADTAVYYCARDKWTWYFDLWGRGT LVTVSS (SEQ ID NO: 7).

The nucleic acid sequence encoding the amino acid sequence is as follows:

CAGGTGCAGCTGCAGCAGTGGGGCGCCGGACTGCTGAAGCCCAGCGAGACAC TGAGCCTGACCTGCGCCGTGTACGGAGGCTCCTTCAGCGGATACTACTGGAGCTGG ATCAGACAGCCACCCGGCAAAGGACTGGAATGGATTGGAGAGATCAACCACAGC GGAAGCACCAACTACAACCCCTCTCTGAAGTCCAGAGTGACCATCAGCGTGGAGA CCTCAAAGAACCAGTTTAGCCTGAAGCTGAGCTCTGTGACTGCCGCCGACACCGCC GTGTACTACTGCGCCCGCGACAAATGGACCTGGTACTTCGACCTGTGGGGCAGAG GCACCCTGGTGACCGTGAGCAGC (SEQ ID NO: 8)

In some embodiments, in the antibody-drug conjugate of formula (I) of the present disclosure, the Ab comprises a light chain variable region ($V_L$), wherein the light chain variable region

(i) comprises or consists of an amino acid sequence having at least 90%, 91%, 92%, 93%, 94%, 95%, 96%, 97%, 98%, or 99% identity to the amino acid sequence set forth in SEQ ID NO: 9; or
(ii) comprises or consists of the amino acid sequence set forth in SEQ ID NO: 9; or
(iii) comprises or consists of an amino acid sequence having one or more amino acid substitutions, insertions or deletions compared with the amino acid sequence set forth in SEQ ID NO: 9, wherein optionally the amino acid substitutions, insertions or deletions do not occur in the CDRs, wherein the number of amino acid changes (i.e., substitutions, insertions or deletions ) is not more than 10, e.g., not more than 9, 8, 7, 6, 5, 4, 3, 2 or 1, and the amino acid substitutions are, for example, conservative amino acid substitutions;

The amino acid sequence set forth in SEQ ID NO: 9 is as follows:

DIEMTQSPDSLAVSLGERATINCRSSQSVLYSSSNRNYLAWYQQNPGQPPKLLIY WASTRESGVPDRFSGSGSGTDFTLTISSLQAEDVAVYYCQQYYSTPRTFGQGTKVEIK (SEQ ID NO: 9).

The nucleic acid sequence encoding the amino acid sequence is as follows:

GACATTGAAATGACCCAGAGCCCCGACAGCCTGGCCGTGAGCCTGGGAGAGA GAGCCACCATCAACTGCAGAAGCAGCCAGAGCGTGCTGTACAGCAGCAGCAACAG AAACTACCTGGCCTGGTATCAACAGAATCCCGGCCAGCCCCCCAAACTGCTGATCT ACTGGGCCTCCACCCGGGAAAGCGGCGTGCCAGACAGATTCAGCGGCAGCGGCAG CGGAACCGACTTCACCCTGACCATCAGCAGCCTGCAGGCTGAGGACGTGGCCGTG TACTACTGCCAGCAGTACTACTCTACCCCCAGAACCTTCGGCCAGGGCACCAAAGT GGAGATCAAG (SEQ ID NO: 10)

In some embodiments, in the antibody-drug conjugate of formula (I) of the present disclosure, the Ab further comprises a heavy chain constant region ($C_H$), and the heavy chain constant region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 11; or
(ii) comprises or consists of the amino acid sequence set forth in SEQ ID NO: 11; or
(iii) comprises or consists of an amino acid sequence having one or more amino acid substitutions, insertions or deletions compared with the amino acid sequence set forth in SEQ ID NO: 11, wherein the number of amino acid changes (i.e., substitutions, insertions or deletions ) is not more than 10, e.g., not more than 9, 8, 7, 6, 5, 4, 3, 2 or 1, and the amino acid substitutions are, for example, conservative amino acid substitutions;

the amino acid sequence set forth in SEQ ID NO: 11 is as follows,

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 11).

In some embodiments, in the antibody-drug conjugate of formula (I) of the present disclosure, the Ab further comprises a light chain constant region ($C_L$), and the light chain constant region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 12; or
(ii) comprises or consists of the amino acid sequence set forth in SEQ ID NO: 12; or
(iii) comprises or consists of an amino acid sequence having one or more amino acid substitutions, insertions or deletions compared with the amino acid sequence set forth in SEQ ID NO: 12, wherein the number of amino acid changes (i.e., substitutions, insertions or deletions ) is not more than 10, e.g., not more than 9, 8, 7, 6, 5, 4, 3, 2 or 1, and the amino acid substitutions are, for example, conservative amino acid substitutions;

The amino acid sequence set forth in SEQ ID NO: 12 is as follows:

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 12).

In some embodiments, in the antibody-drug conjugate of formula (I) of the present disclosure, the Ab comprises a heavy chain (HC) comprising or consisting of the heavy chain variable region and the heavy chain constant region described herein.

In some embodiments, in the antibody-drug conjugate of formula (I) of the present disclosure, the Ab comprises a heavy chain (HC), and the heavy chain

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 13; or
(ii) comprises or consists of the amino acid sequence set forth in SEQ ID NO: 13; or
(iii) comprises or consists of an amino acid sequence having one or more amino acid substitutions, insertions or deletions compared with the amino acid sequence set forth in SEQ ID NO: 13, wherein the number of amino acid changes (i.e., substitutions, insertions or deletions ) is not more than 10, e.g., not more than 9, 8, 7, 6, 5, 4, 3, 2 or 1, and the amino acid substitutions are, for example, conservative amino acid substitutions;

the amino acid sequence set forth in SEQ ID NO: 13 is as follows:

QVQLQQWGAGLLKPSETLSLTCAVYGGSFSGYYWSWIRQPPGKGLEWIGEINHS GSTNYNPSLKSRVTISVETSKNQFSLKLSSVTAADTAVYYCARDKWTWYFDLWGRGT LVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKRVEPKSCDKTHTCPPC PAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPR EPQVYTLPPSREEMTKNQVSLTCLVKGFYPSDIAVEWESNGQPENNYKTTPPVLDSDG SFFLYSKLTVDKSRWQQGNVFSCSVMHEALHNHYTQKSLSLSPGK (SEQ ID NO: 13)

In some embodiments, in the antibody-drug conjugate of formula (I) of the present disclosure, the Ab comprises a light chain (LC) comprising or consisting of the light chain variable region and the light chain constant region described herein.

In some embodiments, in the antibody-drug conjugate of formula (I) of the present disclosure, the Ab comprises a light chain (LC), and the light chain

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 14; or
(ii) comprises or consists of the amino acid sequence set forth in SEQ ID NO: 14; or
(iii) comprises or consists of an amino acid sequence having one or more amino acid substitutions, insertions or deletions compared with the amino acid sequence set forth in SEQ ID NO: 14, wherein the number of amino acid changes (i.e., substitutions, insertions or deletions ) is not more than 10, e.g., not more than 9, 8, 7, 6, 5, 4, 3, 2 or 1, and the amino acid substitutions are, for example, conservative amino acid substitutions;

the amino acid sequence set forth in SEQ ID NO: 14 is as follows:

DIEMTQSPDSLAVSLGERATINCRSSQSVLYSSSNRNYLAWYQQNPGQPPKLLIY WASTRESGVPDRFSGSGSGTDFTLTISSLQAEDVAVYYCQQYYSTPRTFGQGTKVEIK RTVAAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC (SEQ ID NO: 14).

In some embodiments, in the antibody-drug conjugate of formula (I) of the present disclosure, the Ab specifically binds to HER3 and comprises a heavy chain variable region and/or a light chain variable region, wherein the heavy chain variable region comprises the amino acid sequence set forth in SEQ ID NO: 7, and the light chain variable region comprises then amino acid sequence set forth in SEQ ID NO: 9. In some embodiments, in the antibody-drug conjugate of formula (I) of the present disclosure, the Ab specifically binds to HER3 and comprises a heavy chain variable region and a light chain variable region, wherein the heavy chain variable region comprises or consists of the amino acid sequence set forth in SEQ ID NO: 7, and the light chain variable region comprises or consists of the amino acid sequence set forth in SEQ ID NO: 9.

In some embodiments, the Ab further comprises a heavy chain constant region and/or a light chain constant region, wherein the heavy chain constant region comprises the amino acid sequence set forth in SEQ ID NO: 11, and the light chain constant region comprises the amino acid sequence set forth in SEQ ID NO: 12. In some embodiments, the Ab further comprises a heavy chain constant region and a light chain constant region, wherein the heavy chain constant region comprises or consists of the amino acid sequence set forth in SEQ ID NO: 11, and the light chain constant region comprises or consists of the amino acid sequence set forth in SEQ ID NO: 12.

In some embodiments, in the antibody-drug conjugate of formula (I) of the present disclosure, the Ab specifically binds to HER3 and comprises a heavy chain and/or a light chain, wherein the heavy chain comprises the amino acid sequence set forth in SEQ ID NO: 13, and the light chain comprises the amino acid sequence set forth in SEQ ID NO: 14.

In some embodiments, in the antibody-drug conjugate of formula (I) of the present disclosure, the Ab specifically binds to HER3 and comprises a heavy chain and a light chain, wherein the heavy chain comprises or consists of the amino

acid sequence set forth in SEQ ID NO: 13, and the light chain comprises or consists of the amino acid sequence set forth in SEQ ID NO: 14.

In some embodiments, in the antibody-drug conjugate of formula (I) disclosed herein, the Ab is a chimeric antibody or a humanized antibody or a human antibody.

In some embodiments, Ab is a monoclonal antibody or an antigen-binding fragment thereof.

In some embodiments, $R^1$ is H, $R^2$ is $C_1$-$C_6$ alkyl or $C_1$-$C_6$ alkoxy, and $R^3$ is halogen, preferably -F.

In some embodiments, D 1 has a structure of formula (D-2):

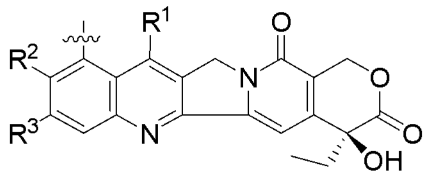
(D-2),

wherein $R^1$, $R^2$ and $R^3$ are as defined above.

In some embodiments, $D^1$ has a structure of formula (D-3):

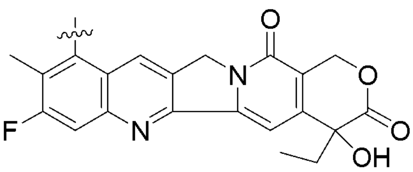
(D-3).

In some embodiments, $D^1$ has a structure of formula (D-4):

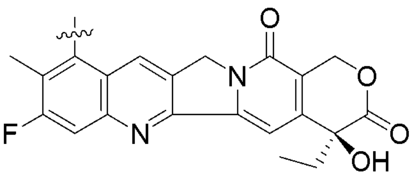
(D-4).

In some embodiments, $-L^2-L^1-$ is -($C_1$-$C_6$ alkylene)-, *-($C_1$-$C_6$ alkylene)-C(O)N($R^5$)-($C_1$-$C_6$ alkylene)- or *-($C_1$-$C_6$ alkylene)-N($R^5$)C(O)-($C_1$-$C_6$ alkylene)-, wherein * indicates that the terminus is covalently linked to Q.

$R^5$ is H or $C_1$-$C_6$ alkyl.

In some embodiments, $-L^2-L^1-$ is -($C_1$-$C_6$ alkylene)-.

In some embodiments, $-Q-L^2-L^1-$ is $-OCH_2-CH_2-CH_2-CH_2-$. It is to be understood that the left side of the group is attached to L.

In some embodiments, Z is linked to Ab, and $-CH_2-$ is linked to Q;

Z is selected from

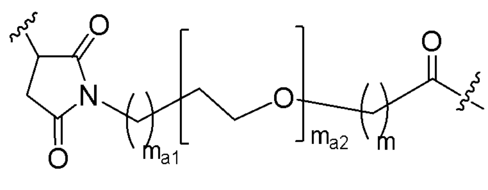
;

wherein $m_{a1}$ and $m_{a2}$ are independently selected from an integer of 0-20, for example, 0, 1, 2, 3, 4, 5, 6, 7, 8, 9, 10, 11, 12, 13, 14, 15, or 16;

m is selected from an integer of 1-10, e.g., 1, 2, 3, 4, 5, 6, 7, or 8;

preferably

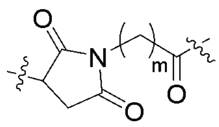
,

wherein m is an integer of 1-10, e.g., 1, 2, 3, 4, 5, 6, 7, or 8, preferably 5; a carbonyl group at the right end thereof is covalently linked to E;

In some embodiments, E is a peptide residue of 2, 3, or 4 amino acids.

In some embodiments, the amino acid is selected from, for example, glycine, alanine, valine, glutamine, glutamic acid, phenylalanine, leucine, tyrosine, lysine, citrulline, serine, tryptophan, aspartic acid, asparagine, isoleucine, arginine, and proline, and wherein the glutamine or glutamic acid is optionally substituted with one polyol group and optionally substituted with one $C_{1-6}$ alkyl. In some embodiments, the amino acid is selected from the group consisting of glycine, alanine, valine, glutamine, glutamic acid, phenylalanine and leucine, and wherein the glutamine or glutamic acid is optionally substituted with one polyol group and optionally substituted with one $C_{1-6}$ alkyl.

In some embodiments, the polyol group is selected from the group consisting of

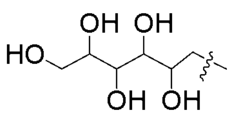
and
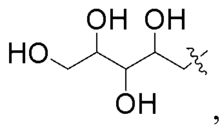
,

preferably

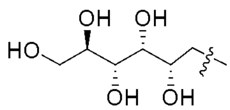
.

In some embodiments, the substituted glutamine or glutamic acid has the structure shown below:

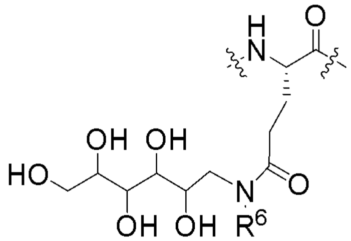
(G-1a),
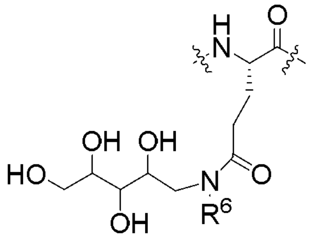
(G-1b),

wherein $R^6$ is H or $C_1$-$C_6$ alkyl;

preferably

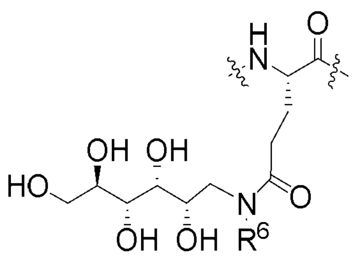
(G-1c)

wherein $R^6$ is H or $C_1$-$C_6$ alkyl.

In some embodiments, E is: -Gln-Val-Ala-, -Gly-Val-Ala-, -Gln-Phe-Ala-, -Gly-Phe-Ala-, -Gly-Gly-Phe-Gly-, -Val-Ala-, -Val-Cit-, -Ala-Ala-, -Ala-Cit-, -Ala-Lys-, -Ala-Val-, -Asn-Cit-, -Asp-Cit-, -Asn-Lys-, -Asp-Val-, -Cit-Ala-, -Cit-Asn-, -Cit-Asp-, -Cit-Cit-, -Cit-Lys-, -Cit-Ser-, -Cit-Val-, -Glu-Val-, -Glu-Gly-, -Ile-Cit-, -Ile-Pro-, -Ile-Val-, -Leu-Cit-, -Lys-Cit-, -Phe-Arg-, - Phe-Cit-, -Phe-Lys-, -Pro-Lys-, -Ser-Cit-, -Trp-Cit-, -Ala-Val-, -Val-Asp-, -Cit-Val-, -Val-Glu-, -Val-Lys-, -Gly-Gly-Gly-, -Gly-Gly-Arg-, -Phe-Lys-Gly-, -Leu-Lys-Gly-, -Leu-Leu-Gly-, -Glu-Val-Cit-, -Cit-Ala-Glu-, -Val-Lys-Gly-, -Val-Lys-Ala-, -Val-Gly-Gly-, -Val-Cit-Gly-, -Val-Gln-Gly-, -Val-Glu-Gly-, -Val-Lys-Gly-, -Val-Lys-Leu-, -Ala-Ala-Ala-, -Asn-Ala-Ala-, -Gly-Gly-Gly-Gly-, -Gly-Gly-Leu-Gly-, -Gly-Phe-Leu-Gly-, -Gly-Val-Lys-Gly-, -Ala-Leu-Ala-Leu-, - Gly-Phe-Leu-Gly-, -Ala-Leu-Ala-Leu-, -Gly-Phe-Gly-Gly-, and -Val-Lys-Gly-Gly, wherein Gln and/Glu are optionally substituted with one polyol group and optionally substituted with one $C_{1-6}$ alkyl,

preferably, the substituted Gln or Glu has the structure of formula (G-la), formula (G-1b), or formula (G-1c) as described above.

It is understood that the indicated E group is covalently linked to Z via the left amino acid (N-terminus).

In some embodiments, E is -Gln-Val-Ala-, -Gly-Val-Ala-, -Gln-Phe-Ala-, -Gly-Phe-Ala-, - Gly-Gly-Phe-Gly-, -Val-Ala-, or

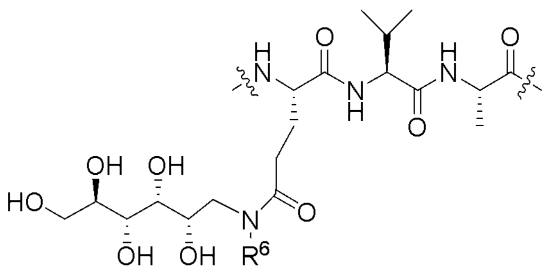

,

wherein $R^6$ is H or $C_1$-$C_6$ alkyl, wherein the E groups are covalently linked to Z through the left N-terminus.

In some embodiments, -Z-E-NH-$CH_2$ - has the following structure:

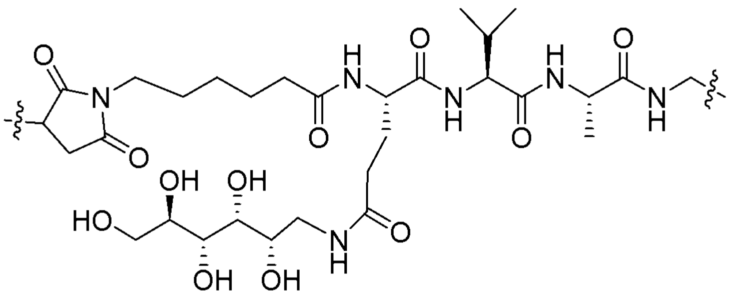

It will be appreciated that the left end of the structure is connected to the Ab moiety.

In some embodiments, in the antibody-drug conjugate of formula (I) of the present disclosure, L is a linker capable of linking the antibody moiety (Ab) to the bioactive compound moiety. In some embodiments, the linker L has the following structure:

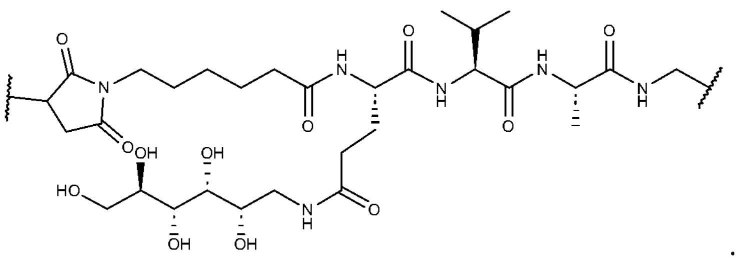

.

In some embodiments, the linker L is linked to a cysteine side chain of the antibody (Ab) via a maleimide linker, for example, via an -S- bond. In this case, the antibody-drug conjugate of formula (I) may also be represented by the following formula:

$$Ab'\text{-}(S\text{-}L\text{-}D)_n \qquad (I')$$

wherein Ab' is as defined above for Ab, and L, D and n are as defined above.

In some embodiments, in the antibody-drug conjugate of formula (I) of the present disclosure, D is a bioactive compound, e.g., an active compound that exerts a biological effect on a tumor (also known as an anti-tumor compound). The anti-tumor compound may be a cytotoxic compound. In some embodiments, the cytotoxic compound D has the following structure:

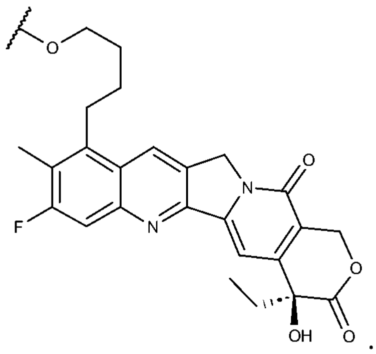

In some embodiments, the antibody-drug conjugate of the present disclosure has the following structure:

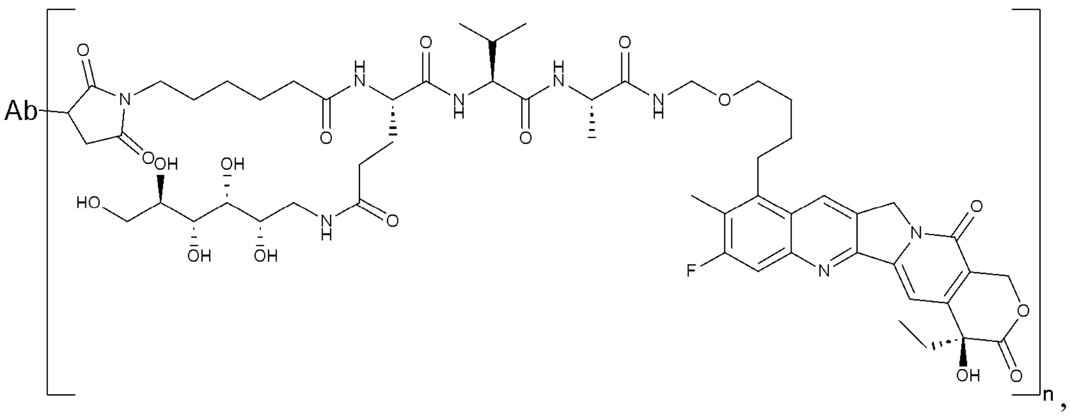

wherein Ab and n are as defined in the present disclosure.

In some embodiments, n is a natural number selected from 1-10; for example, a natural number within a numerical range with any two numerical values of 1, 2, 3, 4, 5, 6, 7, 8, 9 and 10 as endpoints, such as 6-10, 6-9, 6-8, 6-7, 7-10, 7-9, 7-8, 8-10, 8-9 or 9-10, and is, for example, 1, 2, 3, 4, 5, 6, 7, 8, 9 or 10. In some embodiments, n is 7, or n is 8, or n is 9.

In some embodiments, the antibody-drug conjugate has an average DAR of 2-10, 2-8, 3-8, 4-9, or 7.5-8.5.

The antibody-drug conjugate provided herein is described with reference to general formulas and specific compounds. In addition, the antibody-drug conjugate of the present disclosure may exist in a variety of different forms or derivatives, all of which are within the scope of the present disclosure. These include, for example, pharmaceutically acceptable salts, tautomers, stereoisomers, racemic mixtures, positional isomers, prodrugs, solvated forms, different crystalline forms or polymorphs, and active metabolites.

As used herein, unless otherwise noted, the term " pharmaceutically acceptable salts " includes salts that retain the biological effectiveness of the free acid/base form of the particular compound and which are not biologically or otherwise undesirable. Pharmaceutically acceptable salts can include salts formed with inorganic bases or acids and organic bases or acids. In the case that the antibody-drug conjugate of the present disclosure contains one or more acidic or basic groups, the present invention also includes their corresponding pharmaceutically acceptable salts. Thus, the antibody-drug conjugate of the present disclosure containing an acidic group (e.g., a carboxyl group) may exist in the form of a salt, and may be used according to the present invention, e.g., an alkali metal salt, an alkaline earth metal salt, an aluminum salt, or an ammonium salt. More non-limiting examples of such salts include lithium salts, sodium salts, potassium salts, calcium salts, magnesium salts, barium salts, or salts with ammonia or organic amines (e.g., ethylamine, ethanolamine, diethanolamine, triethanolamine, piperidine, N-methylglutamine, or amino acids). These salts are readily available, for example, by reacting a compound having an acidic group with a suitable base such as lithium hydroxide, sodium hydroxide, sodium propoxide, potassium hydroxide, potassium ethoxide, magnesium hydroxide, calcium hydroxide, or barium hydroxide. Other base salts of the antibody-drug conjugate of the present disclosure include, but are not limited to, copper (I), copper (II), iron (II), iron (III), manganese (II), and zinc salts. The antibody-drug conjugate of the present disclosure contains one or more basic groups, e.g., groups that can be protonated, may be present in the form of salts, and may be used in the form of their addition salts with inorganic or organic acids according to the present disclosure. Examples of suitable acids include hydrogen chloride, hydrogen bromide, hydrogen iodide, phosphoric acid, sulfuric acid, nitric acid, methanesulfonic acid, p-toluenesulfonic acid, naphthalenedisulfonic acid, sulfoacetic acid, trifluoroacetic acid, oxalic acid, acetic acid, tartaric acid, lactic acid, salicylic acid, benzoic acid, carbonic acid, formic acid, propionic acid, pivalic acid, diethylacetic acid, malonic acid, succinic acid, pimelic acid, fumaric acid, malonic acid, maleic acid, malic acid, pamoic acid, mandelic acid, sulfamic acid, phenylpropionic acid, gluconic acid, ascorbic acid, isonicotinic acid, citric acid, adipic acid, taurocholic acid, glutaric acid, stearic acid, glutamic acid or aspartic acid, and other acids known to those skilled in the art. Salts formed are especially hydrochlorides, chlorides, hydrobromides, bromides, iodides, sulfates, phosphates, methylsulfonates (mesylates), tosylates, carbonates, bicarbonates, formates, acetates, sulfoacetates, triflates, oxalates, malonates, maleates, succinates, tartrates, malates, pamoates, mandelates, fumarates, lactates, citrates, glutarates, stearates, aspartates and glutamates. In addition, the stoichiometry of the salt formed from the antibody-drug conjugate of the present disclosure may be an integer multiple or a non-integer multiple of 1.

Antibody-drug conjugates of the present disclosure containing basic nitrogen-containing groups can be quaternized using reagents such as $C_{1-4}$ alkyl halides, e.g., methyl, ethyl, isopropyl, and tert-butyl chlorides, bromides, and iodides; di-$C_{1-4}$ alkyl sulfates, e.g., dimethyl, diethyl, and diamyl sulfates; $C_{10-18}$ alkyl halides, e.g., decyl, dodecyl, lauryl, myristyl, and stearyl chlorides, bromides, and iodides; and aryl-$C_{1-4}$ alkyl halides, e.g., benzyl chloride and phenethyl bromide.

If the antibody-drug conjugate of the present disclosure contains both an acidic group and a basic group in the molecule, in addition to the salt forms described above, the present disclosure also includes inner salts or betaines (zwitterions). The corresponding salts can be obtained by customary methods known to those skilled in the art, for example by contacting them with an organic or inorganic acid or base in a solvent or dispersant, or by anion exchange or cation exchange with other salts. The present disclosure also includes all salts of the antibody-drug conjugate of the present disclosure, which are not directly applicable to drugs due to low physiological compatibility, but can be used, for example, as intermediates for chemical reactions or for the preparation of pharmaceutically acceptable salts. For a review of more suitable salts, see Stahl and Wermuth, Handbook of Pharmaceutical Salts: Properties, Selection, and Use (Wiley-VCH, 2002).

The antibody-drug conjugate of formula (I) and the pharmaceutically acceptable salt thereof may exist in unsolvated and solvated forms. As used herein, the term "solvate" refers to a molecular complex comprising an antibody-drug conjugate of formula (I), a stereoisomer thereof, or a pharmaceutically acceptable salt thereof, and one or more pharmaceutically acceptable solvent molecules. For example, the term "hydrate" is used when the solvent is water.

The antibody-drug conjugate of formula (I) may have one or more chiral (asymmetric) centers. The present disclosure encompasses all stereoisomeric forms of the antibody-drug conjugate of formula (I). Asymmetric centers present in the antibody-drug conjugate of formula (I) may independently have (R) or (S) configuration. When bonds to chiral carbons are depicted as straight lines in the structural formulas of the present disclosure, or when a compound name is depicted without (R) or (S) chiral designation of the chiral carbon, it is to be understood that the (*R*) and (*S*) configurations of each such chiral carbon, and thus each enantiomer or diastereomer and mixtures thereof, are included in the formula or name. The production of particular stereoisomers or mixtures thereof may be identified in the examples where such stereoisomers or mixtures are obtained, but this in no way limits the inclusion of all stereoisomers and mixtures thereof within the scope of the present disclosure. When a bond at a chiral carbon is described as a triangular solid or dashed line in the structural formulas of the present disclosure, or when a compound name is described in the context of a chiral (R) or (S) chiral name at a chiral carbon, it is to be understood that the compound represented by the structural formula or name at that time has a determined stereoconfiguration at that chiral carbon position and is to be distinguished from other stereoisomers or enantiomers or diastereomers or mixtures thereof.

The present disclosure includes all possible enantiomers and diastereomers and mixtures of two or more stereoisomers, e.g., mixtures of enantiomers and/or diastereomers in all ratios. Thus, enantiomers are the enantiomerically pure forms (as levorotatory and dextrorotatory enantiomers), the racemic forms and the mixtures of the two enantiomers in all ratios of the subject matter of the present disclosure. In the case of the cis/trans isomer, the present disclosure includes the cis form and the trans form as well as mixtures of these forms in all ratios. If desired, individual stereoisomers can be prepared by separating a mixture by conventional means, for example by chromatography or crystallization, by using stereochemically uniform synthetic starting materials, or by stereoselective synthesis. Optionally, derivatization can be performed prior to stereoisomer separation. The separation of a mixture of stereoisomers may be performed in an intermediate step during the synthesis of the antibody-drug conjugate of formula (I), or may be performed on the final racemic product. Absolute stereochemistry may be determined by X-ray crystallography of crystalline products or crystalline intermediates which are derivatized, if necessary, with reagents containing stereogenic centers of known

configuration. Alternatively, absolute stereochemistry may be determined by vibrational circular dichroism (VCD) spectroscopy.

Unless otherwise stated, structures depicted herein are also meant to include compounds that differ only in the presence of one or more isotopically enriched atoms, that is, compounds in which one or more atoms are replaced by atoms having the same atomic number, but an atomic mass or mass number different from the atomic mass or mass number that predominates in nature. Such compounds are known as "isotopic variants". The present disclosure is intended to include all pharmaceutically acceptable isotopic variants of the antibody-drug conjugate of formula (I). Examples of isotopes suitable for inclusion in the antibody-drug conjugates of the present disclosure include, but are not limited to, isotopes of hydrogen, such as $^{2}H$ (i.e., D, deuterium) and $^{3}H$ (i.e., tritium); carbon, such as $^{11}C$, $^{13}C$ and $^{14}C$; chlorine, such as $^{36}Cl$; fluorine, such as $^{18}F$; iodine, such as $^{123}I$ and $^{125}I$; nitrogen, such as $^{13}N$ and $^{15}N$; oxygen, such as $^{15}O$, $^{17}O$ and $^{18}O$; phosphorus, such as $^{32}P$; and sulfur, such as $^{35}S$. Certain isotopic variants of the antibody-drug conjugate of formula (I), such as those incorporating a radioactive isotope, are useful in drug and/or substrate tissue distribution studies. In particular, compounds having depicted structures that differ only in the substitution of heavier isotopes, for example, the substitution of deuterium ($^{2}H$ or D) for hydrogen, may afford certain therapeutic advantages, for example, greater metabolic stability, increased in vivo half-life, or reduced dosage requirements and, hence, may be useful in some particular circumstances. Isotopic variants of the antibody-drug conjugate of formula (I) can generally be prepared by conventional techniques known to those skilled in the art or by methods similar to those described in the accompanying examples using an appropriate isotopically labeled reagent in place of the non-labeled reagent previously employed for synthesis.

When a structure of a compound is described herein, and it is not specified in the structural formula that a hydrogen atom is D (i.e., $^{2}H$), it should generally be understood that the hydrogen at this position is present in the form of a hydrogen isotope "$^{1}$ hydrogen ($^{1}H$)" or in the form of a natural isotopic abundance in the natural state. When a hydrogen atom is indicated as D (i.e., $^{2}H$, deuterium) in the structure shown, it should be understood that the hydrogen at this position is present in the form of the hydrogen isotope "$^{2}$hydrogen ($^{2}H$, D, deuterium)" or in a form in which deuterium is present at an isotopic abundance greater than natural deuterium (e.g., the deuterium abundance is greater than 50%, greater than 60%, greater than 70%, greater than 80%, greater than 90%, greater than 95%, greater than 96%, greater than 97%, greater than 98%, greater than 99%, greater than 99.5%, or 100%).

Pharmaceutically acceptable solvates in accordance with the present disclosure may include those wherein the solvent of crystallization may be isotopically substituted, e.g. $D_2O$, $d_6$-acetone, $d_6$-DMSO.

## III. Use and Administration

The antibody-drug conjugate (antibody-drug conjugate of formula (I), or a stereoisomer thereof) of the present disclosure - or a pharmaceutically acceptable salt or solvate thereof, including a mixture thereof in all ratios - can be used as a medicament. They were found to exhibit pharmacological activity targeting HER3 and thereby killing cancer cells that highly express HER3. Through such activity, the antibody-drug conjugate of the present disclosure can be used to treat a disorder or disease associated with HER3 activity, such as HER3-positive tumors.

Thus, the antibody-drug conjugate of the present disclosure capable of targeting HER3 is particularly suitable for the treatment of diseases and conditions associated with HER3 activity, such as cancers and tumors, including but not limited to the following: gastric cancer, breast cancer, colorectal cancer, lung cancer, and the like.

The antibody-drug conjugate of the present disclosure may be administered in an amount effective to treat the disease or condition described herein. The antibody-drug conjugate of the present disclosure may be administered as the antibody-drug conjugate itself, or alternatively, as a pharmaceutically acceptable salt. For the purpose of administration and dosing, the antibody-drug conjugate of the present disclosure (the antibody-drug conjugate of formula (I), or the stereoisomer thereof) or the pharmaceutically acceptable salt or solvate thereof will be referred to as the antibody-drug conjugate of the present disclosure.

The antibody-drug conjugate of the present disclosure is administered by any suitable route in the form of a pharmaceutical composition suitable for such route, and is administered at a dose that is therapeutically effective as expected. The antibody-drug conjugate of the present disclosure may be administered orally, rectally, vaginally, parenterally, or topically.

As used herein, the term "administering" refers to absorbing, ingesting, injecting, inhaling, implanting, or otherwise introducing the antibody-drug conjugate or the pharmaceutical composition thereof of the present disclosure. The term "treating" refers to reversing, alleviating, delaying the onset of, or inhibiting the progression of a "pathological condition" (e.g., a disease, disorder, or condition, or one or more signs or symptoms thereof) described herein. In some embodiments, the treatment may be administered after one or more signs or symptoms of the disease or condition have developed or have been observed. In other embodiments, the treatment may be performed without signs or symptoms of the disease or condition. For example, a susceptible individual may be treated prior to the onset of symptoms (e.g., according to a history of symptoms and/or according to genetic or other susceptibility factors). Treatment may also be

continued after symptoms have resolved, e.g., to delay or prevent recurrence. As used herein, the terms "disease," "disorder," "condition," and "pathological condition" are used interchangeably.

One skilled in the art can determine the dosage level to be administered by routine experimentation. The dosage regimen for the antibody-drug conjugate and/or the composition comprising the antibody-drug conjugate of the present disclosure is based on a variety of factors, including the type, age, weight, sex, and medical condition of the patient; the severity of the condition; the route of administration; and the activity of the particular antibody-drug conjugate used. Thus, the dosage regimen can vary widely. For example, the dosage level of the antibody-drug conjugate of the present disclosure may be about 0.001 to about 100 mg/kg (i.e., mg/kg body weight) per day. In some embodiments, the total daily dose of the antibody-drug conjugate of the present disclosure administered in a single dose or in divided doses may be about 0.001 to about 10 mg/kg. It is not uncommon that the administration of the antibody-drug conjugate of the present disclosure may be repeated multiple times a day.

In some embodiments, the antibody-drug conjugate of the present disclosure may be used in combination with one or more additional therapeutic agents. In some embodiments, non-limiting examples of the additional therapeutic agent include a chemotherapeutic agent, an angiogenesis inhibitor, a cytokine, a cytotoxic agent, an additional antibody, a small molecule drug, an immunomodulatory agent, and the like. These therapeutic agents can be administered before, after, or simultaneously with the antibody-drug conjugate of the present disclosure.

## IV PHARMACEUTICAL COMPOSITION, PHARMACEUTICAL COMBINATION AND KIT

In some aspects, the present disclosure relates to a pharmaceutical composition comprising the antibody-drug conjugate of formula (I), the stereoisomer thereof, or the pharmaceutically acceptable salt or solvate thereof provided herein, and at least one pharmaceutically acceptable carrier or excipient. It is also referred to as a "pharmaceutical composition of the invention".

As used herein, the term "pharmaceutically acceptable carrier and/or excipient" refers to a carrier or excipient that can be used to prepare a pharmaceutical composition, which is generally safe, non-toxic, and neither biologically nor otherwise undesirable, and includes carriers or excipients that are acceptable for veterinary use as well as human pharmaceutical use. A pharmaceutically acceptable carrier or excipient as used herein includes one and more than one such carrier or excipient. The specific carrier or excipient used will depend on the mode and purpose for which the antibody-drug conjugate of the present disclosure is applied. Suitable carriers and excipients are well known to those skilled in the art and are described in detail in, for example, Ansel, Howard C et al., Ansel's Pharmaceutical Dosage Forms and Drug Delivery Systems. Philadelphia:Lippincott, Williams & Wilkins, 2004; gennaro, Alfonso R. et al., Remington: The Science and Practice of Pharmacy. Philadelphia:Lippincott, Williams & Wilkins, 2000; and Rowe, Raymond C. Handbook of Pharmaceutical Excipients. Chi cago, Pharmaceutical Press, 2005. One or more of a buffer, a stabilizer, a surfactant, a wetting agent, a lubricant, an emulsifier, a suspension, a preservative, an antioxidant, an opacifier, a glidant, a processing aid, a colorant, a sweetener, a flavoring agent, a diluent, and other known additives may also be included to provide therapeutic performance of the drug (i.e., the antibody-drug conjugate or the pharmaceutical composition provided herein) or to facilitate the production of a pharmaceutical product (i.e., a medicament).

The compositions of the present disclosure may be formulated in a variety of forms. These include, for example, liquid, semi-solid and solid dosage forms, such as liquid solutions (e.g., injectable and infusible solutions), dispersions or suspensions, tablets, pills, powders, liposomes, suppositories, and the like. The form depends on the mode of intended administration and the therapeutic application.

The pharmaceutical composition of the present disclosure may be prepared by any well-known pharmaceutical technique (e.g., effective formulation and administration procedure). The above considerations regarding effective formulations and administration procedures are well known in the art and are described in standard textbooks. The formulation of pharmaceutical products is discussed, for example, in Hoover, John E., Remington's Pharmaceutical Sciences, Mack Publishing Co., Easton, Pennsylvania, 1975; liberman et al., eds., Pharmaceutical Dosage Forms, Marcel Decker, New York, N.Y., 1980; and Kibbe et al., eds., Handbook of Pharmaceutical Excipients, 3rd edition, American Pharmaceutical Association, Washington, 1999.

In one aspect, the present invention also provides a pharmaceutical combination or a pharmaceutical combination product comprising the antibody-drug conjugate of formula (I), the stereoisomer thereof, or the pharmaceutically acceptable salt or solvate thereof, and one or more additional therapeutic agents. Other therapeutic agents that can be combined or administered in combination with the molecule of the present invention encompass various therapeutic agents for treating tumors, such as chemotherapeutic agents, angiogenesis inhibitors, cytokines, cytotoxic agents, other antibodies, small molecule drugs, or immunomodulatory agents (e.g., immune checkpoint inhibitors or agonists).

In yet another aspect, the present disclosure relates to a kit or kit for use in treating diseases and conditions associated with HER3 activity, e.g., cancer, comprising the antibody-drug conjugate of formula (I), the stereoisomer thereof, or the pharmaceutically acceptable salt or solvate thereof as provided herein, or the pharmaceutical composition comprising the antibody-drug conjugate of formula (I), the stereoisomer thereof, or the pharmaceutically acceptable salt or

solvate thereof as provided herein, optionally a container, and optionally a package insert or label indicating treatment.

## V TREATMENT APPROACHES

In yet another aspect, the present disclosure relates to a method for treating diseases and conditions related to HER3 activity, e.g., cancer, in a subject in need thereof, which comprises administering to the subject a therapeutically effective amount of the antibody-drug conjugate of formula (I), the stereoisomer thereof, or the pharmaceutically acceptable salt or solvate thereof provided herein, or the pharmaceutical composition of the present invention.

As used herein, the term "subject in need thereof" is a subject having a disease or condition associated with HER3 activity, e.g., cancer, or a subject having an increased risk of developing a disease or condition associated with HER3 activity relative to the population at large. In some embodiments, the subject is a warm-blooded animal. In some embodiments, the warm-blooded animal is a mammal. In some embodiments, the warm-blooded animal is a human.

As used herein, the term "therapeutically effective amount" refers to an amount effective to achieve a desired therapeutic result, at dosages and for periods of time necessary. The therapeutically effective amount is also such an amount that any toxic or undesired effect of the antibody-drug conjugate or the pharmaceutical composition or the pharmaceutical combination is inferior to the therapeutically beneficial effect. A "therapeutically effective amount" preferably inhibits a measurable parameter (e.g., tumor volume) by at least about 30%, and even more preferably by at least about 40%, 45%, 50%, 55%, 60%, 65%, 70%, 75%, 80%, 85%, 90%, or even 100%, relative to untreated subjects.

As used herein, the term "diseases and conditions associated with HER3 activity" refers to any pathophysiological condition associated with the activity or expression of HER3, e.g., any pathophysiological condition in which inhibition of HER3 would be beneficial. In some embodiments, the disease and condition associated with HER3 activity is cancer. In some embodiments, the diseases and conditions associated with HER3 activity are HER3-positive tumors, e.g., HER3-overexpressing tumors. In some embodiments, the subject (particularly an adult subject) has HER3 expression, particularly HER3 overexpression. In some embodiments, the subject has (e.g., an elevated level of, e.g., nucleic acid or protein level or activity of) HER3 (e.g., compared to a healthy subject). In some embodiments, a biological sample (e.g., tumor cells or tumor tissues) of the subject has (e.g., an elevated level, e.g., an elevation of greater than 10%, greater than 20%, greater than 50%, greater than 80%, greater than 90%, greater than 1-fold, greater than 2-fold, greater than 3-fold, greater than 5-fold, greater than 10-fold, or a range between these values (e.g., 10%-2-fold), e.g., a nucleic acid or protein level or activity) HER3 (e.g., as compared to a biological sample of a healthy subject (e.g., a corresponding tissue or cell in a healthy subject), or as compared to HER3 in an adjacent healthy tissue or cell of the subject). In some embodiments, the HER3-positive tumor is selected from the group consisting of breast cancer, lung cancer, pancreatic cancer, colorectal cancer, gastric cancer, head and neck squamous cell carcinoma, ovarian cancer, melanoma, prostate cancer, and bladder cancer. In some embodiments, the HER3-positive tumor is selected from the group consisting of squamous cell carcinoma, small cell lung cancer, non-small cell lung cancer, lung adenocarcinoma, squamous lung carcinoma, colon cancer, and epidermal cell carcinoma.

In yet another aspect, the present disclosure relates to the antibody-drug conjugate of formula (I), the stereoisomer thereof, or the pharmaceutically acceptable salt or solvate thereof as provided herein, or the pharmaceutical composition of the present disclosure, for use in treating diseases and conditions associated with HER3 activity, e.g., cancer or tumor, wherein the diseases and conditions associated with HER3 activity are as defined herein; for example, is a HER3-positive tumor, e.g., selected from the group consisting of breast cancer, lung cancer, pancreatic cancer, colorectal cancer, gastric cancer, head and neck squamous cell carcinoma, ovarian cancer, melanoma, prostate cancer, and bladder cancer.

In yet another aspect, the present disclosure relates to use of the antibody-drug conjugate of formula (I), the stereoisomer thereof, or the pharmaceutically acceptable salt or solvate thereof as provided herein, or the pharmaceutical composition of the present disclosure in the preparation of a medicament for treating diseases and conditions associated with HER3 activity, e.g., cancer or tumor, wherein the diseases and conditions associated with HER3 activity are as defined herein; for example, is a HER3-positive tumor, e.g., selected from the group consisting of breast cancer, lung cancer, pancreatic cancer, colorectal cancer, gastric cancer, head and neck squamous cell carcinoma, ovarian cancer, melanoma, prostate cancer, and bladder cancer.

## VI. Preparation of ADC molecules of the present invention

Another aspect of the present invention provides a method for preparing an antibody-drug conjugate of formula (I).

In some embodiments, the method comprises the following steps:

(a) adding the antibody Ab to a buffer solution, adding a reducing agent, and incubating;
(b) adding a linker-payload to the reaction solution in step (a) for coupling to obtain a crude product; and

(c) optionally purifying the crude product to obtain the antibody-drug conjugate of the present invention;

wherein Ab is as defined above.

It should be understood that the reaction of the linker-payload with Ab provides the moiety -L-D in the compound of formula I, and the structure of the linker-payload can be determined when -L-D is clearly defined.

In some embodiments, the buffer solution in step a) is a PBS buffer, preferably having a pH of 5.0-9.0, such as 6.0-8.0.

In some embodiments, the reducing agent of step a) is TCEP.

In some embodiments, the linker-payload has the following structure: Z'-E-NH-$CH_2$-Q-$L^2$-$L^1$-$D^1$, wherein E, Q, $L^2$, $L^1$, and $D^1$ are as defined above, Z' is as defined above for Z, except that the maleimide group

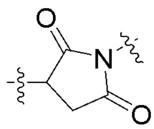

in Z is replaced with

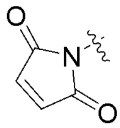
.

In some embodiments, the steps are performed under the specific reaction conditions disclosed in the examples.

It should be noted that embodiments in which the range or specific value of the specific reaction conditions disclosed in the examples varies by 100%, 80%, 60%, 40%, 20%, or 10% are also contemplated by the present invention.

These and other aspects and embodiments of the present invention are described in the accompanying drawings and the following detailed description of the invention and illustrated in the following examples. Any or all of the features discussed above and throughout this application may be combined in various embodiments of the invention. The following examples further illustrate the present invention. However, it is to be understood that the examples are described by way of illustration and not limitation, and that various modifications may occur to those skilled in the art.

## EXAMPLES

The technical solutions of the present invention will be further described in detail below by way of examples and with reference to the accompanying drawings. Unless otherwise stated, the methods and materials of the examples described below are all conventional products commercially available. Those skilled in the art will appreciate that the methods and materials described below are exemplary only and should not be taken as limiting the scope of the invention.

The embodiments of the present invention are not limited to those described in the above examples. Those of ordinary skill in the art may make various changes and modifications to the present invention in form and detail without departing from the spirit and scope of the present invention, and these are all considered to fall within the protection scope of the present invention.

### Example 1. Preparation and Detection of Antibody Molecules and ADCs

### Example 1.1. Preparation of HER3 monoclonal antibody

The nucleic acid fragments encoding the HER3 heavy and light chains were separately inserted into a vector pcDNA3.1 (Invitrogen, V790-20) to obtain anti-HER3 heavy and light chain plasmids. Heavy and light chain plasmids were transiently transfected into ExpiCHO cells (Gibco, A29133) using FectoPRO Transfection Reagent (Polyplus, 101000007). After 7 days, the cell fermentation broth was obtained, clarified by filtration, and captured using a Hitrap Mabselect Sure chromatography column (GE Healthcare, 11-0034-95) to obtain antibodies.

Table 1. Purity and yield of HER3 protein

| Antibody | Cell strain | Purity (%) | Yield (mg/L) |
|---|---|---|---|
| HER3 mAb | Expi CHO | 99.38% | 279 |

**Example 1.2. Preparation of HER3-MB3 conjugate**

A reducing agent TCEP (Sigma, C4706) was added to the HER3 antibody prepared as described above at a ratio of 20:1 (M/M), and the mixture was subjected to a reduction reaction in a water bath at 25 °C for 2 h. MB3 (for preparation, see Example 4 in WO2021173773A1, wherein the payload in MB3 was MB1) was then added to the system at a ratio of HER3 antibody:Drug-linker (MB3) = 1:14 (M/M), and the system was adjusted to a DMSO concentration of 10% (v/v) and subjected to a reaction in a water bath at 25 °C for 1 h. According to the molar ratio of relative MB3, 20-fold N-acetylcysteine (Sigma, A8199) was added, and the mixture was reacted at room temperature for 15 min. The MB3 was not completely reacted in the reaction system. The impurities were removed using a Zeba$^{TM}$ desalting spin column (Thermo, 40K MWCO), and the buffer was replaced with 20 mM histidine buffer at pH 5.5. Finally, the prepared and purified HER3-MB3 was filtered and sterilized using 0.2 μM PVDF (Millipore, SLGVR13SL) and stored at a low temperature of -40 °C.

The structure of the HER3-MB3 molecule is shown below:

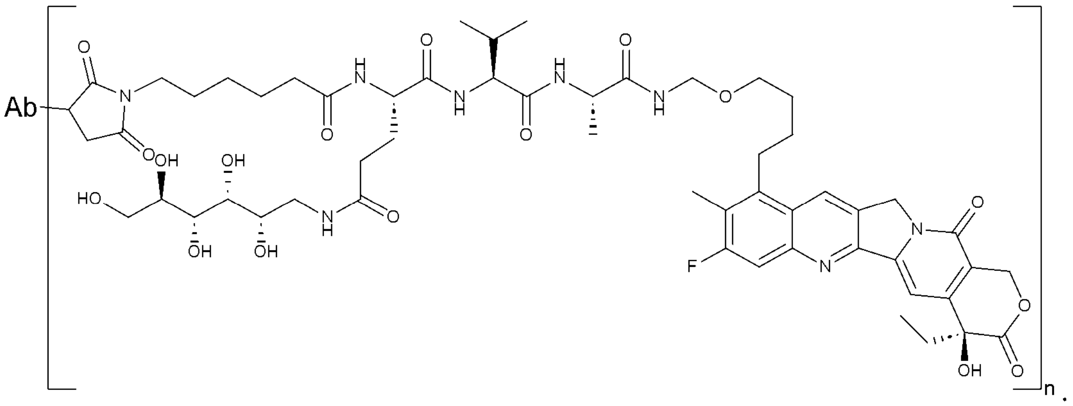

wherein Ab was Patritumab (the antibody prepared in Example 1.1), and n was mainly 8.

FIG. 5 shows a schematic diagram of the HER3-MB3 molecule in Example 1.

Table 2. HER3-MB3 quality test

| Sample | Average DAR | SEC (%) | | | FreeLoad (%wt/wt) | Yield (%) |
|---|---|---|---|---|---|---|
| | | HMWS | Monomer | LMWS | | |
| HER3-MB3 | 7.56 | 0.9 | 99.07 | 0.03 | 0.04 | 75.2 |

**Example 1.3. Preparation of Other ADCs**

ADCs with different linkers and payloads (e.g., different HER3-ADCs) were prepared using different liners and payloads as shown in Table 3 and antibodies (e.g., HER3 antibody patritumab or BL antibody izolontamab) based on methods known in the art. In the table, Liner and Payload are well known to those skilled in the art of ADCs and are commercially available and/or synthesized according to the prior art.

1. Preparation of patritumab-DM4 (HER3-DM4) conjugation

The patritumab antibody was exchanged into 20 mM pH 7.0 phosphate buffer, and SPDB-DM4 was added to the system according to the patritumab antibody: drug-linker (SPDB-DM4) = 1:8 (M/M) ratio. The system was adjusted to make the DMA concentration of the system 15% (v/v), and the conjugation reaction was performed in a water bath at 30 °C for 16 h. The impurities were removed using a Zeba$^{TM}$ desalting spin column (Thermo, 40K MWCO) and replaced with 20 mM histidine buffer at pH 5.5. Finally, the purified patritumab-DM4 was filtered and sterilized using 0.2 μM PVDF (Millipore, SLGVR13SL) and stored at -40 °C.

2. Preparation of conjugated Patritumab-DM1 (HER3-DM1)

The patritumab antibody was exchanged into 20 mM phosphate buffer (pH 7.0), and SPDB-DM1 was added to the system according to the patritumab antibody: drug-linker (SMCC-DM1) = 1:10 (M/M) ratio. The system was adjusted to make the DMA concentration of the system 15% (v/v), and the conjugation reaction was performed in a water bath at 30 °C

for 16 h. The impurities were removed using a Zeba[TM] desalting spin column (Thermo, 40K MWCO) and replaced with 20 mM histidine buffer at pH 5.5. Finally, the purified patritumab-DM1 was filtered and sterilized using 0.2 μM PVDF (Millipore, SLGVR13SL) and stored at -40 °C.

3. Preparation of patritumab-MMAE (HER3-MMAE) conjugation

A reducing agent TCEP (Sigma, C4706) was added to the patritumab antibody at a ratio of 2:1 (M/M), and the mixture was subjected to a reduction reaction in a water bath at 25 °C for 1 h; MC-VC-PAB-MMAE was then added to the system at a ratio of patritumab antibody : drug-linker (MC-VC-PAB-MMAE) = 1:6 (M/M), the system was adjusted to make the concentration of DMSO in the system be 10% (v/v), and the mixture was subjected to a reaction in a water bath at 25 °C for 1 h. The impurities were removed using a Zeba[TM] desalting spin column (Thermo, 40K MWCO) and replaced with 20 mM histidine buffer at pH 5.5. Finally, the purified patritumab-MMAE was filtered and sterilized using 0.2 μM PVDF (Millipore, SLGVR13SL) and stored at -40 °C.

4. Preparation of patritumab-DXd (HER3-DXd) conjugation

A reducing agent TCEP (Sigma, C4706) was added to the patritumab antibody in an 8:1 (M/M) ratio, and the mixture was subjected to a reduction reaction in a water bath at 25 °C for 1 h; MC-GGFG-Dxd was then added to the system in a patritumab antibody : drug-linker (MC-GGFG-Dxd) ratio of 1:10 (M/M), the system was adjusted to make the DMSO concentration of the system be 10% (v/v), and the system was subjected to a reaction in a water bath at 25 °C for 1 h. The impurities were removed using a Zeba[TM] desalting spin column (Thermo, 40K MWCO) and replaced with 20 mM histidine buffer at pH 5.5. Finally, the purified patritumab-Dxd was filtered and sterilized using 0.2-μM PVDF (Millipore, SLGVR13SL) and stored at -40 °C.

The preparation method of the linker-drug specific to patritumab-MMAF was the same as that of patritumab-MMAE. The preparation method of conjugation of Izalontamab and BL-DXd was the same as that of patrituman-DXd.

Table 3. Information on different linkers and payloads of ADC

| **HER3-ADC** | **Linker** | **Payload** | **Average DAR** |
|---|---|---|---|
| **HER3-DXd** | Mc-GGFG | DXd | 7.57 |
| **HER3-DM1** | SPDB | DM1 | 3.9 |
| **HER3-DM4** | SPDB | DM4 | 3.66 |
| **HER3-MMAE** | Mc-VC-PAB | MMAE | 3.67 |
| **HER3-MMAF** | Mc-VC-PAB | MMAF | 3.73 |
| **BL-DXd** | Mc-GGFG | DXd | 7.56 |

**Example 1.4. HER3-MB3 Affinity Assay**

The affinity (KD) of HER3-MB3 for human (Sino Biological, 10201-H08H), monkey (Sino Biological, 90043-K08H), mouse (Sino Biological, 51003-M08H), and rat (Sino Biological, 80111-R08H) HER3 was determined by bio-layer interferometry (BLI). Before the experiment, an appropriate number of AHC sensors (18-5060, Sartorius) were soaked in SD buffer (1× PBS, 0.1% BSA, 0.05% Tween-20), and HER3-MB3 and the corresponding antigens were each diluted to 100 nM. The SD buffer, HER3-MB3, and the corresponding antigens were each added to a 96-well black polystyrene microplate (Greiner, 655209). The assay was performed using Fortebio Octet Red96e, and the KD analysis was performed using ForteBio Octet.

Table 4. Affinity of HER3-MB3 to HER3 from different species

| Antibody | Antigen | KD (M) | Kon (1/Ms) | Kids (1/S) |
|---|---|---|---|---|
| HER3-MB3 | Human | 2.42E-09 | 1.91E+05 | 4.63E-04 |
| | Cynomolgus monkeys | 2.55E-09 | 2.21E+05 | 5.62E-04 |
| | Mice | 4.58E-08 | 2.16E+05 | 9.89E-03 |
| | Rat | 3.88E-08 | 1.58E+05 | 6.15E-03 |

## Example 2. Screening of antibody-drug conjugates

In Example 1, various antibody-drug conjugates were designed and synthesized. For example, HER3 antibodies were conjugated to cytotoxic drug molecules such as DXd, MMAE, MMAF, DM1, and DM4. In this example, HER3-MB3 (patritumab-MB3) was finally selected by experimental comparison in terms of in vitro pharmacodynamic sequence screening, whether the payloads have potential drug resistance, bystander effects of different linker-payloads, and the like.

### 1. Antibody sequence screening

In vitro killing assay method

(1) The cells were plated at 2000 cells/100 μL in a 96-well low-adsorption plate (Corning, CLS7007-24EA) and cultured in a 3D cell culture medium for two days to allow the suspended cells to form cell clusters.
(2) The drug molecules prepared in advance by dilution (maximum concentration: 100 nM, 4-fold dilution) were added to the corresponding cell well plate, mixed well, and cultured and killed at 37 °C with 5% $CO_2$ for 7 days.
(3) After 7 days of culture and killing, the Cell-Titer reagent (Promega, G7572) prepared in advance was added to the corresponding cell well plate, and the plate was left to stand at room temperature for 15-25 min in the dark.
(4) The Cell-Titer and cell mixture were transferred to a 96-well white-bottom plate (NUNC, 136101) and detected by a microplate reader (Molecular Devices, Spectra MAXi3).

### 2. Whether there is potential drug resistance in Payload

Elacridar is an orally active Pgp glycoprotein that can be used to study the effect of efflux transporters on drugs; the adapted Elacridar-resistant cell line HCT15 was screened for potential drug resistance evaluation of Payload.
Experimental methods

(1) A 96-well white-bottom plate (NUNC, 136101) was plated with 1000 cells/well and cultured at 37 °C with 5% $CO_2$ overnight.
(2) The payload drug molecules prepared in advance by dilution (maximum concentration: 100 nM, 3-fold dilution) were added to the corresponding well plate containing 0.5 μM Elacridar or not, mixed well, and cultured at 37 °C with 5% $CO_2$ for killing for four days.
(3) After four days of culture and killing, the Cell-Titer reagent (Promega, G7572) prepared in advance was added to the corresponding cell well plate. The plate was left to stand at room temperature in the dark for 15-25 min and detected on a microplate reader (Molecular Devices, Spectra MAXi3).

### 3. Bystander effect of linker-payload

HER3-MB3 bystander effect was performed in the SW480/SW480HER3-OX system.
Construction of SW480HER3-OX cell line: Lentvirus + HER3 lentivirus was constructed and packaged, SW480 was infected with Lentvirus + HER3 virus, and a SW480HER3-OX stably transfected cell line was obtained by pressurized screening.
Experimental methods

(1) 80000 cells/SW480 and 100000 cells/SW480+HER3 cells were plated in a six-well plate and cultured overnight.
(2) Drugs prepared in advance by dilution were added to the corresponding cell well plates at a final concentration of 5 nM. The mixtures were well mixed and subjected to killing culture at 37 °C with 5% $CO_2$ for 5 days.
(3) On day 5, the cells were digested, and the total number of N cells was counted; according to the expression level of HER3, the percentage relationship between SW480HER3-OX and SW480 was marked by FACS (BD, Celesta).

Calculation method for absolute number of cells:

SW480HER3-OX=N*FACS ($HER3^+$ - SW480HER3OX)*100
SW480= N*FACS ($HER3^-$ - SW480)*100

Experimental results:

As can be seen from the results in FIGs. 1 to 2, HER3-ADCs (patritumab-MB3 and panitumumab-DXd) have

better in vitro efficacy in SW620 and MCF-7 than BL-DXd (Izalontamab-DXd), and HER3-MB3 (patritumab-MB3) has better in vitro efficacy than HER3-DXd (patritumab-DXd). As can be seen from the results in FIG. 3, the potential drug resistance of MB1 was lower than that of MMAF, MMAE, DM1, DM4, and DXd. As can be seen from the results in FIG. 4, HER3-MB3 (patritumab-MB3) has a stronger bystander effect. According to the results in FIGs. 1 to 4, the antibody-drug conjugate HER3-MB3 was finally selected.

**Example 3. Expression abundance in HER3 major tumor cell lines**

In addition to being related to the drug-linker technology, the anti-tumor effect of HER3-MB3 is also related to the intensity and abundance of HER3 expression in tumor cells.

The relative expression of HER3 was analyzed in a variety of tumor cell lines. In the analyzed cell lines, the overall expression level of HER3 was in the order of gastric cancer, breast cancer, colorectal cancer, lung cancer, and pancreatic cancer. The experimental results are shown in FIG. 6.

Experimental method:

1. The cells were resuspended in an FACS buffer, and 100000-200000 cells were plated in a 96-well plate (Corning, CLS3799-50EA). 10 μg/mL HER3 antibody (patritumab) and 10 μg/mL IgG were separately added to the cell well plate, and the plate was incubated at 4 °C for 1 h.
2. The plate was washed twice with PBS, and APC-anti human Fc antibody (Biolegend, 410712) was added to the corresponding cell well plate. The plate was incubated at 4 °C for 30-40 min.
3. The plate was washed twice with PBS and assayed by FACS (BD, Celesta ). The results are shown in FIG. 6.

Cell source:

Gastric cancer cell lines: (1). NUGC4:CoBier, CBP60012; (2). KATO-III:Cell Bank, Chinese Academy of Sciences, SCSP-573; (3). NCI-N87:CoBier, CBP60491; (4). MKN45:CoBier, CBP60488; (5). MGC-803:CoBier, CBP60485.

Breast cancer cell lines: (6). MDA-MB-453:CoBier, CBP60386; (7). MCF-7:CoBier, CBP60380; (8). HCC1569:CoBier, CBP60372; (9). JIMT-1:CoBioer, CBP60378; (10). MDA-MB-231:Cell Bank, Chinese Academy of Sciences, SCSP-5043.

Colorectal cancer cell lines: (11). SW620:CoBioer, CBP60036; (12). HT55:HT55, CBP60012; (13). LOVO: Chinese Academy of Sciences Cell Bank, TCHu 82; (14). HCT-15:Shanghai Cell Bank, Chinese Academy of Sciences, TCHu133; (15). GP2D:CoBioer, CBP60010; (16). H508:CoBioer, CBP60795; (17). LS174T:CoBioer, CBO60033; (18). SW480: Cell Bank of Chinese Academy of Sciences, SCSP-5033; (19). Colo320:CoBioer, CBP61130.

Lung cancer cell lines: (20). Calu-3:CoBioer, CBP60086; (21). H358:CoBioer, CBP60136; (22). DMS53:CoBioer, CBP60172; (23). HCC827:ATCC, CRL-2868; (24). NCI-H1975:ATCC, CRL-5908; (25). A431:ATCC, CRL-1555; (26). A549:CoBioer, CBP60084; (27). NCI-H441:Cobioer, CBP60137; (28). NCI-H2030:ATCC, CRL-5914; (29). NCI-H292:Cell Bank, Chinese Academy of Sciences, SCSP-582; (30). NCI-H4006:ATCC, CRL-2871; (31). NCI-H1703:ATCC, CRL-5889; (32). SK-MES-1:CoBioer, CBP60152; (33). Calu-1:ATCC, HTB-54; (34). PC-9:Shanghai Yubo Biotechnology Co., Ltd., YB-H3210; (35). HCC2279:CoBioer, CBP60098.

Pancreatic cancer cell lines: (36). Capan-1:CoBioer, CBP60543; (37). T3M4:CoBioer, CBP60692; (38). Capan-2:CoBioer, CBP60834; (39). CFPAC-1:CoBioer, CBP60665; (40). Miapaca2:CoBioer, CBP60544; (41). AsPc-1:CoBioer, CBP60546; (42). HuP-T4:CoBioer, CBP60540; (43). PSN-1:CoBioer, CBP61215; (44). BxPc-3:CoBioer, CBP60542; (45). PANC-1:CoBioer, CBP60545.

HCC827 + HER3 (HCC827 HER3OX) overexpression cell line: a Lentvirus + HER3 lentivirus was constructed and packaged, HCC827 was infected with the Lentvirus + HER3 virus, and stably transfected HCC827 + HER3 cells were obtained by pressurized screening and sorting.

**Example 4. In Vitro Efficacy Experiment of HER3-MB3 in Tumor Cell Lines**

Experimental method:

1. In vitro killing assay method

(1) The cells were plated at 2000 cells/100 μL in a 96-well low-adsorption plate (Corning, CLS7007-24EA) and cultured in a 3D cell culture medium for two days to allow the suspended cells to form cell clusters.
(2) The drug molecules prepared in advance by dilution (maximum concentration: 25 nM, 4-fold dilution) were added to the corresponding cell well plate, mixed well, and cultured and killed at 37 °C with 5% $CO_2$ for 7 days.
(3) After 7 days of culture and killing, the Cell-Titer reagent (Promega, G7572) prepared in advance was added to the corresponding cell well plate, and the plate was left to stand at room temperature for 15-25 min in the dark.
(4) The Cell-Titer and cell mixture were transferred to a 96-well white-bottom plate (NUNC, 136101) and detected by a microplate reader (Molecular Devices, Spectra MAXi3).

Experimental results:

As can be seen from the results in FIGs. 7-10, the in vitro pharmacodynamic activity of HER3-MB3 was positively correlated with the expression intensity of HER3, and the higher the expression of HER3, the stronger the in vitro pharmacodynamic activity of HER3-MB3, indicating that HER3-MB3 can be used for treating patients with HER3-positive tumors.
As can be seen from the results in FIG. 7, HER3-MB3 has anti-tumor pharmacodynamic activity in gastric cancer tumors. The in vitro pharmacodynamic activity in the NUGC4 tumor cell line was far superior to that of HER3-DXd (U3-1402). At the highest concentration of 25 nM, the maximum tumor killing rate of HER3-MB3 was 73.34 ± 3.21%, and the $IC_{50}$ was 0.55 nM; the maximum tumor killing rate of HER3-DXd was 45.52 ± 5.82%, and the $IC_{50}$ was 2.02 nM.
As can be seen from the results in FIG. 8, HER3-MB3 has anti-tumor pharmacodynamic activity in breast cancer tumors. The in vitro pharmacodynamic activity in the HCC1569 tumor cell line was far superior to that of HER3-DXd (U3-1402). At the highest concentration of 25 nM, the maximum tumor killing rate of HER3-MB3 was 77.16 ± 0.62%, and the $IC_{50}$ was 0.07 nM; the maximum tumor killing rate of HER3-DXd was 72.90 ± 1.62%, and the $IC_{50}$ was 0.20 nM.
As can be seen from the results in FIGs. 9 and 10, HER3-MB3 has anti-tumor pharmacodynamic activity in colorectal cancer tumors, and is superior to HER3-DXd (U3-1402) in in vitro pharmacodynamic activity. In the NCI-H508 tumor cell line, the maximum in-vitro tumor killing rates of HER3-MB3 and HER3-DXd were 68.07 ± 1.45% and 43.27 ± 2.93%, respectively, at 25 nM effective dose; in the SW620 tumor cell line, the maximum in-vitro tumor killing rates of HER3-MB3 and HER3-DXd were 30.21 ± 2.34% and 25.00 ± 2.68%, respectively, at 25 nM effective dose.
The results in FIG. 11 show that in the HER3-overexpressing HCC827 cell line, HER3-MB3 had significant anti-tumor pharmacodynamic activity.
The results of in vitro killing of other breast and colorectal cancers in Table 5 showed that HER3-MB3 had stronger in vitro efficacy than HER3-DXd (U3-1402).

Table 5. Killing effect of HER3-MB3 on tumor cell lines

| Cell lines | HER3-ADC inhibition% (up to 25 nM) | |
|---|---|---|
| | HER3-MB3 | HER3-DXd |
| MCF-7 | 78.08 ± 1.32 | 65.38 ± 3.08 |
| JITM-1 | 76.26 ± 6.33 | 68.88 ± 8.17 |
| LS174T | 29.72 ± 2.11 | 9.59 ± 5.85 |
| HT55 | 28.90 ± 9.19 | 0 |
| LOVO | 16.05 ± 3.74 | 13.66 ± 4.31 |
| GP2D | 4.96 ± 2.27 | 0 |

**Example 5. Experiment on bystander effect of HER3-MB3**

Bystander effect means that when the ADC kills and inhibits positive target tumor cells, part of the toxin molecules will be released from the positive target cells and enter nearby negative target tumor cells, further exerting a stronger anti-tumor effect.
As shown in FIG. 12, by comparing different drugs, it was found that MB3 showed better anti-tumor activity and bystander effect.

Experimental method:

In the SW480 /SW480HER3-OX system, the HER3-MB3 bystander effect assay was performed as follows:

(1) 80000 cells/SW480 and 100000 cells/SW480+HER3 cells were plated in a six-well plate and cultured overnight.
(2) Drugs prepared in advance by dilution were added to the corresponding cell well plates at a final concentration of 5 nM. The mixtures were well mixed and subjected to killing culture at 37 °C with 5% $CO_2$ for 5 days.
(3) On day 5, the cells were digested, and the total number of N cells was counted. According to the expression level of HER3, the percentage relationship between SW480HER-OX and SW480 was marked by FACS (BD, Celesta).

Calculation method for absolute number of cells:

$$SW480HER3\text{-}OX=N*FACS\ (HER3^{+}\ -\ SW480ER3OX)*100$$

$$SW480=N*FACS\ (HER3^{-}\ -\ SW480)*100$$

**Example 6. In vivo efficacy study of HER3-MB3 in mice**

In vivo efficacy study of SW620

To verify the in vivo efficacy of HER3-MB3 in a colorectal cancer tumor model, BALB/C-Nude (Beijing Vital River Laboratory Animal Technology Co., Ltd., certificate No. 110011221105802464) mice were used to establish a SW620 CDX mouse tumor model.

Experimental method:

SW620 tumor cells were subcultured, resuspended in PBS and Matrigel Matrix in equal proportions, and seeded at 6E6 cells/200 μL on the right side of the abdomen of the mice. On day 9, the mice were divided into groups and administered intraperitoneally. The administration cycle was once a week for a total of three times, and the tumor size and body weight were measured twice a week.

Tumor growth inhibition (TGI%) was calculated by the following formula: TGI% = 100% × (control group tumor volume - treatment group tumor volume)/(control group tumor volume - control group initial tumor volume).

Tumor volume measurement: The maximum length of major axis (L) and maximum length of minor axis (W) of tumors were measured with a vernier caliper, and tumor volume was calculated using the following formula: V=L*W2/2

Experimental results

On day 21 after SW620 cell modeling, the tumor inhibition rate in mice was calculated, and the in vivo efficacy of HER3-MB3 was evaluated. As shown in FIG. 13, at the dose of 10 mg/kg, the maximum tumor inhibition rates of HER3-MB3 and HER3-DXd (U3-1402) were 111.80% and 108.76%, respectively, and the efficacy of HER3-MB3 was slightly better than that of HER3-DXd (U3-1403); at the dose of 10 mg/kg, IgG-MB3 showed non-specific tumor killing. At a dose of 3 mg/kg, the maximum tumor growth inhibition rates of HER3-MB3 and HER3-DXd (U3-1402) were 71.43% and 72.43%, respectively. The results in FIG. 14 show that at high and low doses, HER3-MB3 and HER3-DXd mice did not show significant changes in body weight, indicating that HER3-ADC is safe.

In vivo efficacy study of NUGC4

To verify the in vivo efficacy of HER3-MB3 in a gastric cancer tumor model, NOG mice (Beijing Vital River Laboratory Animal Technology Co., Ltd., NO. 110011221107474276) to establish a NUGC4 CDX mouse tumor model.

Experimental method:

NUGC4 tumor cells were subcultured, resuspended in PBS and Matrigel Matrix in equal proportions, and inoculated at 4E6 cells/200 μL/mouse on the right side of the abdomen. On day 18, the mice were divided into groups and intraperitoneally administered with the cells. The administration cycle was once a week for a total of three times. The tumor size and body weight were measured twice a week.

Tumor growth inhibition (TGI%) was calculated by the following formula: TGI% = 100% × (control group tumor volume - treatment group tumor volume)/(control group tumor volume - control group initial tumor volume).

Tumor volume measurement: The maximum length of major axis (L) and maximum length of minor axis (W) of tumors were measured with a vernier caliper, and tumor volume was calculated using the following formula: V = L × W2/2.

Experimental results

On day 21 after the modeling of NUGC4 cells, the tumor inhibition rate in mice was calculated, and the in vivo efficacy of HER3-MB3 was evaluated. As shown in FIG. 15, at a dose of 10 mg/kg, the maximum tumor growth inhibition rates of HER3-MB3 and HER3-DXd (U3-1402) were 99.27% and 99.93%, respectively; at a dose of 3 mg/kg, the efficacy of HER3-MB3 was significantly better than that of HER3-DXd, with the maximum tumor growth inhibition rate of HER3-MB3 being 81.31% and the maximum tumor growth inhibition rate of HER3-DXd being 46.61%. At the high dose of 10 mg/kg, both IgG-MB3 and IgG-DXd also showed non-specific tumor killing. The results in FIG. 16 show that at high and low doses, HER3-MB3 and HER3-DXd mice did not show significant changes in body weight, indicating that HER3-ADC is safe.

**Example 7. PK of HER3-MB3 in mice**

In order to verify the pharmacokinetics (PK) of HER3-MB3 and HER3-DXd in mice, 10 mg/kg HER3-MB3 and 10 mg/kg HER3-DXd were separately administered via the tail vein of mice. Blood was collected from the orbit at 5 min, 30 min, 2 h, 6 h, 24 h, 48 h, 96 h, 168 h, 336 h, and 504 h, and serum was prepared. The plasma concentration was determined using the HER3 mAb and HER3-ADC assay methods.

Mouse serum HER3 mAb PK assay

1. 1 μg/mL HER3 protein (Acrobiosystem, ER3-H82E6) was plated on a 96-well microplate (Thermo, 442404) and incubated overnight at 4 °C.
2. The plate was washed 3 times with a washing solution (PBST, PBS solution containing 0.05% Tween-20).
3. The plate was blocked with a blocking solution (containing 5% skim milk powder PBST) at room temperature for 2 h.
4. The plate was washed 3 times with wash buffer, and standards (HER3-MB3, HER3-DXd) and serum samples to be tested were added. The plate was incubated at room temperature for 2 h.
5. The plate was washed 6 times with wash buffer and incubated with Goat anti human IgG-Fc-HRP (Bethyl, A80-104P) at room temperature for 1 h.
6. The plate was washed 6 times with wash buffer, and TMB (Solarbio, PR1200) was added for color development in the dark for 5-10 min.
7. ELISA stop solution (Solarbio, C1058) was added, and OD450/OD620 nm detection was performed using a microplate reader (Thermo, Multiskan FC).

Mouse serum HER3-ADC PK assay method

1. 1 μg/mL Payload antibody (Innovent, 18E7C9) was plated in a 96-well microplate (Thermo, 442404) and incubated overnight at 4 °C.
2. The plate was washed 3 times with a washing solution (PBST, PBS solution containing 0.05% Tween-20).
3. The plate was blocked with a blocking solution (containing 5% skim milk powder PBST) at room temperature for 2 h.
4. The plate was washed 3 times with wash buffer, and standards (HER3-MB3, HER3-DXd) and serum samples to be tested were added. The plate was incubated at room temperature for 2 h.
5. The plate was washed 6 times with washing buffer and incubated with 1 μg/mL HER3 protein (Acrobiosystem, ER3-H82E6) at room temperature for 2 h.
6. The plate was washed 6 times with wash buffer and incubated with SA HRP (BD, 554066) at room temperature for 1 h.
7. The plate was washed 6 times with wash buffer, and TMB (Solarbio, PR1200) was added for color development for 5-10 min in the dark.
8. ELISA stop solution (Solarbio, C1058) was added, and OD450/OD620 nm detection was performed using a

microplate reader (Thermo, Multiskan FC).

Experimental results:

As shown in FIG. 17 and the data results in Table 6, the total HER3 antibody and HER3-ADC concentrations in the mouse plasma were detected by the different detection methods described above, and PK of HER3-MB3 in mice is better than HER3-DXd, further indicating that HER3-MB3 had better in vivo efficacy than HER3-DXd.

Table 6. PK parameters of 10 mg/kg HER3-ADC in mice

| Group | $C_{max}$ (ug/mL) | $AUC_{0\text{-}t}$ (ug*h/mL) | $AUC_{0_inf_obs}$ (ug*h/mL) | $MRT_{0\text{-}inf_obs}$ (h) | $t_{1/2}$ (h) | C1 (ml/kg/h) | $V_{ss}$ (ml/kg) |
|---|---|---|---|---|---|---|---|
| HER3-DXd (TAB) | 142 | 4690 | 4743 | 68 | 54 | 2.11 | 144 |
| HER3-DXd (ADC) | 118 | 5616 | 5643 | 60 | 43 | 1.77 | 105 |
| HER3-MB3 (TAB) | 204 | 7908 | 7993 | 80 | 54 | 1.25 | 100 |
| HER3-MB3 (ADC) | 205 | 10374 | 10446 | 78 | 49 | 0.96 | 74 |

**Example 8. Toxicokinetics (TK) of HER3-MB3 in cynomolgus monkeys and rats**

Compared with the in vitro PD data, in vivo PD data, bystander effect, and in vivo PK data of HER3-DXd, the PD data of HER3-MB3 was better than that of HER3-DXd. In order to further verify the safety of HER3-MB3, according to the protocol designed by Innovent Biologics, JOINN Laboratories (Suzhou) Co., Ltd. was commissioned to carry out the pre-toxicology safety study of HER3-MB3 in cynomolgus monkeys, as shown in Table 7.

Table 7. Preliminary toxicology study protocol for HER3-MB3

| Animals | Group | Qty. | Dose (mg/kg) | Mode of administration | Treatment period |
|---|---|---|---|---|---|
| Cynomolgus monkeys | HER3-MB3 | 2 | 30 | Intravenous | Once every three weeks for a total of three doses |
| | HER3-MB3 | 2 | 80 | Intravenous | Once every three weeks for a total of three doses |
| CLINICAL OBSERVATIONS | Mortality, food intake, body constitution monitoring, and injection site observation | | | | |
| TK Study | Total antibody amount (TAB) and plasma concentration of HER3-MB3 were measured at 0 min, 4 h, 8 h, 12 h, 24 h, 48 h, 72 h, 168 h, 336 h, and 504 h after the first and second dosings. | | | | |

Experimental results:

According to clinical observation, 30 mg/kg HER3-MB3 and 80 mg/kg HER3-MB3 had no death and serious toxic and side effects in cynomolgus monkeys, and HER3-MB3 was safe.

According to Table 8 and FIG. 18, the accumulation of HER3-MB3 in cynomolgus monkeys increased with increasing dose. In cynomolgus monkeys, HER3-MB and total HER3 mAb exposures were generally consistent: $1^{st}$-30 mg/kg (ADC/TAB = 90%), $1^{st}$-80 mg/kg (ADC/TAB = 109%), $2^{nd}$-30 mg/kg (ADC/TAB = 95%), $2^{nd}$-80 mg/kg (ADC/TAB = 96%).

Table 8. Main TK parameters of HER3-MB3 pre-toxicology study

| Number of doses | Test method | Cynomolgus monkeys | | | |
|---|---|---|---|---|---|
| | | 30mg/kg | | 80mg/kg | |
| | | $C_{max}$(ug/ml) | $AUC_{last}$(ug*h/ml) | $C_{max}$(ug/ml) | $AUC_{last}$(ug*h/ml) |
| 1st dose | ADC | 691 | 37900 | 1142 | 68900 |
| | TAB | 692 | 42100 | 1053 | 63250 |
| 2st dose | ADC | 584 | 33600 | 1675 | 67100 |
| | TAB | 577 | 35200 | 1670 | 69800 |

The foregoing description is considered as illustrative only of the principles of the invention. Furthermore, since many modifications and variations will be apparent to those skilled in the art, it is not desired to limit the invention to the exact construction and procedures described above. Accordingly, all suitable modifications and equivalents may be considered to fall within the scope of the invention as defined by the appended claims.

All publications, patents, and patent applications cited herein are incorporated by reference in their entirety into the present disclosure.

The amino acid sequences of the antibody molecules and the nucleic acid sequences encoding the antibody molecules mentioned herein are as follows.

Amino acid sequence of HCDR1 of HER3 antibody: SEQ ID NO: 1
GYYWS

Amino acid sequence of HCDR2 of HER3 antibody: SEQ ID NO: 2
EINHSGSTNYNPSLKS

Amino acid sequence of HCDR3 of HER3 antibody: SEQ ID NO: 3
DKWTWYFDL

Amino acid sequence of LCDR1of HER3 antibody: SEQ ID NO: 4
RSSQSVLYSSSNRNYLA

Amino acid sequence of LCDR2 of HER3 antibody: SEQ ID NO: 5
WASTRES

Amino acid sequence of LCDR3 of HER3 antibody: SEQ ID NO: 6
QQYYSTPRT

Amino acid sequence of the heavy chain variable region of HER3 antibody: SEQ ID NO: 7

QVQLQQWGAGLLKPSETLSLTCAVYGGSFSGYYWSWIRQPPGKGLEWIGEI
NHSGSTNYNPSLKSRVTISVETSKNQFSLKLSSVTAADTAVYYCARDKWTWYFD
LWGRGTLVTVSS

Nucleotide encoding sequence of the heavy chain variable region of HER3 antibody: SEQ ID NO: 8

CAGGTGCAGCTGCAGCAGTGGGGCGCCGGACTGCTGAAGCCCAGCGAGA CACTGAGCCTGACCTGCGCCGTGTACGGAGGCTCCTTCAGCGGATACTACTGG AGCTGGATCAGACAGCCACCCGGCAAAGGACTGGAATGGATTGGAGAGATCA ACCACAGCGGAAGCACCAACTACAACCCCTCTCTGAAGTCCAGAGTGACCAT CAGCGTGGAGACCTCAAAGAACCAGTTTAGCCTGAAGCTGAGCTCTGTGACT GCCGCCGACACCGCCGTGTACTACTGCGCCCGCGACAAATGGACCTGGTACTT

CGACCTGTGGGGCAGAGGCACCCTGGTGACCGTGAGCAGC

Amino acid sequence of the light chain variable region of HER3 antibody: SEQ ID NO: 9

DIEMTQSPDSLAVSLGERATINCRSSQSVLYSSSNRNYLAWYQQNPGQPPKL LIYWASTRESGVPDRFSGSGSGTDFTLTISSLQAEDVAVYYCQQYYSTPRTFGQGT KVEIK

Nucleotide encoding sequence of the light chain variable region of HER3 antibody: SEQ ID NO: 10

GACATTGAAATGACCCAGAGCCCCGACAGCCTGGCCGTGAGCCTGGGAG AGAGAGCCACCATCAACTGCAGAAGCAGCCAGAGCGTGCTGTACAGCAGCAG CAACAGAAACTACCTGGCCTGGTATCAACAGAATCCCGGCCAGCCCCCCAAA CTGCTGATCTACTGGGCCTCCACCCGGGAAAGCGGCGTGCCAGACAGATTCA GCGGCAGCGGCAGCGGAACCGACTTCACCCTGACCATCAGCAGCCTGCAGGC TGAGGACGTGGCCGTGTACTACTGCCAGCAGTACTACTCTACCCCCAGAACCT TCGGCCAGGGCACCAAAGTGGAGATCAAG

Amino acid sequence of the heavy chain constant region of HER3 antibody: SEQ ID NO: 11

ASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKRVEPKSCDKTHT CPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIE KTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLS LSPGK

Amino acid sequence of the light chain constant region of HER3 antibody: SEQ ID NO: 12

RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGEC

Amino acid sequence of the heavy chain of HER3 antibody: SEQ ID NO: 13

QVQLQQWGAGLLKPSETLSLTCAVYGGSFSGYYWSWIRQPPGKGLEWIGEI NHSGSTNYNPSLKSRVTISVETSKNQFSLKLSSVTAADTAVYYCARDKWTWYFD LWGRGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKRV EPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKV SNKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK

Amino acid sequence of the light chain of HER3 antibody: SEQ ID NO: 14

DIEMTQSPDSLAVSLGERATINCRSSQSVLYSSSNRNYLAWYQQNPGQPPKL LIYWASTRESGVPDRFSGSGSGTDFTLTISSLQAEDVAVYYCQQYYSTPRTFGQGT KVEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRG EC

## Claims

1. An antibody-drug conjugate of formula (I), a stereoisomer or a pharmaceutically acceptable salt or a solvate thereof:

$$Ab\text{-}(L\text{-}D)_n \qquad (I)$$

wherein,

Ab is an antibody or an antigen-binding fragment thereof that binds to HER3, comprising a heavy chain variable region and a light chain variable region, wherein the heavy chain variable region comprises a HCDR1, a HCDR2 and a HCDR3, comprising the amino acid sequences set forth in SEQ ID NO: 1, SEQ ID NO: 2 and SEQ ID NO: 3, respectively; and the light chain variable region comprises a LCDR1, a LCDR2 and a LCDR3, comprising the amino acid sequences set forth in SEQ ID NO: 4, SEQ ID NO: 5 and SEQ ID NO: 6, respectively;
L is a linker having the following structure: $-Z-E-NH-CH_2-$,
wherein Z is linked to Ab, and $-CH_2-$ is linked to D;
Z is selected from

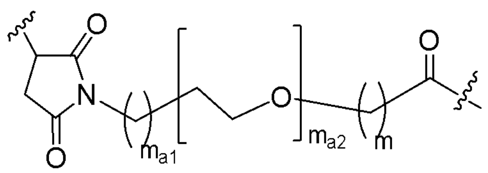

,

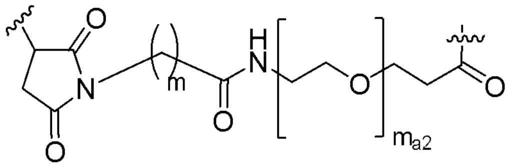, and 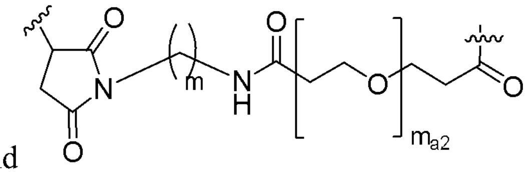 ;

wherein $m_{a1}$ and $m_{a2}$ are independently selected from an integer of 0-20;
m is selected from an integer of 1-10,
the carbonyl group at the right end of Z is covalently linked to E;
E is a peptide residue comprising 2-10 amino acids, wherein the peptide residue is optionally substituted with one or more groups selected from $C_{1-6}$ alkyl and a polyol group, wherein the N-terminus of the peptide residue is covalently linked to Z,
D is a cytotoxic compound having a structure of formula $-Q-L^2-L^1-D^1$,
wherein Q is -O- or -S-;
$L^1$ is absent or $-(C_1-C_{10}$ alkylene)-;
$L^2$ is absent, $*-(C_1-C_{10}$ alkylene)-$C(O)N(R^5)$- or $*-(C_1-C_{10}$ alkylene)-$N(R^5)C(O)$-; wherein * indicates that the end is covalently linked to Q; and $R^5$ is H or $C_1-C_6$ alkyl,
wherein $D^1$ has a structure of formula (D-1):

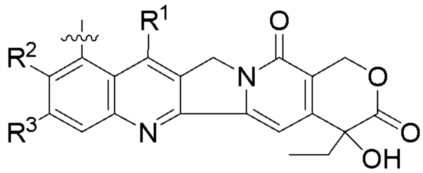 (D-1)

wherein $R^1$ is selected from H, $C_1-C_6$ alkyl, $C_2-C_6$ alkenyl, $C_2-C_6$ alkynyl, $C_1-C_6$ haloalkyl, $C_2-C_6$ haloalkenyl and $C_2-C_6$ haloalkynyl;
$R^2$ is selected from H, halogen, $C_1-C_6$ alkyl, $C_1-C_6$ haloalkyl, $-OR^4$ and $-SR^4$; $R^3$ is selected from H, halogen, CN, $C_1-C_6$ alkyl, $C_1-C_6$ haloalkyl and $-OR^4$; or $R^2$ and $R^3$ together form $-O(CH_2)_{n1}O-$ or $-O(CF_2)_{n1}O-$, wherein n1 is 1 or 2;
$R^4$ is selected from H or $C_1-C_4$ alkyl; and
n represents DAR, and n is a natural number selected from 1-15.

2. The antibody-drug conjugate, the stereoisomer or pharmaceutically acceptable salt or solvate thereof according to claim 1, wherein the heavy chain variable region of the Ab

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 7; or
(ii) comprises or consists of the amino acid sequence set forth in SEQ ID NO: 7; or
(iii) comprises or consists of an amino acid sequence having one or more amino acid substitutions, insertions or deletions compared with the amino acid sequence set forth in SEQ ID NO: 7, wherein optionally, the amino acid substitutions, insertions or deletions do not occur in the CDRs.

3. The antibody-drug conjugate, the stereoisomer or pharmaceutically acceptable salt or solvate thereof according to claim 1 or 2, wherein the light chain variable region of the Ab

(i) comprises or consists of an amino acid sequence having at least 90%, 91%, 92%, 93%, 94%, 95%, 96%, 97%, 98%, or 99% identity to the amino acid sequence set forth in SEQ ID NO: 9; or
(ii) comprises or consists of the amino acid sequence set forth in SEQ ID NO: 9; or
(iii) comprises or consists of an amino acid sequence having one or more amino acid substitutions, insertions or deletions compared with the amino acid sequence set forth in SEQ ID NO: 9, wherein optionally, the amino acid substitutions, insertions or deletions do not occur in the CDRs.

4. The antibody-drug conjugate, the stereoisomer or pharmaceutically acceptable salt or solvate thereof according to claim 1, wherein the heavy chain variable region of the Ab comprises or consists of the amino acid sequence set forth in SEQ ID NO: 7, and the light chain variable region of the Ab comprises or consists of the amino acid sequence set forth

in SEQ ID NO: 9.

5. The antibody-drug conjugate, the stereoisomer or pharmaceutically acceptable salt or solvate thereof according to any one of claims 1-4, wherein the Ab further comprises a heavy chain constant region, wherein the heavy chain constant region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 11; or
(ii) comprises or consists of the amino acid sequence set forth in SEQ ID NO: 11; or
(iii) comprises or consists of an amino acid sequence having one or more amino acid substitutions, insertions, or deletions compared with the amino acid sequence set forth in SEQ ID NO: 11.

6. The antibody-drug conjugate, the stereoisomer or pharmaceutically acceptable salt or solvate thereof according to any one of claims 1-5, wherein the Ab further comprises a light chain constant region, wherein the light chain constant region

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 12; or
(ii) comprises or consists of the amino acid sequence set forth in SEQ ID NO: 12; or
(iii) comprises or consists of an amino acid sequence having one or more amino acid substitutions, insertions, or deletions compared with the amino acid sequence set forth in SEQ ID NO: 12.

7. The antibody-drug conjugate, the stereoisomer or pharmaceutically acceptable salt or solvate thereof according to any one of claims 1-6, wherein the Ab comprises a heavy chain, wherein the heavy chain

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 13; or
(ii) comprises or consists of the amino acid sequence set forth in SEQ ID NO: 13; or
(iii) comprises or consists of an amino acid sequence having one or more amino acid substitutions, insertions, or deletions compared with the amino acid sequence set forth in SEQ ID NO: 13.

8. The antibody-drug conjugate, the stereoisomer or pharmaceutically acceptable salt or solvate thereof according to any one of claims 1-7, wherein the Ab comprises a light chain, wherein the light chain

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 14; or
(ii) comprises or consists of the amino acid sequence set forth in SEQ ID NO: 14; or
(iii) comprises or consists of an amino acid sequence having one or more amino acid substitutions, insertions, or deletions compared with the amino acid sequence set forth in SEQ ID NO: 14.

9. The antibody-drug conjugate, the stereoisomer or pharmaceutically acceptable salt or solvate thereof according to any one of claims 1-8, wherein the Ab comprises a heavy chain and a light chain, wherein the heavy chain comprises or consists of the amino acid sequence set forth in SEQ ID NO: 13, and the light chain comprises or consists of the amino acid sequence set forth in SEQ ID NO: 14.

10. The antibody-drug conjugate, the stereoisomer or pharmaceutically acceptable salt or solvate thereof according to any one of claims 1-9, wherein the Ab is selected from antigen-binding fragments Fv, Fab, Fab ', or $F(ab')_2$; a single-chain antibody scFv; or a single-domain antibody VHH.

11. The antibody-drug conjugate, the stereoisomer or pharmaceutically acceptable salt or solvate thereof according to any one of claims 1-10, wherein the linker is linked to Ab via a side chain of a cysteine residue.

12. The antibody-drug conjugate, the stereoisomer or pharmaceutically acceptable salt or solvate thereof according to any one of claims 1-11, wherein
$R^1$ is H, $R^2$ is $C_1$-$C_6$ alkyl or $C_1$-$C_6$ alkoxy, and $R^3$ is halogen, preferably -F.

13. The antibody-drug conjugate, the stereoisomer or pharmaceutically acceptable salt or solvate thereof according to any one of claims 1-12, wherein

$D^1$ has a structure represented by formula (D-2):

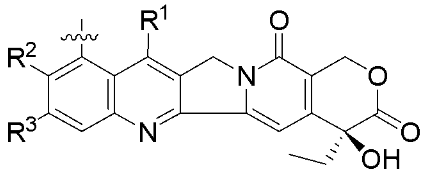

(D-2),

wherein $R^1$, $R^2$ and $R^3$ are as defined in any one of claims 1 to 12.

**14.** The antibody-drug conjugate, the stereoisomer or pharmaceutically acceptable salt or solvate thereof according to any one of claims 1-13, wherein $D^1$ has a structure represented by formula (D-4):

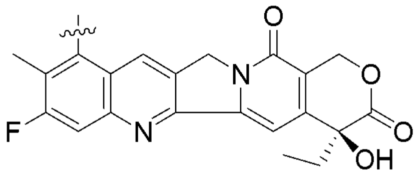

(D-4).

**15.** The antibody-drug conjugate, the stereoisomer or pharmaceutically acceptable salt or solvate thereof according to any one of claims 1-14, wherein

$-L^2-L^1-$ is $-(C_1-C_6$ alkylene$)-$, $*-(C_1-C_6$ alkylene$)-C(O)N(R^5)-(C_1-C_6$alkylene$)-$ or $*-(C_1-C_6$alkylene$)-N(R^5)C(O)-(C_1-C_6$alkylene$)-$, wherein * indicates that the terminus is covalently linked to Q;
$R^5$ is H or $C_1-C_6$alkyl.

**16.** The antibody-drug conjugate, the stereoisomer or pharmaceutically acceptable salt or solvate thereof according to any one of claims 1-15, wherein $-L^2-L^1-$ is $-(C_1-C_6$alkylene$)-$.

**17.** The antibody-drug conjugate, or the stereoisomer or pharmaceutically acceptable salt or solvate thereof according to any one of claims 1-16, wherein $-Q-L^2-L^1-$ is $-OCH_2-CH_2-CH_2-CH_2-$.

**18.** The antibody-drug conjugate, the stereoisomer or pharmaceutically acceptable salt or solvate thereof according to any one of claims 1-17, wherein Z is

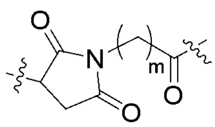

,

wherein m is an integer of 1-10.

**19.** The antibody-drug conjugate, the stereoisomer or pharmaceutically acceptable salt or solvate thereof according to any one of claims 1-18, wherein E is a peptide residue consisting of 2, 3 or 4 amino acids selected from glycine, alanine, valine, glutamine, glutamic acid, phenylalanine, leucine, tyrosine, lysine, citrulline, serine, tryptophan, aspartic acid, asparagine, isoleucine, arginine and proline, and wherein the glutamine or glutamic acid is optionally substituted with one polyol group and optionally substituted with one $C_{1-6}$alkyl;
preferably, the amino acid is selected from glycine, alanine, valine, glutamine, glutamic acid, phenylalanine, and leucine, wherein the glutamine or glutamic acid is optionally substituted with one polyol group and optionally substituted with one $C_{1-6}$alkyl.

**20.** The antibody-drug conjugate, the stereoisomer or pharmaceutically acceptable salt or solvate thereof according to claim 19, wherein the substituted glutamine or glutamic acid has a structure shown below:

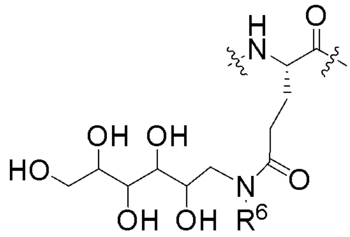 (G-1a), 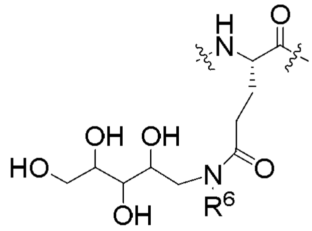 (G-1b),

wherein $R^6$ is H or $C_1$-$C_6$alkyl;
preferably

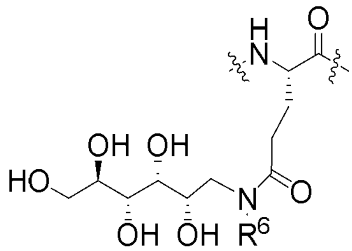 (G-1c)

wherein $R^6$ is H or $C_1$-$C_6$alkyl.

**21.** The antibody-drug conjugate, the stereoisomer or pharmaceutically acceptable salt or solvate thereof according to any one of claims 1-20, wherein E is: -Gln-Val-Ala-, -Gly-Val-Ala-, -Gln-Phe-Ala-, -Gly-Phe-Ala-, -Gly-Gly-Phe-Gly-, -Val-Ala-, -Val-Cit-, -Ala-Ala-, -Ala-Cit-, - Ala-Lys-, -Ala-Val-, -Asn-Cit-, -Asp-Cit-, -Asn-Lys-, -Asp-Val-, -Cit-Ala-, -Cit-Asn-, -Cit-Asp-, -Cit-Cit-, -Cit-Lys-, -Cit-Ser-, -Cit-Val-, -Glu-Val-, -Glu-Gly-, -Ile-Cit-, -Ile-Pro-, -Ile-Val-, - Leu-Cit-, -Lys-Cit-, -Phe-Arg-, -Phe-Cit-, -Phe-Lys-, -Pro-Lys-, -Ser-Cit-, -Trp-Cit-, -Ala-Val-, -Val-Asp-, -Cit-Val-, -Val-Glu-, -Val-Lys-, -Gly-Gly-Gly-, -Gly-Gly-Arg-, -Phe-Lys-Gly-, - Leu-Lys-Gly-, Leu-Leu-Gly-, -Glu-Val-Cit-, -Cit-Ala-Glu-, -Val-Lys-Gly-, -Val-Lys-Ala-, - Val-Gly-Gly-, -Val-Cit-Gly-, -Val-Gln-Gly-, -Val-Glu-Gly-, -Val-Lys-Gly-, -Val-Lys-Leu-, - Ala-Ala-Ala-, -Asn-Ala-Ala, -Gly-Gly-Gly-Gly, -Gly-Gly-Leu-Gly-, -Gly-Phe-Leu-Gly-, -Gly-Val-Lys-Gly-, -Ala-Leu-Ala-Leu-, -Gly-Phe-Leu-Gly-, -Ala-Leu-Ala-Leu-,-Gly-Phe-Gly-Gly-, and -Val-Lys-Gly-Gly-, wherein Gln and/or Glu are optionally substituted by one polyol group and optionally substituted by one $C_{1-6}$alkyl group.

**22.** The antibody-drug conjugate, the stereoisomer or pharmaceutically acceptable salt or solvate thereof according to claim 21, wherein
wherein E is -Gln-Val-Ala-, -Gly-Val-Ala-, -Gln-Phe-Ala-, -Gly-Phe-Ala-, -Gly-Gly-Phe-Gly-, -Val-Ala-, or

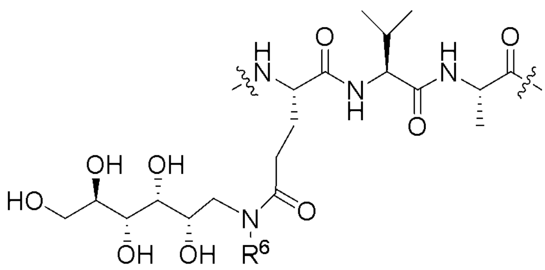

wherein $R^6$ is H or $C_1$-$C_6$alkyl, wherein the E groups are covalently linked to Z through the left N-terminus.

**23.** The antibody-drug conjugate, the stereoisomer or pharmaceutically acceptable salt or solvate thereof according to any one of claims 1-22,

wherein -Z-E-NH-$CH_2$- has the following structure

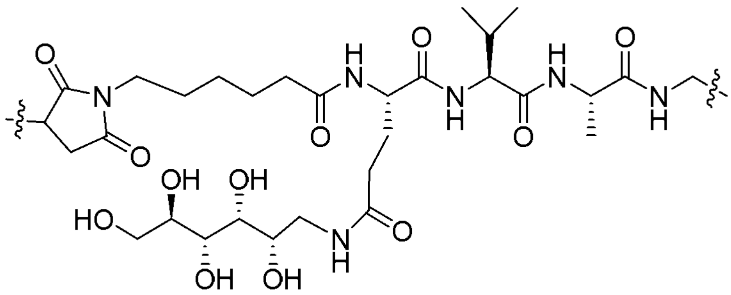

the left end thereof is linked to the Ab moiety.

**24.** The antibody-drug conjugate, the stereoisomer or pharmaceutically acceptable salt or solvate thereof according to any one of claims 1-23, wherein the antibody-drug conjugate has the following structure:

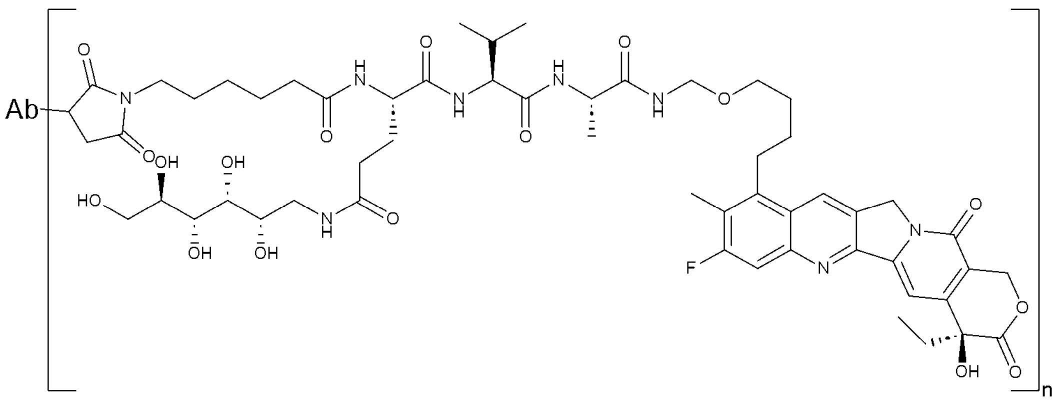

wherein Ab is as defined in any one of claims 1-23, n is DAR, and n is 1, 2, 3, 4, 5, 6, 7, 8, 9 or 10.

**25.** The antibody-drug conjugate, the stereoisomer or pharmaceutically acceptable salt or solvate thereof according to any one of claims 1-24, wherein the antibody-drug conjugate has an average DAR of 2-10, 4-9 or 7.5-8.5.

**26.** A pharmaceutical composition, comprising the antibody-drug conjugate, the stereoisomer or pharmaceutically acceptable salt or solvate thereof according to any one of claims 1-25, and a pharmaceutically acceptable carrier or excipient.

**27.** A method for treating or preventing a disease or disorder associated with HER3 activity, comprising administering to a subject an effective amount of the antibody-drug conjugate, the stereoisomer or pharmaceutically acceptable salt or solvate thereof according to any one of claims 1-25, or the pharmaceutical composition according to claim 26.

**28.** The method according to claim 27, wherein the disease or disorder associated with HER3 activity is a HER3-positive tumor.

**29.** The method according to claim 28, wherein the HER3 positive tumor is selected from the group consisting of breast cancer, lung cancer, pancreatic cancer, colorectal cancer, gastric cancer, head and neck squamous cell carcinoma, ovarian cancer, melanoma, prostate cancer and bladder cancer.

**30.** The method according to any one of claims 27-29, wherein the method further comprises co-administering one or more therapeutic agents selected from the group consisting of a chemotherapeutic agent, an angiogenesis inhibitor, a cytokine, a cytotoxic agent, an additional antibody, a small molecule drug, and an immunomodulatory agent.

**31.** The method according to claim 30, wherein the antibody-drug conjugate, the stereoisomer or pharmaceutically acceptable salt or solvate thereof and said one or more therapeutic agents are administered simultaneously or sequentially in any order.

**32.** The method according to claims 27-31, wherein the method further comprises administering to the patient one or more

treatment modalities selected from the group consisting of radiation therapy and surgery.

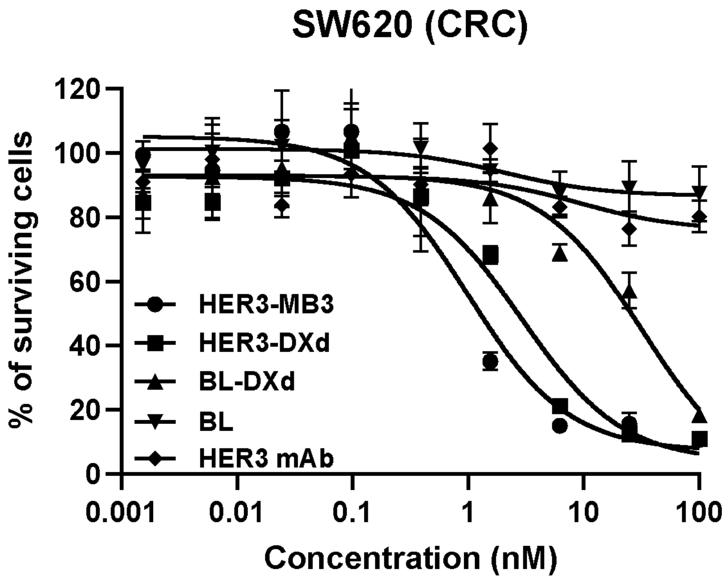


Figure 1

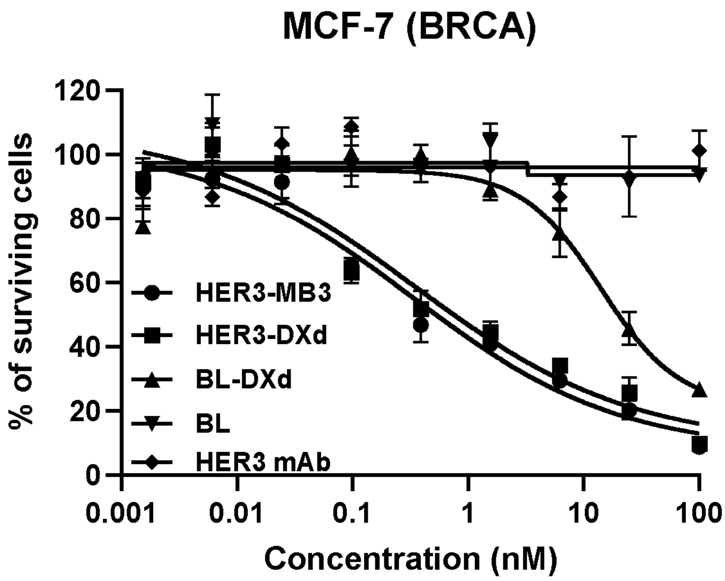


Figure 2

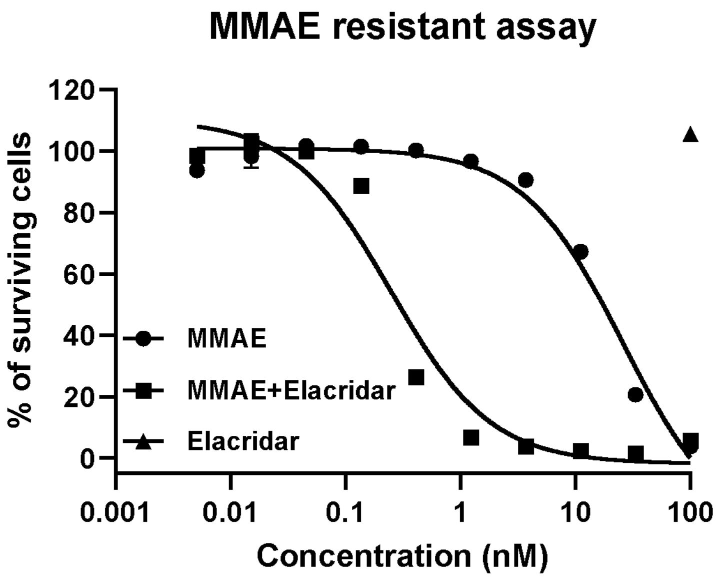


| | MMAE | MMAE+Elacridar |
|---|---|---|
| IC50 | 26.14 | 0.2514 |

A

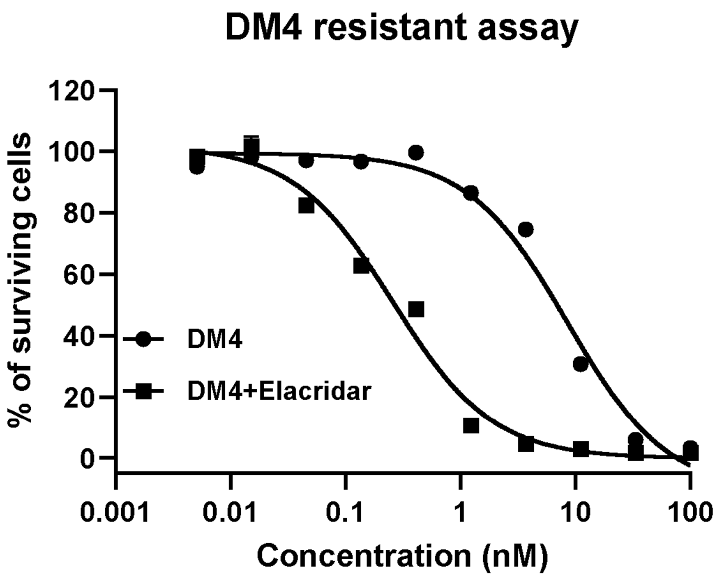


| | DM4 | DM4+Elacridar |
|---|---|---|
| IC50 | 8.614 | 0.2605 |

B

**Figure 3**

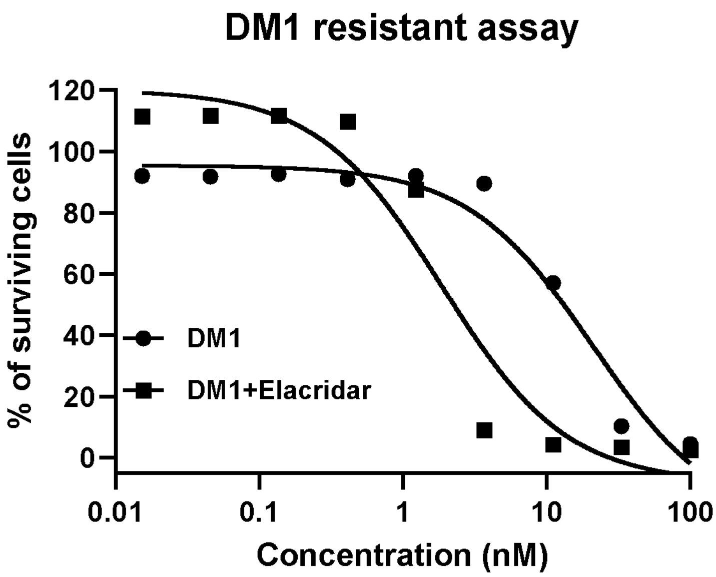


| | DM1 | DM1+Elacridar |
|---|---|---|
| IC50 | 20.97 | 1.877 |

C

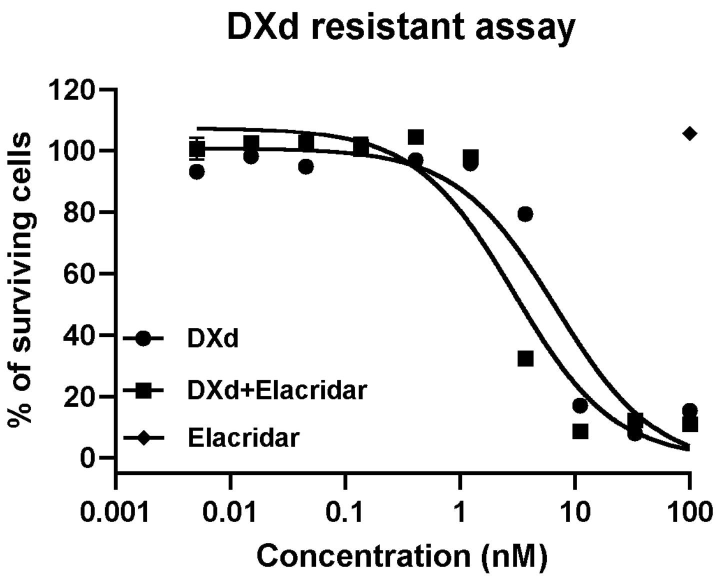


| | DXd | DXd+Elacridar |
|---|---|---|
| IC50 | 6.867 | 2.987 |

D

Figure 3 (continued)

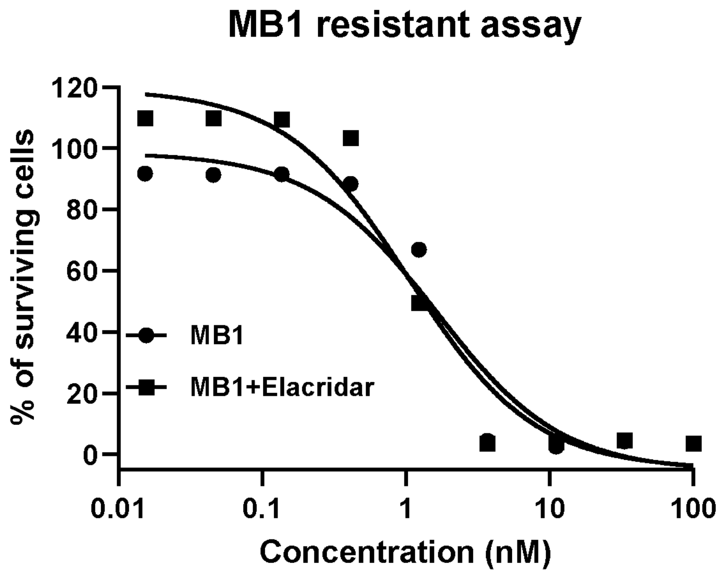


| | MB1 | MB1+Elacridar |
|---|---|---|
| IC50 | 1.631 | 1.058 |

E

**Figure 3 (continued)**

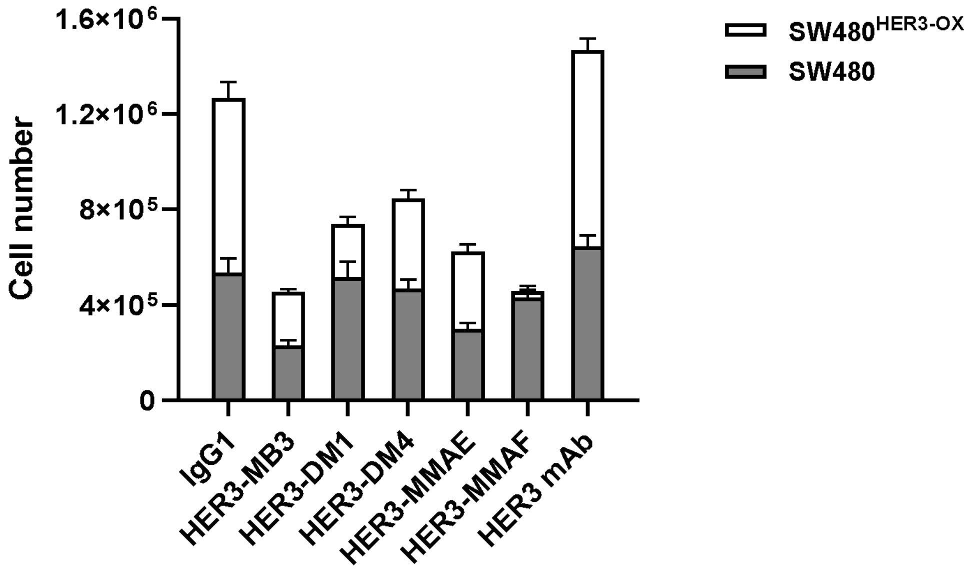


**Figure 4**

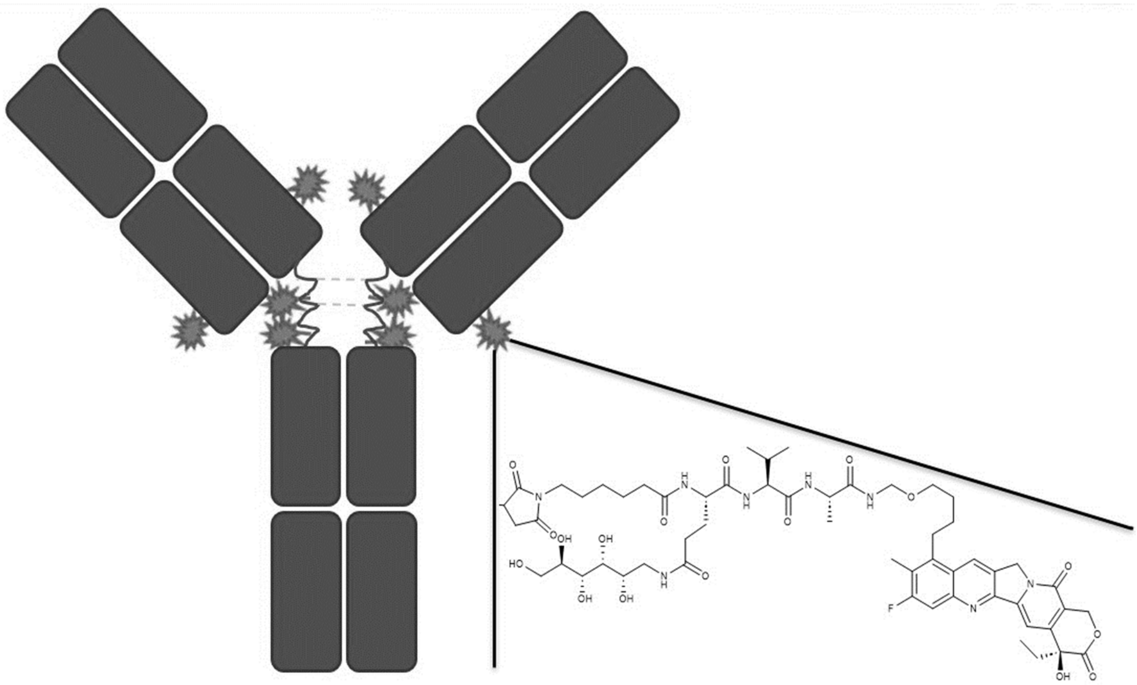

Figure 5

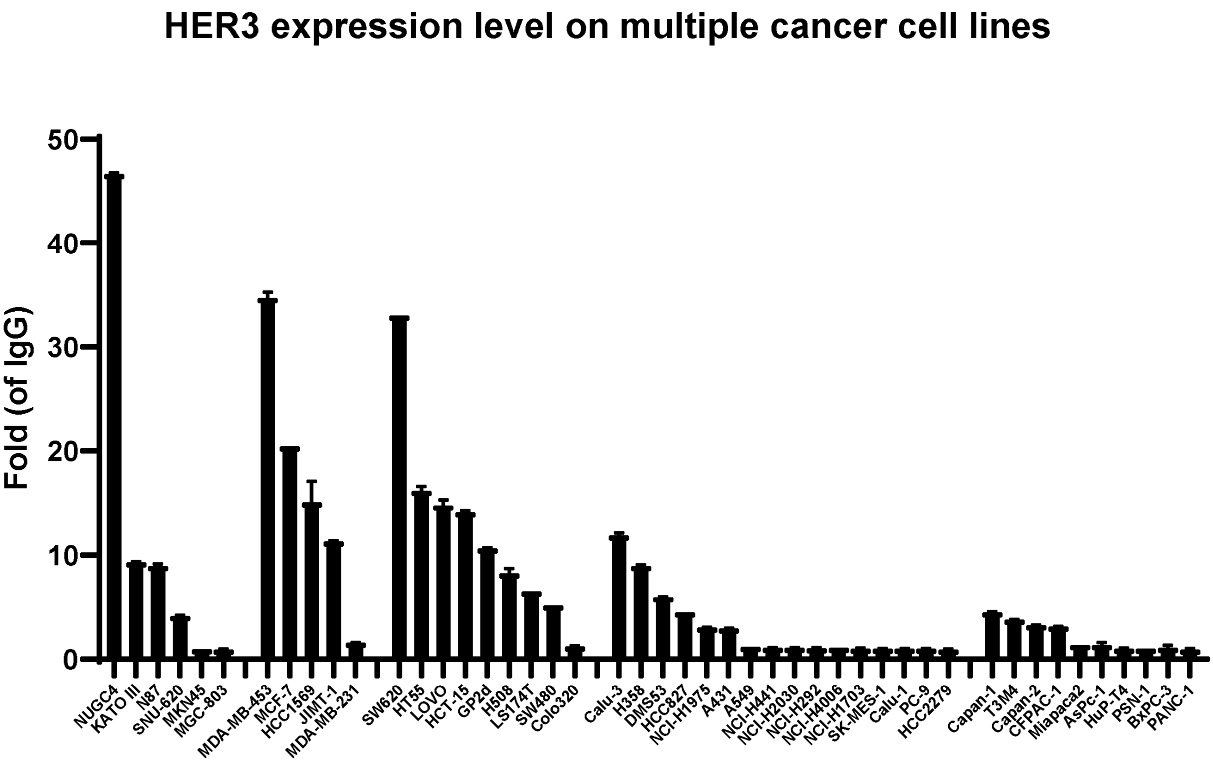
HER3 expression level on multiple cancer cell lines
Fold (of IgG)
50
40
30
20
10
0
NUGC4
KATO III
N87
SNU-620
MKN45
MGC-803
MDA-MB-453
MCF-7
HCC1569
JIMT-1
MDA-MB-231
SW620
HT55
LOVO
HCT-15
GP2d
H508
LS174T
SW480
Colo320
Calu-3
H358
DMS53
HCC827
NCI-H1975
A431
A549
NCI-H441
NCI-H2030
NCI-H292
NCI-H4006
NCI-H1703
SK-MES-1
Calu-1
PC-9
HCC2279
Capan-1
T3M4
Capan-2
CFPAC-1
Miapaca2
AsPc-1
HuP-T4
PSN-1
BxPC-3
PANC-1

Figure 6

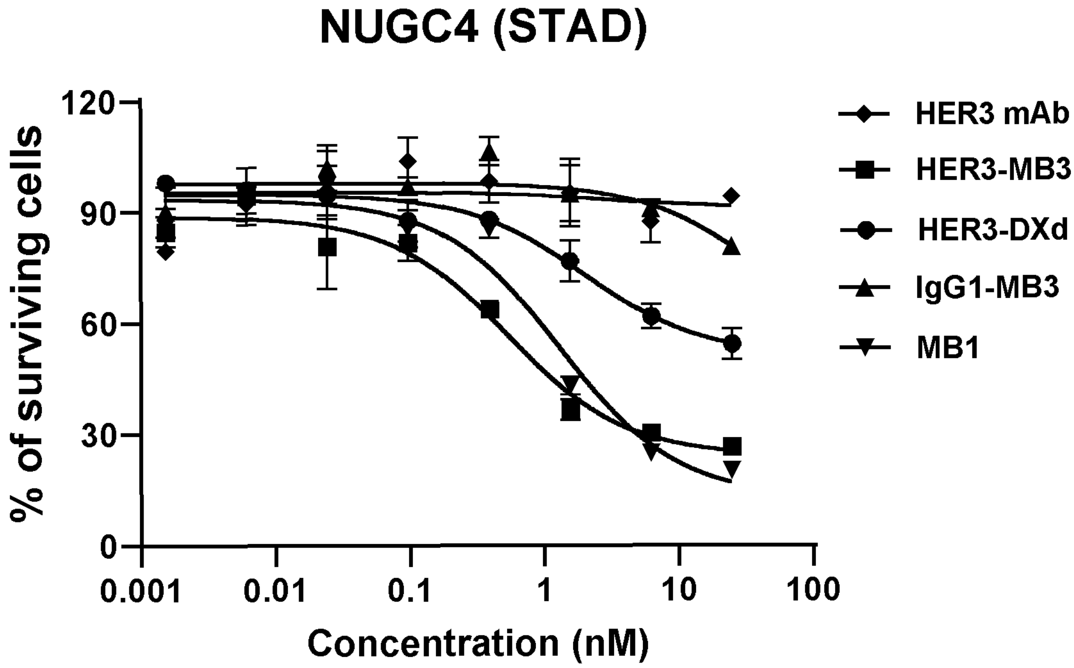


Figure 7

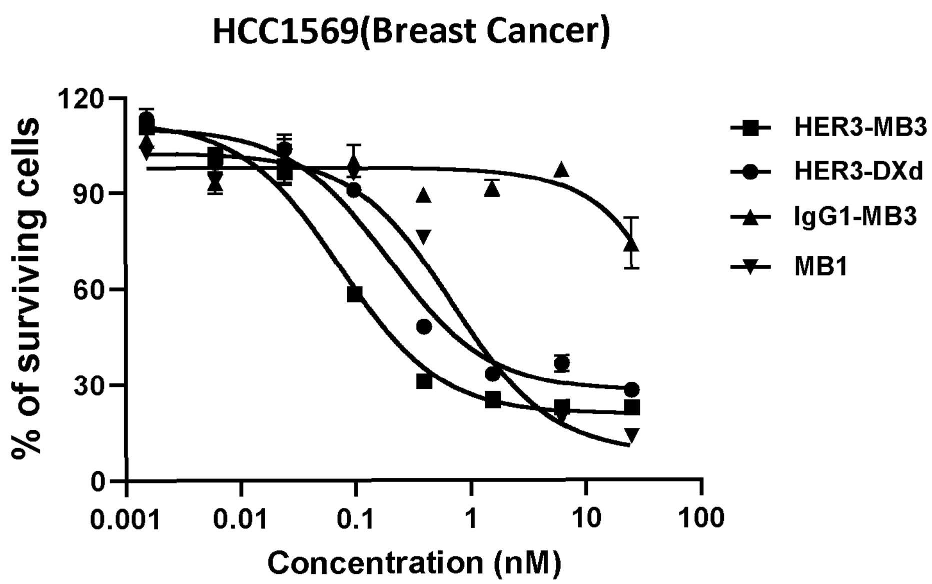


Figure 8

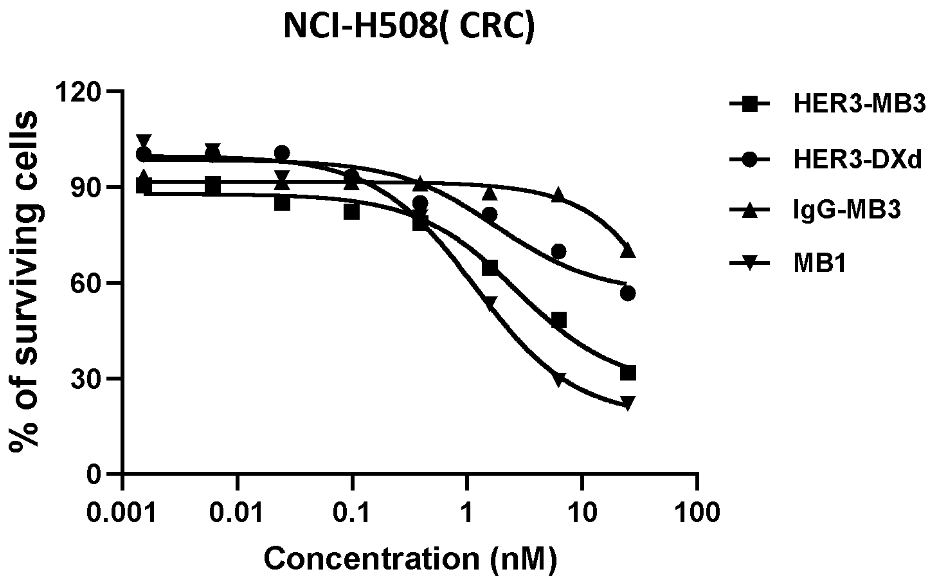


Figure 9

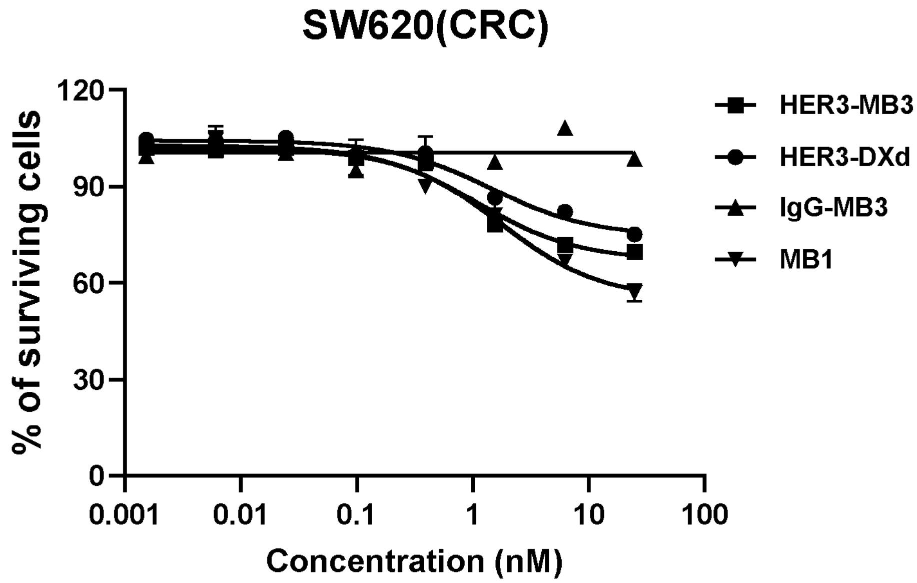


Figure 10

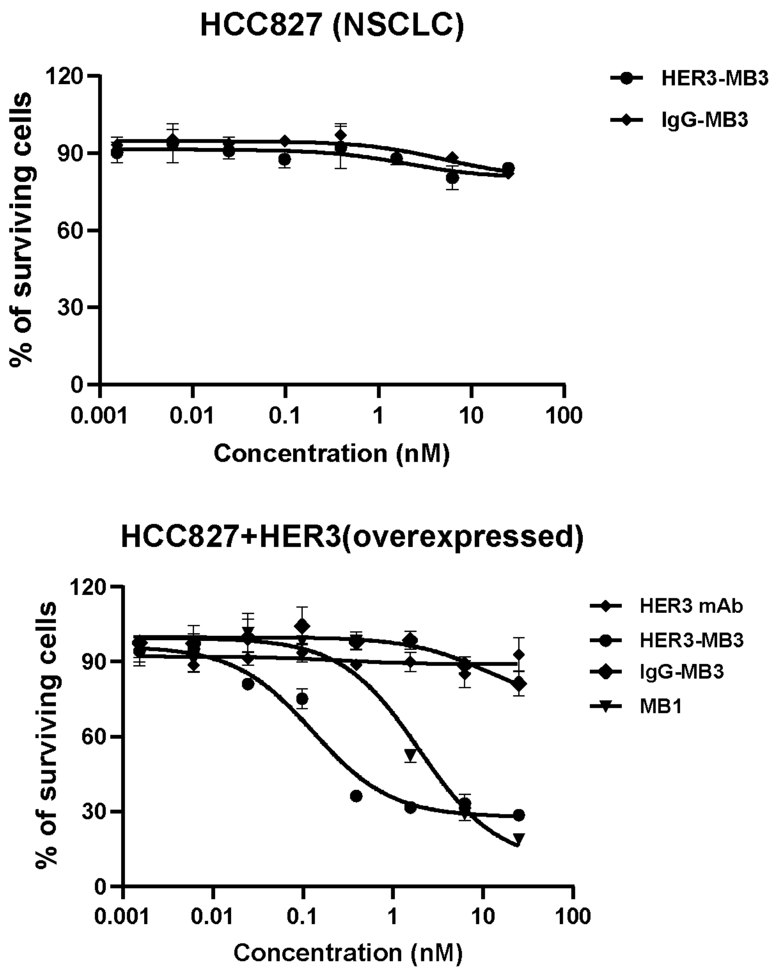


Figure 11

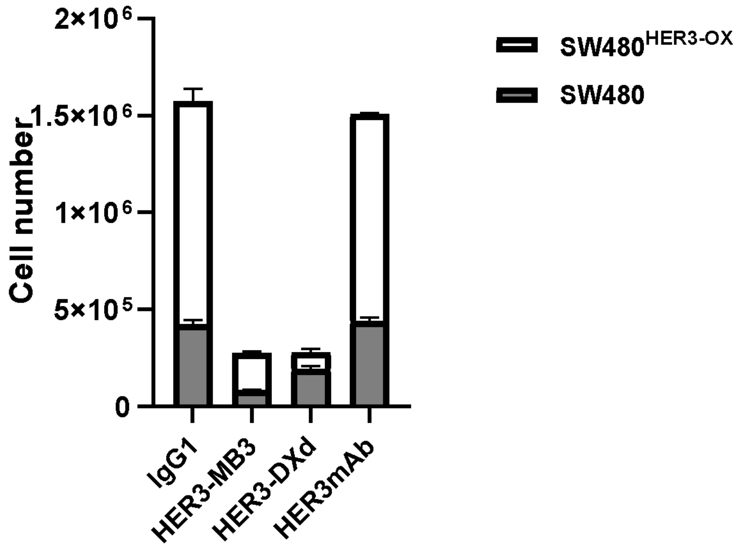


Figure 12

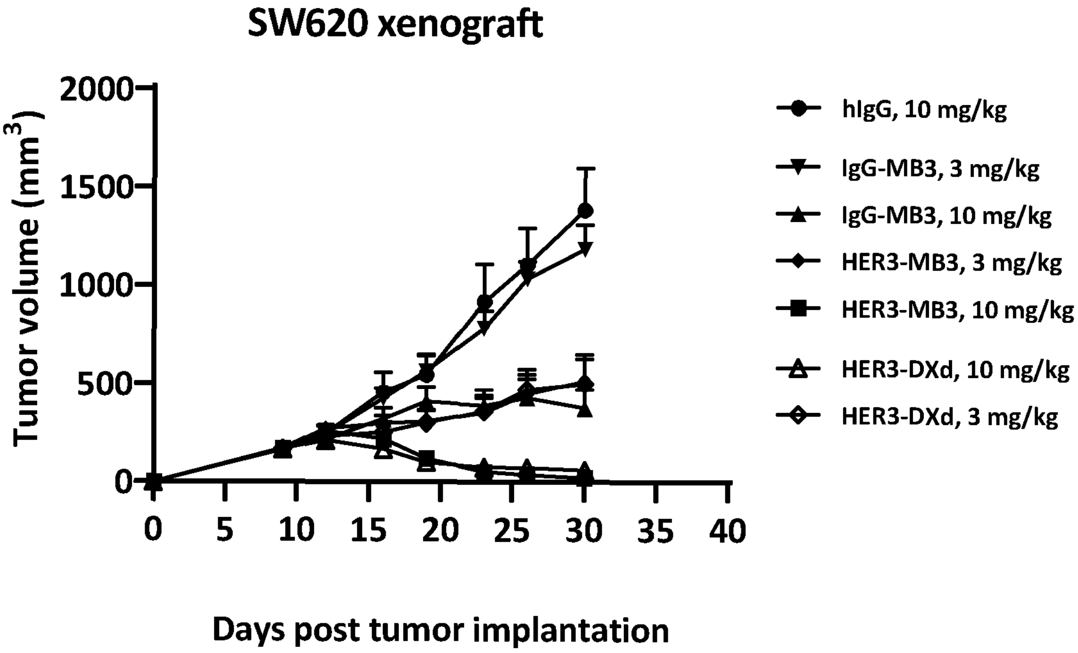


Figure 13

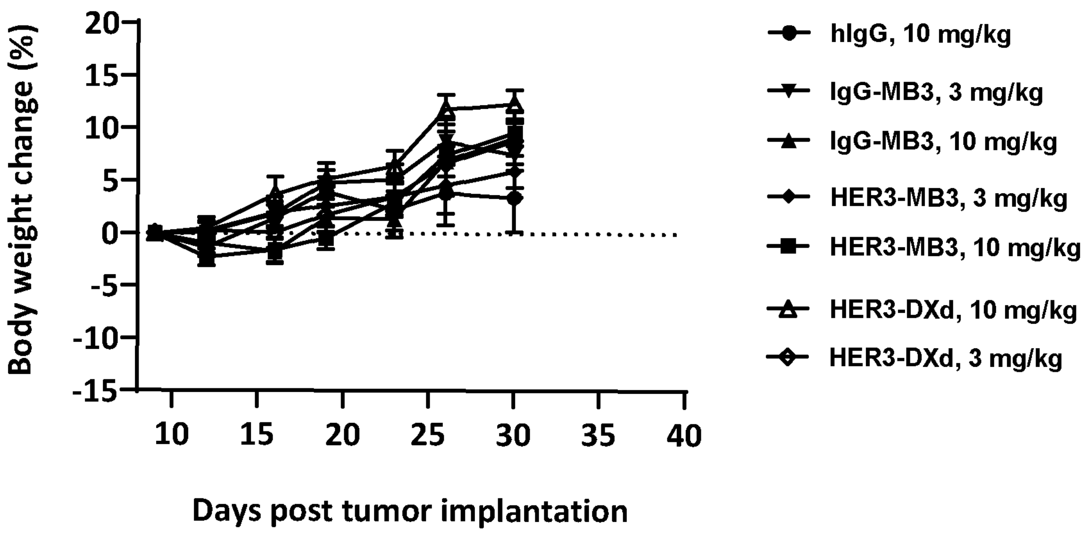


Figure 14

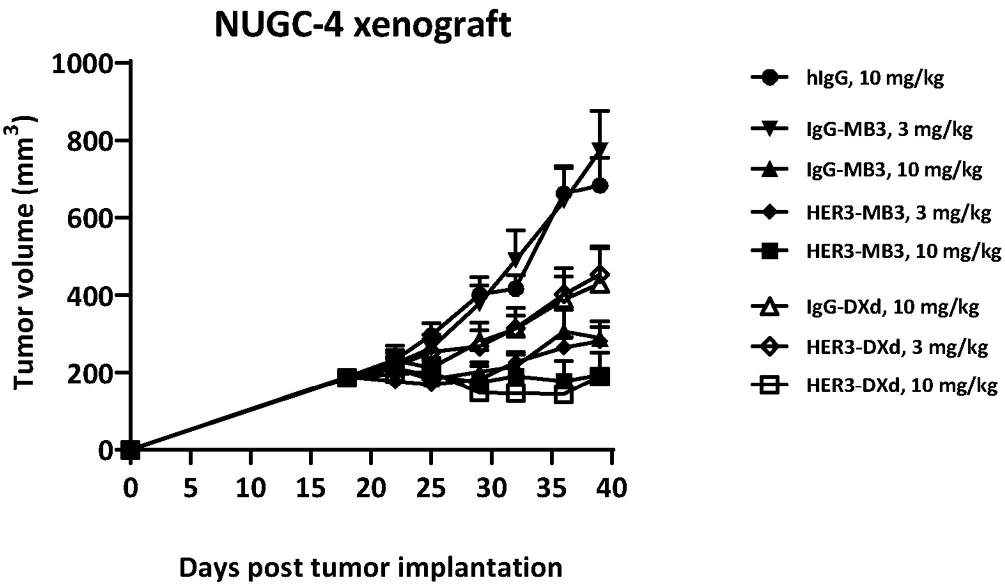


Figure 15

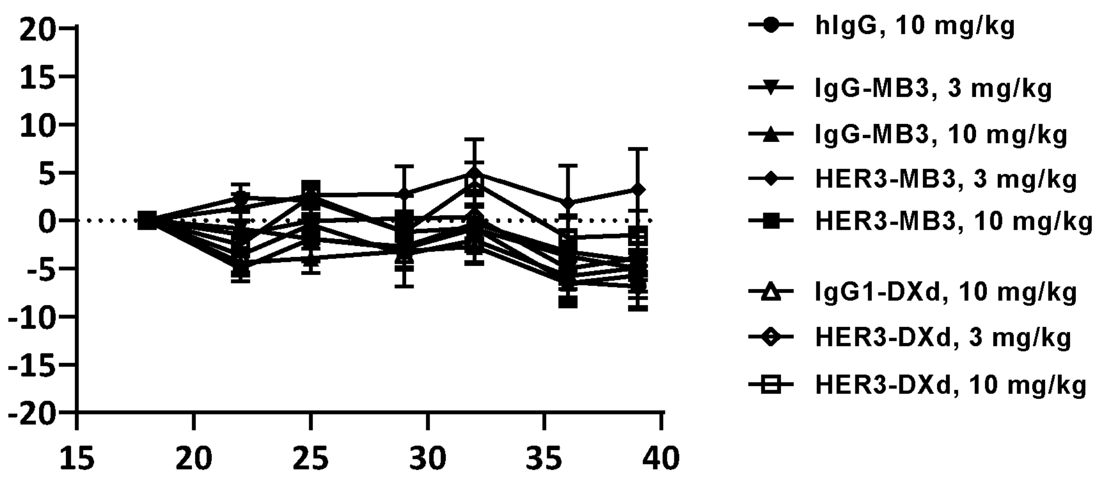


Figure 16

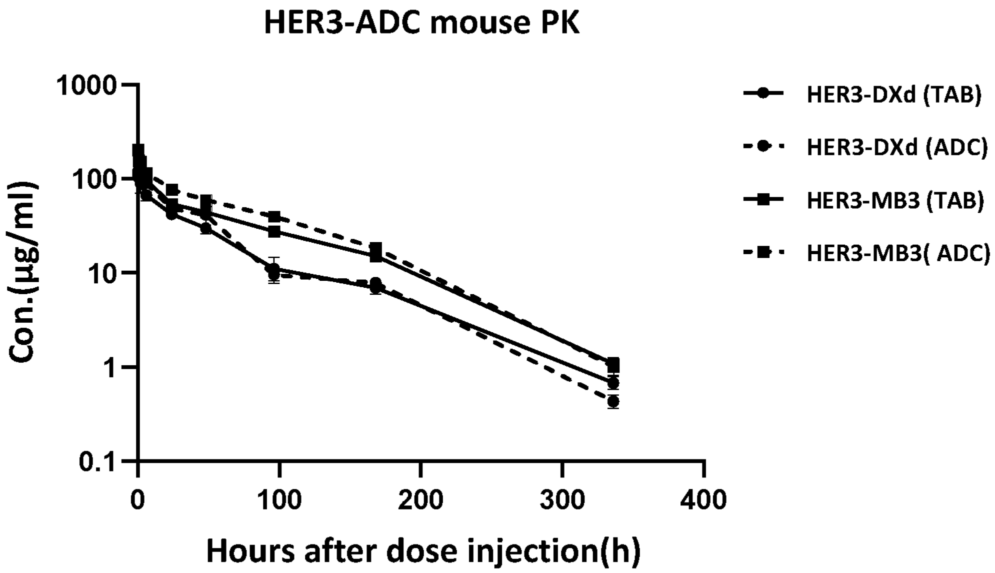


Figure 17

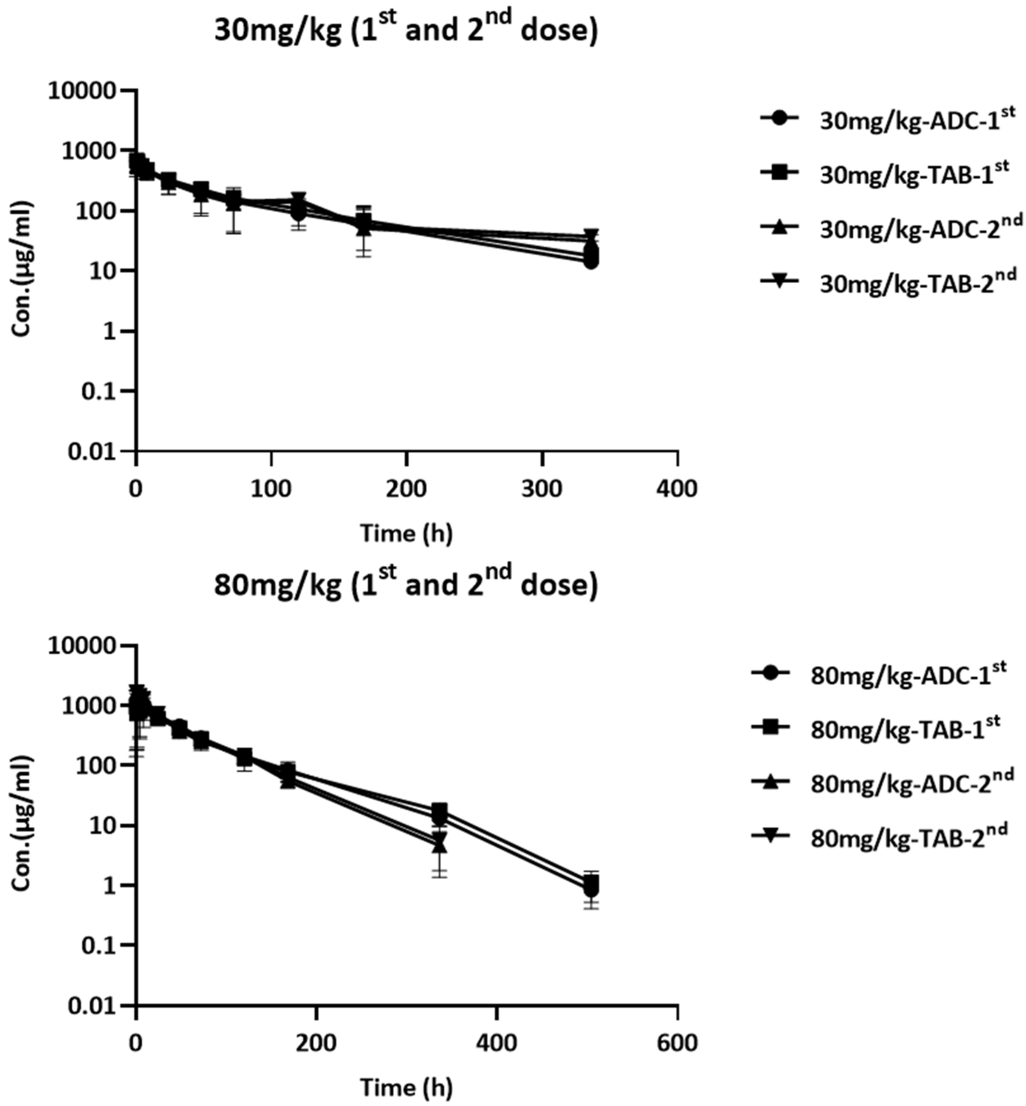


Figure 18

## INTERNATIONAL SEARCH REPORT

International application No.
**PCT/CN2024/121324**

**A. CLASSIFICATION OF SUBJECT MATTER**

A61K47/68(2017.01)i; A61K31/4745 (2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, 万方, WANFANG, 读秀, DUXIU, PUBMED, ISI_Web of Science, Science Direct, STNext: 基于序列1-6, 7, 9, 11-14的序列检索, search based on sequences 1-6, 7, 9 and 11-14, 基于D-1, D-2, D-4的结构检索, structure search based on D-1, D-2 and D-4, HER3抗体, 偶联物, 缀合物, ADC, conjugate

**C. DOCUMENTS CONSIDERED TO BE RELEVANT**

| Category* | Citation of document, with indication, where appropriate, of the relevant passages | Relevant to claim No. |
|---|---|---|
| Y | CN 115551552 A (MEDIBOSTON, INC.) 30 December 2022 (2022-12-30) claims 1-101, and description, paragraphs 789-791, 926, 962-963, 958 and 977, and embodiments 4-5 | 1-32 |
| Y | WO 2022180581 A2 (MEDIBOSTON LTD.) 01 September 2022 (2022-09-01) claims 1-51, and description, embodiments 3-4 | 1-32 |
| Y | CN 110944667 A (DAIICHI SANKYO COMPANY, LIMITED) 31 March 2020 (2020-03-31) claims 1-19 | 1-32 |
| Y | CN 112566942 A (DAIICHI SANKYO COMPANY, LIMITED) 26 March 2021 (2021-03-26) claims 1-28, and description, paragraphs 457-460 | 1-32 |
| A | CN 111601619 A (REGENERON PHARMACEUTICALS INC.) 28 August 2020 (2020-08-28) claims 1-34 | 1-32 |
| A | CN 112237634 A (SHANGHAI FUDAN-ZHANGJIANG BIO-PHARMACEUTICAL CO., LTD.) 19 January 2021 (2021-01-19) claims 1-10 | 1-32 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2024** | **23 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/121324**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/121324**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **27-32**
because they relate to subject matter not required to be searched by this Authority, namely:

Claims 27-32 set forth a method for treating or preventing diseases or conditions related to HER3 activity, which is a method for treatment of the human body, and thus said claims do not comply with PCT Rule 39.1(iv). The search for claims 27-32 is performed on the basis of the following amendment: the use of the antibody-drug conjugate of any one of claims 1-25, a stereoisomer or pharmaceutically acceptable salt or solvate thereof or the pharmaceutical composition of claim 26 in the preparation of a drug for treating or preventing diseases or conditions related to HER3 activity.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/121324**

| Patent document cited in search report | | | Publication date (day/month/year) | Patent family member(s) | | | Publication date (day/month/year) |
|---|---|---|---|---|---|---|---|
| CN | 115551552 | A | 30 December 2022 | JP | 2023520605 | A | 17 May 2023 |
| | | | | US | 2021283125 | A1 | 16 September 2021 |
| | | | | US | 12029736 | B2 | 09 July 2024 |
| | | | | WO | 2021173773 | A1 | 02 September 2021 |
| | | | | TW | 202146055 | A | 16 December 2021 |
| | | | | AU | 2021226341 | A1 | 29 September 2022 |
| | | | | CA | 3168882 | A1 | 02 September 2021 |
| | | | | MX | 2022010457 | A | 16 November 2022 |
| | | | | EP | 4110402 | A1 | 04 January 2023 |
| | | | | KR | 20230004453 | A | 06 January 2023 |
| | | | | BR | 112022017064 | A2 | 16 November 2022 |
| WO | 2022180581 | A2 | 01 September 2022 | JP | 2024509099 | A | 29 February 2024 |
| | | | | US | 2022378929 | A1 | 01 December 2022 |
| | | | | CA | 3208591 | A1 | 01 September 2022 |
| | | | | WO | 2022180581 | A3 | 13 October 2022 |
| | | | | KR | 20230154892 | A | 09 November 2023 |
| | | | | TW | 202302643 | A | 16 January 2023 |
| | | | | EP | 4297797 | A2 | 03 January 2024 |
| | | | | AU | 2022228004 | A1 | 28 September 2023 |
| | | | | AU | 2022228004 | A9 | 25 January 2024 |
| CN | 110944667 | A | 31 March 2020 | WO | 2019039483 | A1 | 28 February 2019 |
| | | | | RU | 2020111448 | A | 24 September 2021 |
| | | | | RU | 2020111448 | A3 | 29 November 2021 |
| | | | | JPWO | 2019039483 | A1 | 01 October 2020 |
| | | | | JP | 7204651 | B2 | 16 January 2023 |
| | | | | JP | 2023036900 | A | 14 March 2023 |
| | | | | JP | 7551729 | B2 | 17 September 2024 |
| | | | | EP | 3673918 | A1 | 01 July 2020 |
| | | | | EP | 3673918 | A4 | 19 May 2021 |
| | | | | IL | 272721 | A | 30 April 2020 |
| | | | | MA | 49987 | A | 01 July 2020 |
| | | | | KR | 20200044044 | A | 28 April 2020 |
| | | | | US | 2021128741 | A1 | 06 May 2021 |
| | | | | TW | 201919710 | A | 01 June 2019 |
| | | | | SG | 11202000996 | UA | 30 March 2020 |
| | | | | BR | 112020003474 | A2 | 20 October 2020 |
| | | | | CA | 3073383 | A1 | 28 February 2019 |
| | | | | CA | 3073383 | C | 31 October 2023 |
| | | | | AU | 2018320470 | A1 | 13 February 2020 |
| CN | 112566942 | A | 26 March 2021 | AU | 2019311557 | A1 | 04 February 2021 |
| | | | | KR | 20210038904 | A | 08 April 2021 |
| | | | | CA | 3107417 | A1 | 30 January 2020 |
| | | | | CA | 3107417 | C | 17 October 2023 |
| | | | | IL | 280295 | A | 25 March 2021 |
| | | | | JP | 2024050683 | A | 10 April 2024 |
| | | | | TW | 202019972 | A | 01 June 2020 |
| | | | | TWI | 847992 | B | 11 July 2024 |
| | | | | SG | 11202100653 | YA | 25 February 2021 |
| | | | | US | 2021283269 | A1 | 16 September 2021 |
| | | | | EP | 3828206 | A1 | 02 June 2021 |

Form PCT/ISA/210 (patent family annex) (July 2022)

**INTERNATIONAL SEARCH REPORT**
**Information on patent family members**

International application No.
**PCT/CN2024/121324**

| Patent document cited in search report | Publication date (day/month/year) | Patent family member(s) | Publication date (day/month/year) |
|---|---|---|---|
| | | EP 3828206 A4 | 20 April 2022 |
| | | JPWO 2020022363 A1 | 02 August 2021 |
| | | BR 112021001194 A2 | 27 April 2021 |
| | | WO 2020022363 A1 | 30 January 2020 |
| CN 111601619 A | 28 August 2020 | US 2021332080 A1 | 28 October 2021 |
| | | US 12134631 B2 | 05 November 2024 |
| | | JP 2024045283 A | 02 April 2024 |
| | | AU 2018365946 A1 | 14 May 2020 |
| | | SG 11202004151 YA | 29 June 2020 |
| | | JP 2021502341 A | 28 January 2021 |
| | | JP 7426931 B2 | 02 February 2024 |
| | | MX 2020004691 A | 20 August 2020 |
| | | EP 3706805 A2 | 16 September 2020 |
| | | KR 20200085807 A | 15 July 2020 |
| | | EA 202091135 A1 | 14 October 2020 |
| | | WO 2019094395 A2 | 16 May 2019 |
| | | WO 2019094395 A3 | 22 August 2019 |
| | | IL 274427 A | 30 June 2020 |
| | | IL 274427 B1 | 01 July 2024 |
| | | IL 274427 B2 | 01 November 2024 |
| | | IL 313068 A | 01 July 2024 |
| | | CA 3080857 A1 | 16 May 2019 |
| CN 112237634 A | 19 January 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021173773 A
- WO 2022180581 A
- CN 102574866
- WO 2021173773 A1


### Non-patent literature cited in the description


- Immunology-A Synthesis. Sinauer Associates, Sunderland, 1991
- **STAHL** ; **WERMUTH**. Handbook of Pharmaceutical Salts: Properties, Selection, and Use. Wiley-VCH, 2002
- **ANSEL, HOWARD C et al.** Ansel's Pharmaceutical Dosage Forms and Drug Delivery Systems. Lippincott, Williams & Wilkins, 2004
- **GENNARO, ALFONSO R. et al.** Remington: The Science and Practice of Pharmacy. Lippincott, Williams & Wilkins, 2000
- **ROWE, RAYMOND C.** Handbook of Pharmaceutical Excipients. Pharmaceutical Press, 2005
- **HOOVER, JOHN E.** Remington's Pharmaceutical Sciences. Mack Publishing Co., 1975
- Pharmaceutical Dosage Forms. Marcel Decker, 1980
- Handbook of Pharmaceutical Excipients. American Pharmaceutical Association, 1999